(19) Europäisches Patentamt
European Patent Office
Office européen des brevets

(11) **EP 1 379 634 B1**

(12) **EUROPEAN PATENT SPECIFICATION**

(45) Date of publication and mention
of the grant of the patent:
**13.12.2017 Bulletin 2017/50**

(51) Int Cl.:
*C12N 9/50* *(2006.01)*   *C12N 9/99* *(2006.01)*
*C12Q 1/34* *(2006.01)*   *C12Q 1/37* *(2006.01)*
*C12Q 1/42* *(2006.01)*   *C12Q 1/44* *(2006.01)*

(21) Application number: **02728885.1**

(22) Date of filing: **22.04.2002**

(86) International application number:
**PCT/US2002/012611**

(87) International publication number:
**WO 2002/086115 (31.10.2002 Gazette 2002/44)**

(54) **ACTIVE SITE INACTIVATORS**

INAKTIVATOREN AKTIVER ZENTREN

INACTIVATEURS DE SITE ACTIF

(84) Designated Contracting States:
**AT BE CH CY DE DK ES FI FR GB GR IE IT LI LU MC NL PT SE TR**

(30) Priority: **20.04.2001 PCT/US01/12919**
**20.04.2001 US 839428**
**31.10.2001 US 335025 P**

(43) Date of publication of application:
**14.01.2004 Bulletin 2004/03**

(73) Proprietor: **THE UNIVERSITY OF GEORGIA RESEARCH FOUNDATION, INC.**
**Athens**
**Georgia 30602-7411 (US)**

(72) Inventor: **BAIG, Salman**
**Athens, GA 30605 (US)**

(74) Representative: **Sauer, Philippe et al**
**Henkel, Breuer & Partner**
**Patentanwälte**
**Brienner Straße 1**
**80333 München (DE)**

(56) References cited:
**WO-A-00/09653      WO-A-00/63350**
**US-A- 6 068 982      US-B1- 6 200 771**
**US-B1- 6 280 940      US-B1- 6 331 408**

• **WHITE A C ET AL: "TAENIA SAGINATA ONCOSPHERE EXCRETORY/SECRETORY PEPTIDASES" JOURNAL OF PARASITOLOGY, AMERICAN SOC. OF PARASITOLOGISTS, WINSTON-SALEM,, US, vol. 82, no. 1, February 1996 (1996-02), pages 7-10, XP000965374 ISSN: 0022-3395**

**Description**

BACKGROUND OF THE INVENTION

Peptide scission enzymes are important chemical species in nature that cleave peptide and protein molecules at specific regions, whether cleaving the backbone or cleaving a prosthetic group or substituent, where cleavage patterns are determined by enzyme recognition of substrate subdomains, and by the particular configuration of each peptide scission enzyme's active site in the transition state and/or in the ground state. Hereinafter the term scission enzyme refers to a peptide scission enzyme. Currently among the most important members of this class of enzymes are proteases, which cleave peptide bonds, and which play key roles in the pathological processes of many disease organisms. Other important members include, for example: protein phosphatases, which are important in physiological regulation of enzyme activity; esterases, such as amino acid esterases; enzymes cleaving saccharide-based side chains of proteins; enzymes that cleave lipoproteins or lipid-functionalized proteins; and enzymes that cleave cofactors from proteins. Note that most proteolytic enzymes also catalyze hydrolysis of an ester bond, hence a scientific advance in manipulating the chemistry of one category of enzymatic scission can be transferable to other scission reactive categories.

The utility of inhibiting scission enzymes is illustrated by protease inhibitors, which are among the most promising classes of drugs for the treatment of a wide array of devastating diseases, including but not limited to AIDS, cancer, malaria, diabetes, Alzheimer's, and arthritis. These diseases proliferate by using proteases to cleave cellular proteins, thereby weakening and potentially killing the host. Protease inhibitor drugs treat these diseases by inhibiting the pathological protease. Until recently, most of these drugs were produced by a trial and error mechanism (Appelt et al., 1991). To combat the problems innate to this random process, rational drug design was introduced and implemented in the 1990's (Appelt et al., 1991). Rational drug design employs complex 3-dimensional computer modeling, combinatorial chemistry and extremely high throughput screening (HTS). This advance paved the way for the development of HIV protease inhibitors (Vacca et al., 1994, Erickson et al., 1990, Roberts et al., 1990). Protease inhibitor successes have also been noted for other disorders including high blood pressure (Radzicka and Wolfenden, 1996).

Antiparasitic protease inhibitor chemotherapy has been shown to be efficacious in animal models. Fluoromethylketone-derivatized dipeptides have been shown to cure murine malaria (Rasnick, 1985); vinylsulfone derivatized dipeptides can cure infection of cutaneous leishmaniasis (Palmer et al., 1985) and replication of *Trypanasoma cruzi* in Chagas disease in animal models (Bromme et al., 1996); and cysteine protease inhibitors can arrest or cure animal models of schistosomiasis (Rasnick, 1985). Moreover, investigations have shown that total cures of lethal parasite burden can be achieved with clinically acceptable protease inhibitor dosing regimens (Bromme et al. (1996) and through oral administration routes (Klenert et al., 1992). Finally, the lack of toxicity noted in many protease inhibitor treatments, even when the inhibitor was not entirely specific for its target, has drawn significant attention to these compounds as attractive platforms for chemotherapeutic development. This absence of host side-effects may be related to relative lack of redundancy in the proteases of foreign organisms compared to the mammalian systems that they reside in (McKerrow et al., 1999). Additionally, host proteases may simply exceed the concentration of proteases within the foreign organism. Moreover, pathogens may be naturally adapted to intake and consequently concentrate the small molecular weight inhibitors. Indeed, the development of animal models for infectious diseases has proved the attractiveness of protease inhibitors for the rational drug design model.

Rational drug design nonetheless has severe limitations with respect to speed (Service, 2000), cost (Service 2000), and efficacy (Ladbury and Peters, 1994). For example, a significant majority of X-ray crystal structures for prospective targets that rational drug design employs are not available (Service, 2000). Consequently, most known drug targets are unexploitable through this experimental paradigm. Even for the 1 % of exploitable targets, tremendous failure rates occur due to the lack of an efficient algorithm that can lead to an efficacious lead compound (Ladbury and Peters, 1994; Lahana, 1999). For methods that rely upon traditional screening mechanisms, the systems are still non-logical and require more rational development in the heuristic algorithms. Only 1 in 10,000 lead compounds are estimated to become final drugs (Parril et al., 1999). In fact many of the most efficacious drugs on the market today, including CAPTROPRIL (anti-hypertensive agent), PREDNISONE (antibacterial agent), and PRAZIQUANTEL (antiparasitic agent), have been discovered by sheer serendipity (Kubinyi, 1999). The result of the inefficacy of rational drug design is that only 40-45 new drugs are produced annually (Kubinyi, 1999). Consequently, one third of the world is without basic medication (Service, 2000). Clearly, there is a need for a better algorithm to reduce failure at the lead compound discovery stage of protease inhibitor development.

WO 00/63350 A1 discloses a *Taenia* cyst wall cysteine proteinase, inhibitors thereof and pharmaceutical compositions comprising an inhibitor molecule inhibiting the activity of a cyst wall cysteine protease and a pharmaceutical acceptable carrier. In particular potential fluoromethylketone inhibitors which abolish activity of a target cysteine protease purified from *Taenia solium* are described.

WO 00/09653A1 refers to methods of identifying inhibitors of cathepsin K and methods of designing inhibitors of cathepsin K using structural and physical data based on the crystallographic structure of procathepsin K.

White et al. describe in Am. Soc. of parasitologists 1996, vol. 82, No. 2 on pages 7 to 10 general protease inhibitors against the purified *Taenia saginata* protease.

White et al. refer in Mol. Biochem. Parasit. 1997, pages 243 to 253 to the purification and characterization of cysteine protease from Taenia crassiceps cysts.

Barrett et al. disclose in Biochem. J. 1982, pages 189 to 198 E64, which is a commercially available enzyme inhibitor. Specifically, this inhibitor is described in this reference as specific for cysteine proteases. Some of the key components described are the active site nature of interaction between E64 and the enzyme active site, methods to determine the molarity of the enzymes, inactivation rate constants for inhibition of the several known enzymes and a comparison between EP-475 and E64.

SUMMARY OF THE INVENTION

The present invention relates to a method for identifying a peptide scission enzyme inhibitor comprising:

a. performing a low kcat substrate assay by contacting each of a plurality of substrates with a target peptide scission enzyme to identify at least one high kcat peptide substrate and at least one low kcat peptide substrate being poorly cleavable by the target peptide scission enzyme, wherein the low kcat peptide substrate has a cleavability rate of less than 35% of the cleavability rate of the most highly cleaved substrate in the assay; and

b. performing an enzyme active site competitive binding assay using the target peptide scission enzyme, at least one high kcat substrate identified in step a. and at least one low kcat substrate identified in step a. and identifying thereby at least one low kcat substrate being an active site binding inhibitor, which inhibits the cleavage of the at least one high kcat substrate by the target peptide scission enzyme by binding to the active site of the target peptide scission enzyme, thereby identifying a low kcat active site binding enzyme inhibitor comprising an amino acid core or peptide core.

Preferably, the low kcat substrate has a cleavability rate of less than 20% of the cleavability rate of the most highly cleaved substrate in the assay.

Moreover, it is preferred that the low kcat substrate has a cleavability rate of less than 10% of the cleavability rate of the most highly cleaved substrate in the assay.

Preferably, the enzyme active site competitive binding assay comprises assessment of the binding of the substrate to the enzyme by employing a spectroscopic method selected from the group consisting of nuclear magnetic resonance spectroscopy, infrared spectroscopy, ultra-violet-visual spectroscopy, fluorometric spectroscopy, phosphorometric spectroscopy, mass spectrometry, and microwave spectroscopy.

Moreover, it is preferred that performing the low kcat substrate assay comprises assessing the kcat of the substrate for the enzyme by employing a spectroscopic method selected from the group consisting of nuclear magnetic resonance spectroscopy, infrared spectroscopy, ultraviolet-visual spectroscopy, fluorometric spectroscopy, phosphorometric spectroscopy, mass spectrometry, and microwave spectroscopy.

Preferably, the method further comprised the additional steps of selecting a functional and structural analog of the low kcat inhibitor and performing an inhibitory assay using the target peptide scission enzyme and at least one structural analog of the low kcat inhibitor to identify at least one low kcat inhibitor.

Moreover, it is preferred that the method further comprises assessing the selectivity of the low kcat inhibitor with respect to enzymes of the same class as the target peptide scission enzyme in order to determine whether the inhibitor is selective for the target peptide scission enzyme.

Preferably, the method further comprises determining optimal substrate cleavage conditions for the target peptide scission enzyme prior to contacting the target peptide scission enzyme with the plurality of substrates.

Moreover, it is preferred that the peptide scission enzyme inhibitor comprises an amino acid core or a peptide core containing between 1 and 9 amino acid residues.

Preferably, the amino acid core or peptide core contains between 1 and 6 amino acid residues.

Moreover, it is preferred that the amino acid core or peptide core consists of 1, 2 or 3 amino acid residues.

Preferably, the amino acid core or peptide core comprises one or more residues from the group consisting of L-amino acids, D-amino acids, derivatized amino acids, and modified amino acids.

Moreover, it is preferred that the amino acid core or peptide core is composed entirely or in part of peptide analogs selected from the group consisting of: beta-amino acids, peptoids, and amino acids in which one or more atoms have been replaced by different atomic elements or by functional groups.

Preferably, the amino acid core or peptide core comprises two or more amino acids linked by a bond other than a peptide bond.

Moreover, it is preferred that the target peptide scission enzyme is provided as a purified enzyme or in a crude mix.

Preferably, the method of claim 1, further comprising prior to performing the enzyme active site binding assay, determining an optimal pH range of about 2 pH units for the cleavage reaction.

Moreover, it is preferred that the method comprises performing a competitive binding assay on a selected number of low kcat substrates, wherein performing the competitive binding assay comprises, for each selected low kcat substrate:

- performing a first competitive binding assay using the target peptide scission enzyme, a first population of high kcat substrates, and the selected low kcat substrate to identify a plurality of low kcat inhibitors;
- for each low kcat inhibitor, performing a second competitive binding assay using the target peptide scission enzyme, a second population of high kcat substrates and the low kcat inhibitor, wherein the second population of high kcat substrates includes a greater number of substrates that the first population of high kcat substrates; and
- quantifying the inhibitory effect of the low kcat inhibitors to yield a ranked list of low kcat inhibitors.

Preferably, a blocking group is located at a site selected from the following combination: the C-terminus of the peptide core; the N-terminus of the peptide core.

Moreover, it is preferred that the enzyme is selected from the group consisting of: proteases, protein phosphatases, esterases, enzymes cleaving saccharide-based side chains of proteins, enzymes that cleave lipoproteins or lipid-functionalized proteins, and enzymes that cleave cofactors from proteins.

Preferably, the amino acid core or peptide core comprises one or more residues from the group consisting of L-amino acids, D-amino acids, derivatized amino acids, and modified amino acids.

Moreover, it is preferred that the amino acid core or peptide core is composed entirely or in part of peptide analogs selected from the group consisting of: beta-amino acids, peptoids, and amino acids in which one or more atoms have been replaced by different atomic elements or by functional groups.

Preferably, the amino acid core or peptide core comprises two or more amino acids linked by a bond other than a peptide bond.

BRIEF DESCRIPTION OF THE DRAWINGS

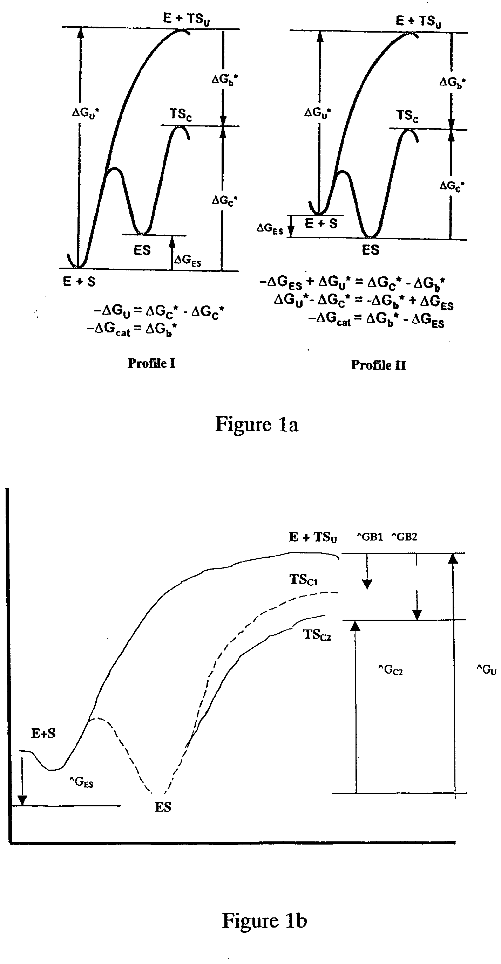

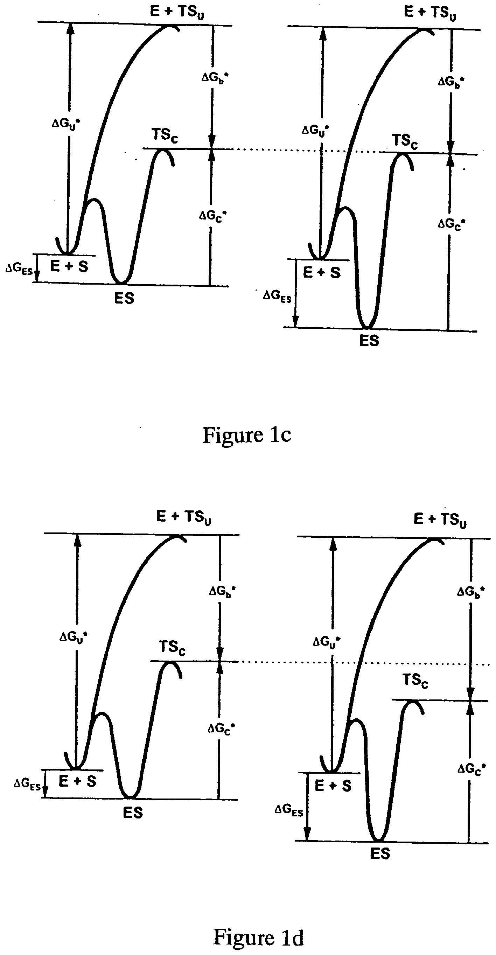

Figure 1. Free energy diagrams for various profiles and scenarios of enzyme catalysis; from Murphy, 1995. Biochemistry. 34(14):4507-4510: (a) Free energy diagrams for different concentrations of substrate S in catalyzed and in uncatalyzed reactions. Left panel: Profile I. S<Km such that enzyme is in a "loose" binding configuration with substrate. Right panel: Profile II. S>Km such that the enzyme is in a "tight" binding configuration with the substrate. Nomenclature below the figure indicates derivation of various free energy constants which are referred to in the text. Since catalysis is known to occur for Profile II like enzymes, this plot demonstrates how tight binding interactions need not be wasteful for substrate turnover, as long as they are utilized for catalysis; (b) free energy diagram for catalysis driven by transition state stabilization for a Profile II scenario where $TS_C$ is decreased. In this scenario, the transition state is decreased from $TS_{C1}$ to $TS_{C2}$ resulting in a net increase of $\Delta G_B$ from situation 1 to situation 2. Hence an increase in $\Delta AGB$ is the hallmark of the transition state effect. Since $\Delta G_{ES}$ is unchanged in this plot, 100% of the additional binding energy is utilized for catalysis. This property and the increase in $\Delta AGb$ is the definition of a catalytic event driven by transition state

stabilization, with the latter being the defining catalytic parameter; (c) free energy diagram for catalysis driven by ground state destabilization for a Profile II scenario where ES is decreased and $TS_C$ is not changed. From the left panel to the right panel, ES is decreased, whereas TS remains unchanged. Consequently, $\Delta\Delta Gb$ remains constant although catalysis is noted (thus the free energy of enzyme/transition state binding is unchanged). The constant $\Delta\Delta Gb$ in the case where catalysis has occurred, is the defining kinetic parameter for a substrate turnover event driven by catalysis; (d) free energy diagram for a uniform binding change. From the left panel to the right panel, ES and $TS_C$ are decreased in parallel and by the same amount. $\Delta\Delta Gb$ has increased although there is no catalysis noted. Hence, there is no net difference between the catalyzed and uncatalyzed states. This is the hallmark of a uniform binding change.

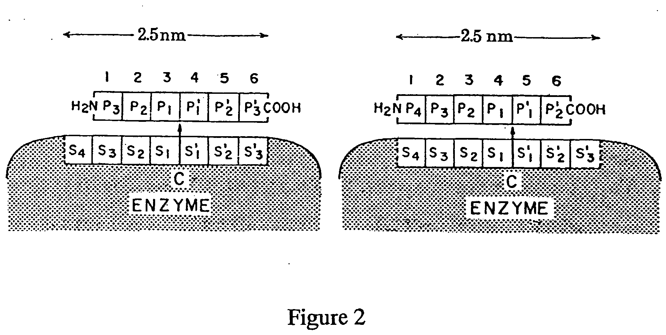

Figure 2. Standard nomenclature for residues involved in enzyme/substrate binding: S, amino acid on enzyme; P, amino acid on substrate; the scissile bond that is cleaved is between the P1 site and the P1'site on the substrate.

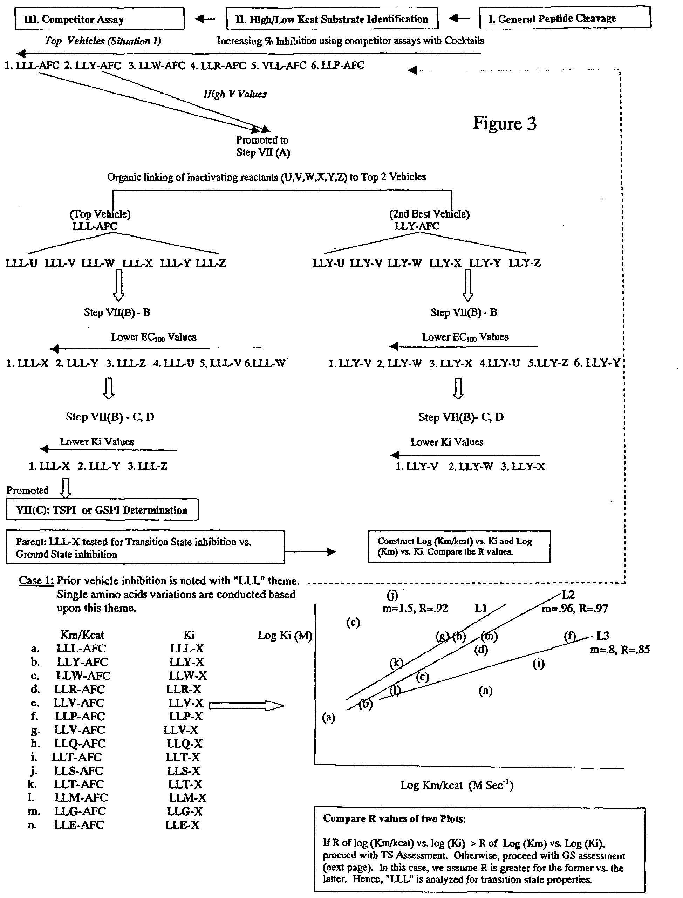

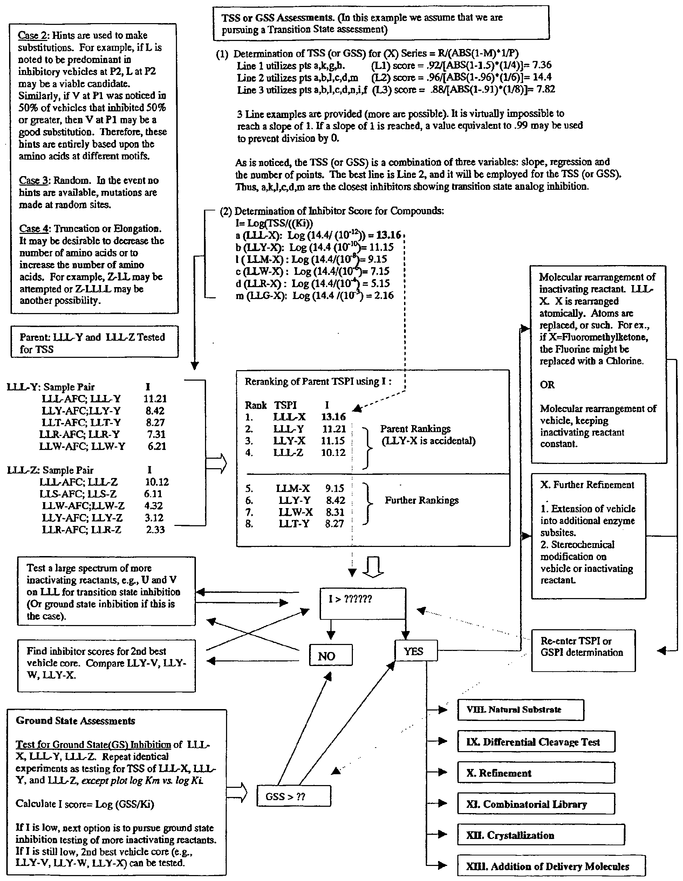

Figure 3. A schematic outline of selected steps of the method of the invention for identifying active site inhibitors of a target protease.

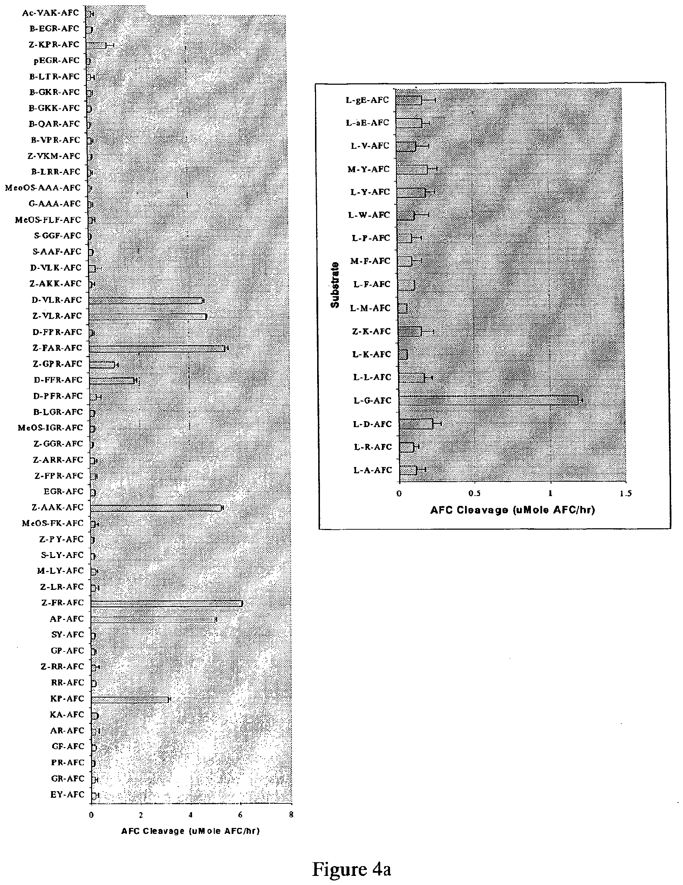

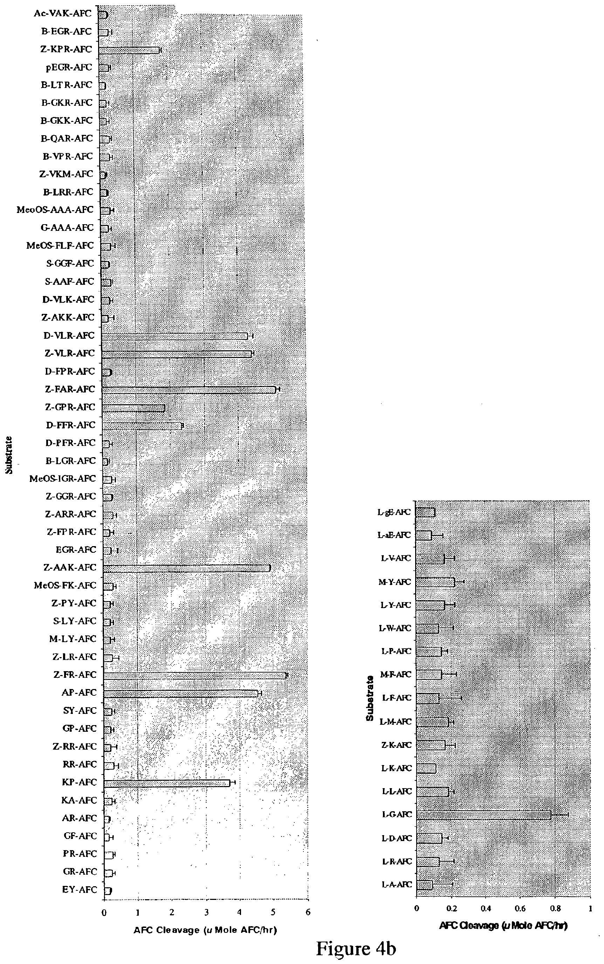

Figure 4. (a) Graphic representation of a substrate assay utilizing a panel of substrates and purified *Taenia solium* cysteine protease (Step II). Left panel depicts cleavage of AFC-linked tripeptides and dipeptides by the purified enzyme. Right panel depicts cleavage of single monopeptides by the purified enzyme. Each value is the mean of assays performed in triplicate. (b) Graphic representation of a substrate assay utilizing a panel of substrates with *Taenia solium* crude mix (Step II). Left panel depicts cleavage of AFC-linked tripeptides and dipeptides by the crude mix. Right panel depicts cleavage of monopeptides by the crude mix. Each value is the mean of assays performed in triplicate.

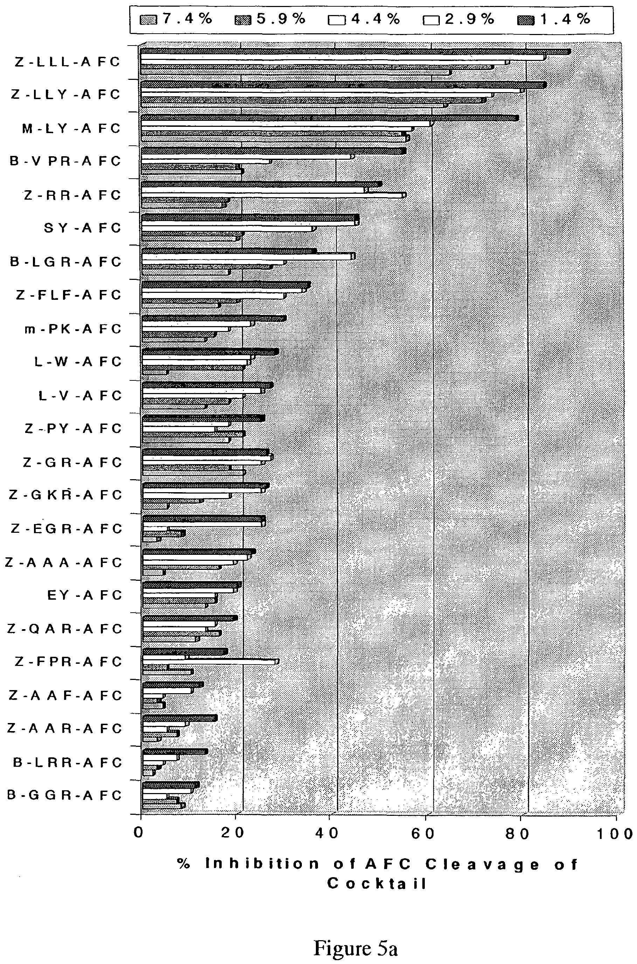

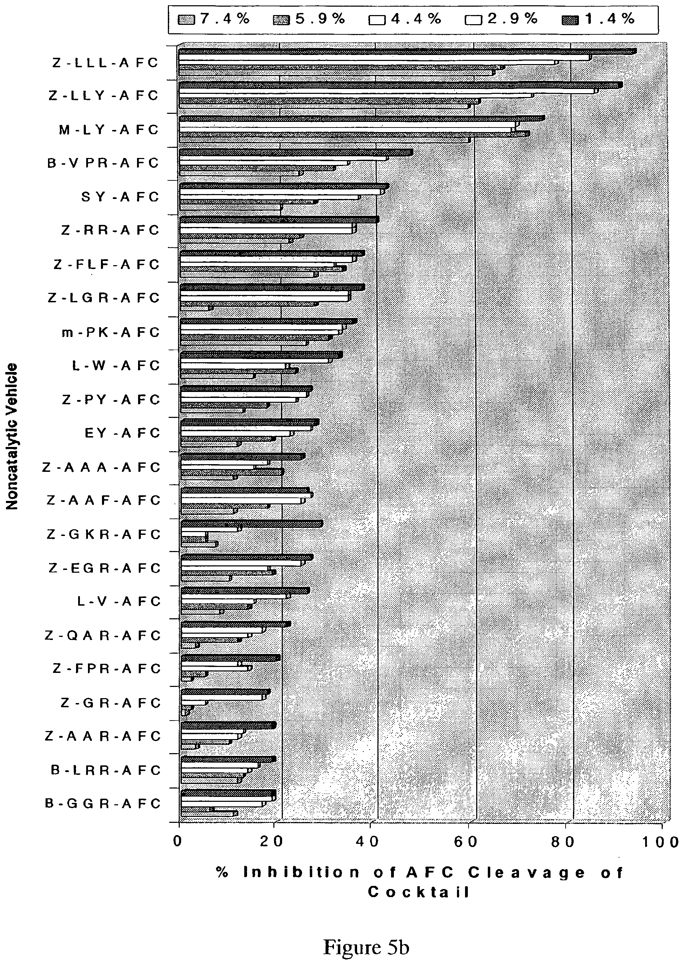

Figure 5. Competitor assay measuring percent inhibition of AFC cleavage of a cocktail with (a) purified *Taenia solium* cysteine protease and (b) *Taenia solium* crude mix (Step III). 1.4% cocktail = Z-FR-AFC, 2.9% cocktail = Z-FR-AFC + Z-FAR-AFC, 4.4% cocktail = Z-FR-AFC + Z-FAR-AFC + Z-AAK-AFC, 5.9% cocktail = Z-FR-AFC + Z-FAR-AFC + Z-AAK-AFC + AP-AFC, 7.4% cocktail = Z-FR-AFC + Z-FAR-AFC + Z-AAK-AFC + AP-AFC + Z-VLR-AFC.

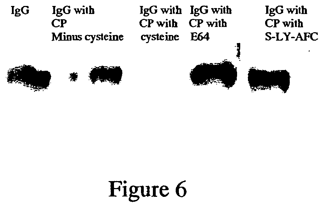

Figure 6. IgG degradation of heavy chain by purified cysteine protease of *Taenia solium.* Human IgG was incubated for 18 hours with *T. solium* purified cysteine protease (CP). Incubated tubes were subsequently fractionated by SDS-PAGE under reducing conditions, blotted onto PVDF membrane, and visualized using biotinylated anti-IgG (heavy chain/light chain), biotin-peroxidase conjugated-strepavidin complex and enhanced chemiluminescence. Bands represent heavy chains. The absence of heavy and light chains (light chain bands not shown) suggest that the proteins are broken down into peptides.

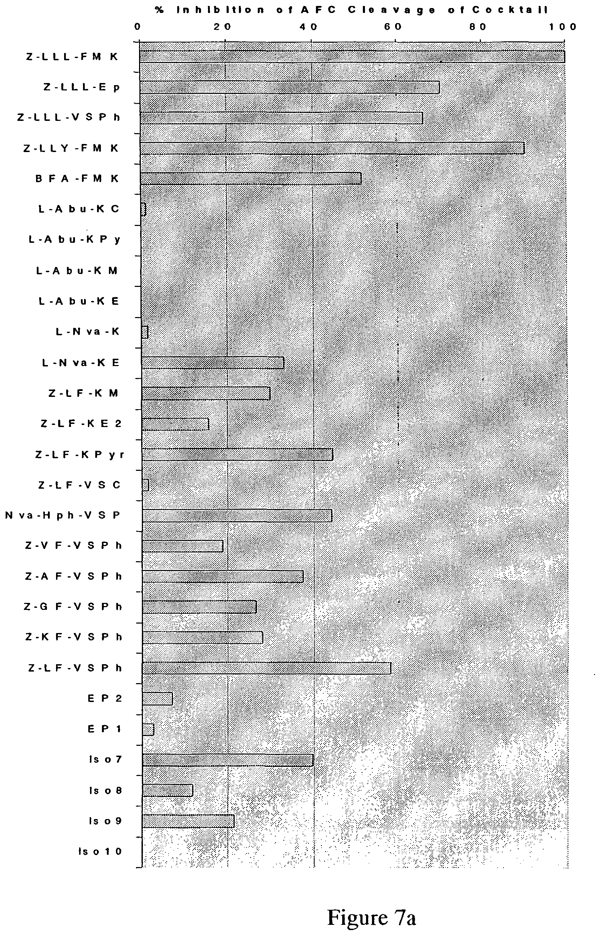

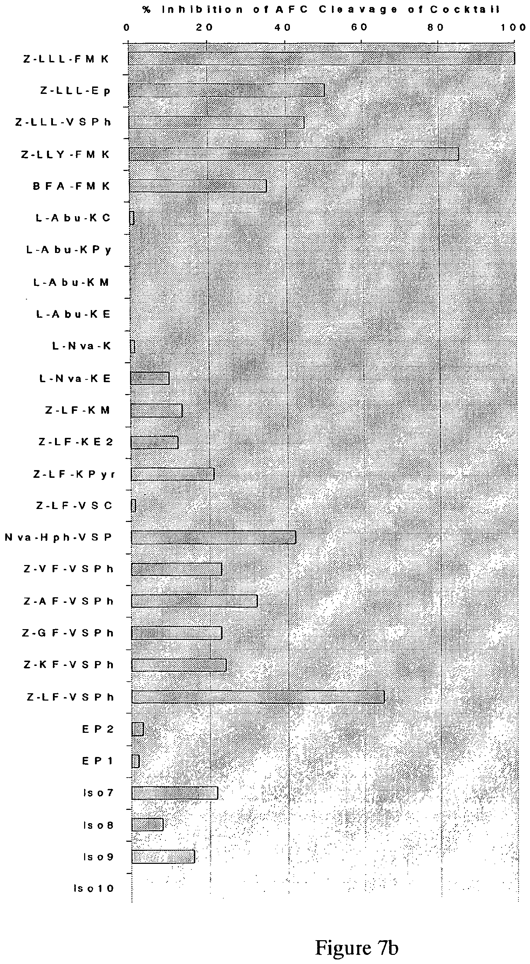

Figure 7. Inhibitor profile for (a) purified *Taenia solium* cysteine protease, and (b) *Taenia solium* crude mix, using inhibitors employing a variety of inactivating reactants for the leading vehicle core hypotheses. Each value is the mean of assays performed in triplicate. Iso7, 3,4-dichloroisomethyl coumarin; iso8, 4-chloro-3-(4-f-benzyl) oxyisocoumarin; iso9, 7-EtNHCONH-4-Cl-3-OMe-isocoumarin; iso10, 7-(M-NO$_2$-C$_6$H$_4$)CONH-4-Cl-3-OPr-isocoumarin.

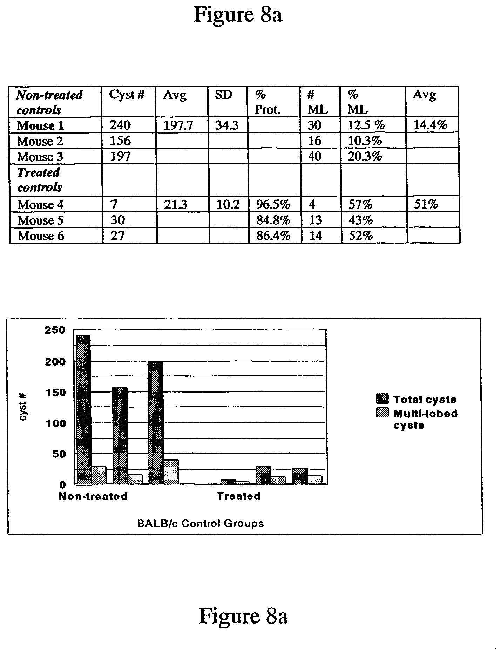

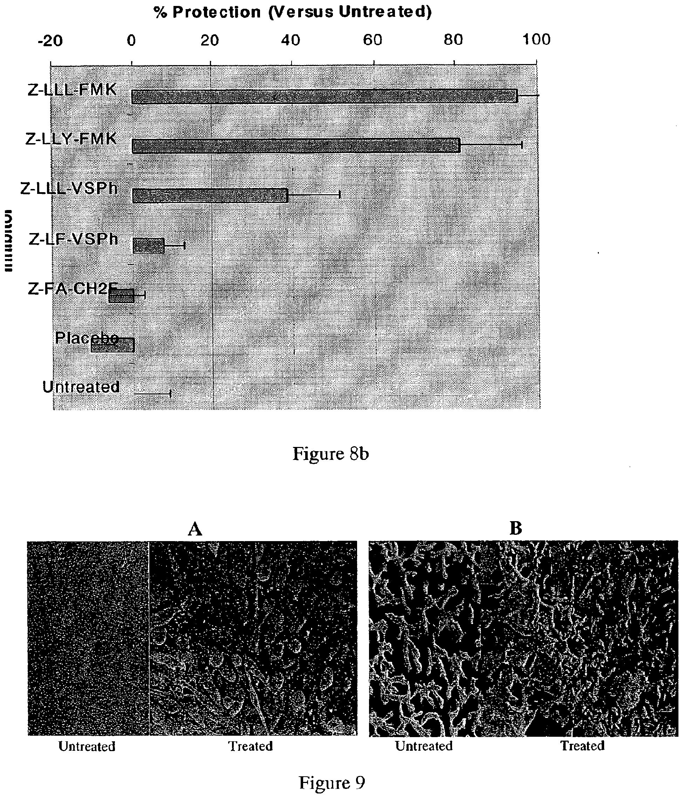

Figure 8. (a) Graphically shows the effect of Z-LLY-FMK treatment of BALB/c mice challenged with *Taenia crassiceps* cysts. Each treated mouse was pre-injected intraperitoneally with inhibitor for two days. During treatment phase, each treated mouse received 1.48 X 10$^{-2}$ $\mu$g/$\mu$l of N2 daily for three weeks and every other day during the last week. Injections were carried out in 150$\mu$l of dimethyl sulfoxide for each mouse. Both treated groups and positive controls were challenged intraperitoneally with 10 *Taenia crassiceps* cysts in 200$\mu$l 0.15M PBS at the time of initial infection. After one month of infection, both groups were euthanized and cysts removed by washing with sterile 0.15M PBS. A minimum of two attached cysts were scored as multilobed (ML). Upon visual inspection, no mice showed effects of inhibitor toxicity. (b) Graphically represents the effect of protease inhibitor treatment of BALB/c mice challenged with *Taenia crassiceps* cysts over a long-term period. Mice were divided into treated and untreated groups (5 mice/group). Each mouse in the treated group was pre-injected intraperitoneally with inhibitor for two days. Subsequently, all groups were challenged with 10 *T. crassiceps* cysts in 200$\mu$l of 0.15M PBS. Treatment with inhibitor or placebo (0.15M PBS) was carried out every day for 30 days post-challenge. Cysts were left alone (untreated) for 5 months, and then euthanized. Cysts were then counted by visual inspection. Percent protection is based on the reduction of the total cyst number in comparison to control mice, which never received any treatment.

Figure 9. Scanning electron microscopy of the surface of cysts removed from untreated mice versus cysts removed from treated mice. Magnifications are identical for both slides in each panel. Panel A (4000x). SEM shows a vigorous host immune response on cysts treated with LLY-FMK. No immune cells were seen on cysts removed from untreated mice. Panel B (25,000x). SEM results show fibroblasts, holes in the tegument, and sloughed microtriches in cysts from treated mice. Microtriches in untreated cysts can be seen to be visibly longer. Visible anchors were also noted for the untreated cyst group, whereas these anchors were not observed in microtriches from treated cysts.

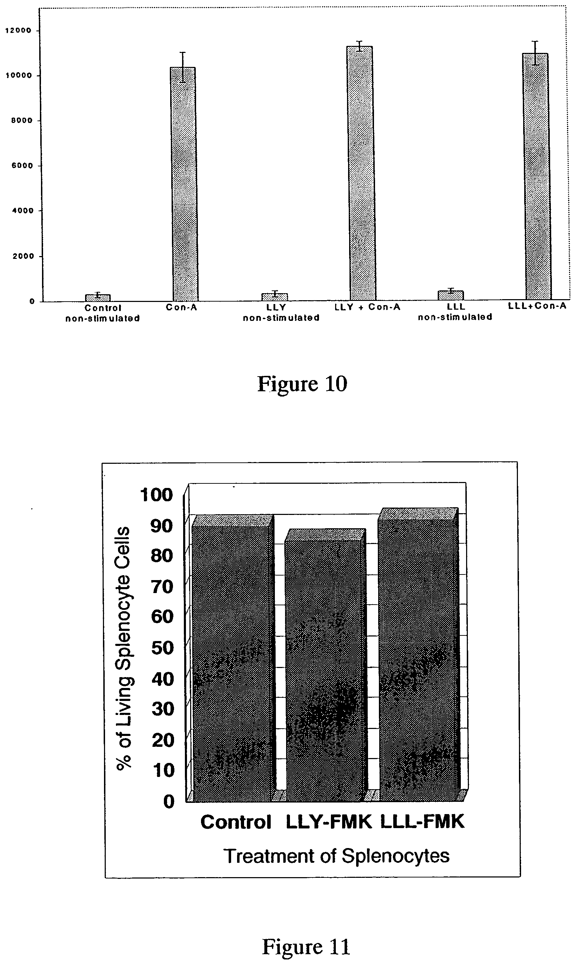

Figure 10. Graphically shows that the stimulated response of mouse splenocytes to Con-A is unaffected in the presence of the protease inhibitors Z-LLY-FMK and Z-LLL-FMK. The y-axis denotes cell proliferation in counts per minute.

Figure 11. Graphically shows the effect of inhibitors after 96 hours of incubation on BALB/c splenocytes as measured by Trypan Blue uptake.

## DETAILED DESCRIPTION OF THE INVENTION

*PART I. THEORY*

*A. Models of Enzyme Catalysis*

The method for protease inhibitor development provided by the invention is based on an unlikely and novel integration of a number of different assumptions derived from several disparate theories of enzymatic catalysis. Some of our major assumptions are drawn from the "split-site" enzyme model, formulated by Menger (1992) and later advanced by Murphy (1995), which predicts that an enzyme's active site is composed of discrete and separate binding and reaction centers. This concept was first advanced in 1950 by the proposition that acetylcholinesterase is characterized by a binding site composed of an anion which binds to acetylcholine's quaternary ammonium group and a reactive ester-specific site which causes the actual ester hydrolysis event (Bergmann et al., 1950). Although the split-site theory is an idealization for describing the events of the enzyme/substrate (ES) interaction, it provides a useful framework upon which protease inhibitors may be designed. In this way, the "split-site" theory is superior to the popular theory known as the "fundamentalist" position of enzyme catalysis (Schowen 1978).

*The "Fundamentalist" Position of Enzyme Catalysis*

The "fundamentalist" position of enzyme catalysis postulates that Pauling's account of (Pauling 1946, Pauling 1948) transition state (TS) stabilization is the primary mechanism for enzyme catalysis. Indeed, transition state theory has been in existence for almost 60 years, and has its roots in the accurate prediction of gas-phase reaction rates for diatom-diatom reactions as well as bimolecular atom-diatom reactions even when tunneling corrections are included. (Kraut, 1988). There are two important assumptions to transition state theory. First, the decomposition of the transition state controls enzymatic rate and second, the transition state complex is in equilibrium with the reactants. The basic fundamental equation for transition state theory is $k = k\nu K$, wherein k is the rate constant observed under experimental conditions, $k$ is the transmission coefficient, $\nu$ is the normal mode oscillation frequency of the transition state on the reaction coordinate, and K is the equilibrium constant for transition state formation from reactants (Kreevoy and Truhlar, 1986).

Two profiles are relevant for transition state stabilization (Menger 1992), and these will be referred to throughout. In the first profile (Profile I-Figure 1a), the concentration of substrate (S) is lower than the Km of the enzyme (E) for the substrate (S), such that the apparent binding energy of the enzyme/substrate complex (ES) is lower (higher Km) than that of E+S. Hence, the free energy of ES is higher than the free energy of E+S. In the second profile (Profile II-Figure 2a), the concentration of substrate (S) is higher than the Km of the enzyme (E) for the substrate (S) such that the apparent binding energy of ES is higher (lower Km), and the free energy of ES is thus lower than the E+S level.

An important assumption in both cases is that a tighter complex between E and S will lead to a lower free energy of ES (the ground state). Based upon these models, the "fundamentalist" position (Schowen, 1978) on catalysis is that the tightness of an ES complex will either have no effect in a Profile I scenario (since ES is always higher than E+S, it does not influence catalysis as ΔGcat is not influenced) or that it will decelerate catalysis in a Profile II scenario, since the overall free energy for catalysis is increased (ΔGcat is increased). Hence, the fundamentalist theory predicts that ground state reactant interactions are wasteful (Profile I) and can even hinder the catalytic event (Profile II) in some instances. However, there is a key flaw in this theoretical observation (Murphy, 1995) since there are numerous examples of tight ground state interactions between ES which are known to be beneficial to catalysis.

*Split site model*

Menger's (1995) "split-site" model is an advance over the classical fundamentalist (Schowen, 1978) theory. It offers an elegant proposition that rectifies the flaw in the incorrect prediction that tight ground state interactions are wasteful, and therefore explains the paradox in the fundamentalist theory. The problem with the fundamentalist position is that it treats binding and reactivity between ES as taking place in one spatial "center". The essence of the "split site" theory is conversely that ground state (ES) and transition state (TS) interactions can be described as the sum of the energetics which proceed between a discrete *binding* region ($ES_B$ and $TS_B$) of the enzyme as well as a discrete *reactive* region of the enzyme ($ES_R$ and $TS_R$). In other words, the enzyme can be described as a "split-site" wherein the free energy of ES equals $ES_B + ES_R$ and the free energy of TS equals $TS_B + TS_R$ (with $_B$ and $_R$ representing the independent binding center and reaction center, respectively). The energy of ES dictates Km. The conversion of binding energy into catalysis dictates kcat. It is to be noted that as used herein, the terms "E+S", "ES", and "TS" may refer to the physical entity of those species or the free energy of those species on a reaction coordinate, depending upon the context.

Several assumptions underlie the split-site model. First, energetics at the $ES_B$ centers in the ground state and transition state levels are always stabilizing and attractive (e.g., hydrogen bonding, Van der Waals and ionic forces). Second, energetics at the $ES_R$ center are always destabilizing (e.g., due to expensive energetic tasks like desolvation, strain from enzyme conformational changes to surround the substrate and changes in strain due to "substrate bending"). The catalytic groups are located in the reaction center. Since no reaction takes place between the catalytic groups at the ground level, $ES_R$ can only be dominated by destabilizing effects and a consequent increase in free energy. $ES_B$ is, as a consequence, always at a lower energy than $ES_R$. Third, the stabilizing forces in $ES_B$ are conserved in $TS_B$. This does not imply that the *strength* of the stabilizing forces are the same between $ES_B$ and $TS_B$. (Indeed, numerous examples in the art demonstrate that the forces at $ES_B$ are strengthened at $TS_B$). However, it is the *origin* and *destination* of the forces originating at $ES_B$ that are conserved at $TS_B$. Indeed, various examples of this type of conservation are known in the art.

For example, both cysteine (Kamphuis, 1984) and serine proteases (Polgar, 1988) employ an oxyanion hole. This structure serves an important binding function for the substrate P1 carbonyl in both the ground state at $ES_B$ and the transition state at $TS_B$. X-ray crystallography has shown that many of the important binding contacts made at the ground state are indeed shared at the transition state in many enzymes. Indeed, the contacts at $ES_B$ and $TS_B$ as well as their conservation are of prime importance, since specific structural characteristics conferred by them may be a source for exploitation by an inhibitor.

In another example, a unique chirality is present at $TS_B$ via the formation of a tetrahedral adduct which forms as a result of displacement reactions at the substrate $sp^2$ hybridized carbon atoms (Wolfenden, 1999). The bonding interactions that are the basis for this chirality begin via weak contacts formed at $ES_B$, but they do not emanate from E+S or from the products. Based upon the discussion above, it is notable that the geometry of the atoms of the substrate, while it is complexed with the enzyme (these atoms providing binding interactions), is not the same as the geometry of the substrate while it is free in solution. Thus, the binding interactions, as well as their consequent geometry, are created at $ES_B$ and subsequently, shared (and strengthened) at $TS_B$. Hence, the importance of "binding" is manifested in both binding interactions as well as their implicit geometry- both defining the concept of "conservation" at $ES_B$ and $TS_B$ as it is presented here. Indeed, it is such structural features that contribute to an enzyme's ability to discriminate between a free substrate and one that is undergoing strain to form ES as well as TS.

The significance of these observations is that a potential inhibitor must not only take advantage of the binding forces themselves, but also the *geometry* of the atoms composing those binding interactions in ES. As discussed below, we believe that a key flaw in the traditional paradigm of inhibitor development is that it does not allow full exploitation of these binding interactions.

The alternative corollary to the previous observations is that destabilizing interactions at $ES_R$ are not conserved at $TS_R$. For example, events such as desolvation at $ES_R$ have already occurred before the transition state at $TS_R$ is reached. Given this, since $TS_R$ is the location where catalytic groups will actually react, we would expect that $TS_R$ would be stabilizing for a highly cleavable substrate analog, with the lowering of its energy leading to the enhancement of catalytic rate. The opposite consequence (inhibition) is expected if $TS_R$ is destabilized by a destabilizing force such as an electrophile on an enzyme inhibitor (see Step VII for a description of such electrophiles). The main point here is that the actual catalytic event does not occur in the ground state (e.g., the catalytic triad of cysteine and serine proteases only reacts in $TS_R$, not $ES_R$), and often, a transition state level inhibitor will exert its effects by counteracting the stabilization of $TS_R$.

The preceding discussion of the split-site model of enzyme catalysis identifies the framework for our argument, presented below, which postulates a significant weakness in the traditional design of protease inhibitors. It further provides a foundation for how the inhibitor design of the present invention overcomes this shortcoming.

*Additional Relevant Principles of Enzyme Kinetics*

*Kcat, Km and Kcat/Km*

Given that there are multiple ways to view enzyme catalysis, it is important to review assumptions regarding the values of kcat, Km and Kcat/Km as they apply to our defined framework for kinetics. Kcat, a first order rate constant, is defined as the maximum number of substrate molecules converted to products per enzyme active site per unit time, or the number of times the enzyme turns over per unit time. On the transition state plot, kcat is simply the difference between the highest and lowest points on an energy diagram. Thus, in the case of Profile I kinetics (where S<Km), it is the difference between the energies of E + S and TS whereas in Profile II kinetics (where S>Km), it is the difference between the energies of ES and TS. Kcat/km is the apparent second order rate constant referring to the concentration of free rather than total enzyme at low substrate concentrations in which Km>S such that the reaction rate, v, equals E ∗ S ∗ kcat/Km. This result holds at any substrate concentration therefore and is significant since it refers to the properties of the reaction of free enzyme and free substrate. Moreover, the value of kcat/Km cannot be greater than diffusion control ($10^8$-$10^9$), which is typically characteristic of "optimally evolved" enzymes (Albery and Knowles, 1977). Catalytic rate is described by a third constant that refers to the moles of product produced per mole of enzyme/second. Traditionally, it is understood that the maximization of kcat/Km parallels the maximization of rate (Fersht, 1985).

There are three major assumptions for kcat/Km (Fersht, 1985):

*1. High kcat/Km values designate complementarity of the enzyme to a transition state*

A high kcat/Km is a reflection of maximal *complementarity* of the enzyme towards the transition state of the substrate. This is presumed to be the case especially in situations where transition state stabilization is the major mode of enzyme catalysis. (In the opinion of many, ground state destabilization should also not be ignored as a mechanism for catalysis)

*2. kcat/Km is a specificity constant*

The second assumption is that kcat/Km is a *specificity* constant, such that a high value designates a high specificity towards a given substrate. In this context, "specificity" refers to the ability of an enzyme to discriminate between different substrates. Whereas large substrates can be discriminated simply by their steric bulk, the discrimination of smaller substrates is a more challenging task and is a function of both binding (Km) and turnover (kcat). Simply put, as it is understood in the art, neither of these kinetic parameters, alone, will suffice for a characterization of specificity, but their combination will.

In order to be "specific", an enzyme must be capable of distinguishing structural features on a substrate that are unique to its activated form (e.g., its transition state). Traditionally, it has been understood that an ensemble of subtle collective structural differences between the substrate in its ground state and the substrate in its transition state are employed by the enzyme as a distinguishing mechanism for specificity. It follows that an effective inhibitor may potentially exploit that collective ensemble, but how it does so is entirely unknown (Wolfenden, 1999). The location of the structural differences may be in non-active site positions as well as those that are near or at the active site. We suggest, based upon the split-site model that, the structural differences collectively contribute to the binding energy at $ES_B$ which is conserved and strengthened at $TS_B$. Consequently, an inhibitor should be characterized by the ability to exploit the binding forces which are conserved between $ES_B$ and $TS_B$. However, since this entire binding force "ensemble" is highly connected, it follows that the event of enzyme binding is also intricately connected to the events in enzyme reactivity (unlike the split-site model would suggest, as will be discussed below). Later we will discuss how, in many instances, a single enzyme residue will contribute to both binding and reactivity, such that the functions of binding and reactivity are not as disconnected as is postulated by the split-site model.

However, the major point here is that due to the interconnected nature of these *ensemble* of interactions (Ma et al., 2000) in both the binding and reactivity centers it is difficult to simultaneously take advantage of the events of binding and reactivity. Indeed, it is rare to find an inhibitor which is capable of exclusively preventing the lowering of the transition state (reactivity function), without disrupting the favorable binding energy of $ES_B$ and $TS_B$ (binding function). This may be a reason why historically it has been difficult to produce a transition state inhibitor of high quality. Moreover, as will be seen later, we postulate that the specific manner by which this complexity arises comes from the addition of a "reactive moiety" to the tight binding peptide core (a traditional procedure in the construction of transition state analogs).

*3. Optimally evolved enzymes with high rates are characterized by a high kcat and a high Km*

A third assumption has been that the maximization of kcat/Km typically parallels maximization of enzymatic rate. Although this is typically the trend in most enzymes, it is not always the rule. For example, control enzymes such as those in metabolic pathways are more likely to be marked by a low Km since it allows the enzyme to quickly enter the metabolic pathway so that it can control the rate of entry and prevent the pathway from being overloaded with reactive intermediates (Fersht, 1985). However, for most enzymes, it is understood that for an enzyme with a given kcat/Km and a given constant substrate value, the enzyme with the highest respective values of kcat and Km will have the highest rate whereas the converse is true for the enzyme with a low kcat and a correspondingly low Km (Fersht, 1985).

Fersht (1985) articulated several principles about the evolution of a target enzyme. These principles are entirely theoretical, but provide a useful framework upon which to base several predictions made below. First, it is assumed that an enzyme is evolving toward teleological maximization of its catalytic rate; i.e., the primordial enzyme evolves toward obtaining maximal complementarity toward the transition state of the target substrate and hence, maximal specificity toward the target substrate. To this end, a high kcat/km of the enzyme for the target substrate is the first step. In this mode, it is argued by Fersht (1985) that enzymes begin with a Profile II-like scenario in which Km is low (Km<S) and kcat is low for the given kcat/Km parameter. Using the assumptions of the split-site model, it can be assumed that the reduction in $ES_B$ is less than the increase in $ES_R$, which leads to low Km. Consequently, a low $ES_B$ leads to a low $TS_B$. However, although $TS_B$ is low (as would be predicted for maximal complementarity of the enzyme for the transition state of the substrate) $TS_R$'s value is not highly beneficial for catalysis, which leads to a low kcat of the enzyme. Hence, in the primordial enzyme, the mediocre conversion of intrinsic binding energy at ES is what causes the rather low catalytic rate. Fersht (1985) argues that this may be due to the possibility that a low Km causes a "thermodynamic pit" from which the reaction must "climb" out, whereas this is not the case for an enzyme with a high Km toward a particular substrate. Consequently, the main points for a primordial enzyme are that 1) Km is more important than kcat and 2) that an enzyme which is marked by a low kcat and low Km is conceivably in a nascent state of evolution.

In the second phase of evolution, the enzyme will evolve to increase its catalytic rate, while maintaining its kcat/Km ratio for the target substrate. Thus, the enzyme maintains a constant specificity for the enzyme as well as a maximal complementarity for the transition state (constant kcat/Km), but individually raises both Km as well as kcat in a manner that keeps kcat/Km constant. The means by which both kcat and Km are increased can involve a variety of mechanisms.

For example, if we are to employ the principles of the split-site model, the raising of Km is manifested through an increase of the $ES_R$. This is based upon the assumption that $ES_B$ contacts would be expected to be maintained (and not increased) since they are catalytically favorable and are thus conserved at $TS_B$. Thus, we postulate that $ES_B$ and $TS_B$ contacts are maintained during the evolutionary stage, whereas the raising of $ES_R$ is the specific modification by which Km is raised. Several mechanisms are known in the art that may be employed to raise $ES_R$. One of these is known as "strain," a phenomenon that refers to the distortion of a substrate in the ground state, so that it will maximally fit into the transition state configuration. Although the major binding contacts are already established at the ground state and conserved in the transition state (discussed below) specific additional chemical groups on the substrate may be employed to further distort the substrate so that it will mold into its transition state form.

A second postulated mechanism for the increase of $ES_R$ is that of the famous "induced fit" model (examples in Fersht et al. 1988)). In this model, the enzyme (as opposed to the substrate) is being distorted at the reaction center (therefore increasing $ES_R$) so that it can fit around a rigid substrate. The chemical groups on the enzyme are used to provide energy for this distortion. Numerous examples of induced fit exist in the literature including observations noted in the first crystal structure transition state ever produced (in 1970), of triosephosphate isomerase. Structurally, it was found that the enzyme contracted along its major axis when an inhibitor was bound to produce the transition state, but it expanded when the inhibitor was released by dialysis (Johnson and Wolfenden, 1970). This flexibility in the enzyme's geometry is sensible since there are several transition states, and it would be advantageous for binding if the active site pocket could adjust as a result of minor shifts in the catalytic cleft.).

There are advantages and disadvantages to the induced fit theory. A disadvantage is that induced fit is employed to increase Km without a corresponding increase in kcat, and is therefore argued by Fersht (1985) to be non-beneficial to catalysis. Moreover, induced fit faces an enormous entropic bottleneck, as the enzyme must reduce the entropic barrier to reduce the free energy of activation. (Ma et al., 2000). However, a potential advantage to induced fit is that enzyme flexibility at the active sites allows the option of an enzyme to bind flexibly to a medley of transition states (Ma et al., 2000) rather than being restricted to precise binding of a single transition state configuration. This is a significant advantage, especially if the transition state, as discussed above, is actually the average of an ensemble rather than a single structure. Conversely, since "strain" helps by increasing positive reinforcements that will increase both kcat and Km and therefore provide increased catalytic rates, it is often considered to be the more important of the two $ES_R$ raising forces (Fersht, 1985).

Regardless of the mechanism by which Km is increased via an increase of $ES_R$, the most important point here regards the hypothetical conclusion that an optimally evolved enzyme shifts from its primordial Profile II kinetic scenario into a Profile I kinetic scenario (Km>S) through a venue which has also increased catalytic rate. This observation is important because the method of the invention was developed using the assumption that the target protease is in an advanced stage of evolution, which follows Profile I kinetics, and therefore has high Km values. Typically, the higher Km values are now in a range of 1 to 10 times S. (Kraut, 1988). This higher Km may be depicted as a Boltzmann distribution of substrate molecules that occurs before the transition state rate-limiting step, such that they are well populated due to similarities that occur between the transition state and ground state (Kraut, 1988).

In order to keep the value of kcat/Km constant (and therefore maintain specificity and complementarity of the enzyme towards the substrate's transition state), the increase of Km results in an equivalent increase of kcat. This increase of kcat in the evolved enzyme is conceivably achieved through the lowering of $TS_R$ (in reference to the $TS_R$ of the primordial enzyme.) The mechanism by which this would occur is not well understood and will not be explored here. With respect to catalysis, significant evidence demonstrates that the kcat of the evolved enzyme is more responsible for the rate enhancement than the Km value.

This observation appears to suggest that a weak binding between E and S is preferred (as is argued by Fersht, 1985) for maximal catalytic rate, thereby diminishing the importance of binding between E and S. However, the major point postulated here is that *Km is raised solely through the raising of $ES_R$* (due to strain, induced fit, or other forces) and not $ES_B$. An important assumption of ours is counterintuitive to the hypothesis that ES and therefore Km are high in an optimally evolved enzyme: *$ES_B$ and $TS_B$ still serve a major function as stabilizing forces,* even in an optimally evolved enzyme where kcat is the important variable for catalysis. Indeed, this observation is supported by several investigators in the art, who have demonstrated that tight binding interactions at the ground state are not wasteful to catalysis (Murphy, 1995, and Menger, 1992). This observation will be important to the method in this invention since the method exploits the binding interactions at $ES_B$ and $TS_B$.

To summarize this section: during evolution, specificity and complementarity of the enzyme to the transition state of the substrate were first maximized (via conserved binding contacts at $ES_B$ and $TS_B$) and then maintained, whereas catalytic rate was enhanced through the mechanism of raising $ES_R$ (raising Km) via distortion forces (strain or induced fit) causing a corresponding increase in kcat (such that kcat/Km remains constant and "maintained") which became the kinetic variable responsible for catalysis. However, the important point is that while kcat is more important than Km for catalysis in the optimally advanced enzyme, intrinsic binding energies at $ES_B$ and $TS_B$ are still conserved and very important for conversion into catalytic energy. We propose herein that it is these optimal energies that an efficacious protease inactivator should exploit.

*Relevance of Assumptions about Kcat/Km to the Method of the Invention*

The method of the present invention, described in detail below, is initially grounded in the previously discussed assumptions: first, the target proteases are in an advanced stage of evolution such that, second, their catalysis is driven by kcat, and therefore that third, high kcat substrates exist for their active sites. Optimal permutations of tripeptide, dipeptide, or monopeptide combinations are postulated to exist for a target enzyme at its S1-S3 binding sites (Berger and Schechter, 1970), which may be subject to a high turnover. Fourth, Km may not be as important for their catalysis (such that the enzymes are driven by Profile I kinetics), although the intrinsic binding energy of Km conferred by $ES_B$ and $TS_B$ is a very important component of the catalytic machinery. It follows that optimal permutations of tripeptides, dipeptides, and monopeptides exist that are conducive to binding. In its most robust embodiment, the method of the invention screens all possibilities of amino acid combinations at P3-P1 of a tripeptide substrate of the target enzyme (8,420 amino acids, including the dipeptides and monopeptides). We believe that tripeptides are sufficiently long, since it has been noted (Tsai and Jordan, 1993) through eigenmode search methods, that the number of first order transition state saddle points increases exponentially as the degrees of freedom of the transition state increase (the degrees of freedom increase as the cluster size is increased). The use of tripeptides allows the kinetics to remain simple while keeping the predictability of the method as high as possible. However, shorter or longer peptides can also be used.

*Assumptions about the Mechanism of Catalysis: Transition State Effects and Ground State Effects*

The assumptions and theories discussed up until this point can be employed to explain how enzymatic catalysis is driven. As noted, various theories, sometimes completely contradictory in nature, abound in the art regarding this topic. This section highlights which of those assumptions we have adopted, and which are challenged and potentially replaced by our new system.

The primary dogma in enzymology is that either transition state stabilization or ground state destabilization or a combination of both are employed to drive enzymatic catalysis. With respect to transition state effect (Figure 1b), the two most important scenarios among several possible are reviewed.

In the first scenario, $\Delta G_{ES}$ (and therefore Km, and the apparent ES binding energy) is constant in the enzyme reaction. Despite the constant Km, tight binding interactions do occur between ES. As will be recalled, since $ES_R$ must be destabilizing, $ES_B$ must be equally stabilizing to account for a constant Km. An important observation here is that $ES_B$ can still be lowered even when Km is constant. Moreover, since $\Delta G_{ES}$ is unchanged, all of the energy that is gained in the binding interaction from ES is completely converted into catalytic energy. The more important observation here is the mechanism by which this occurs: since the lowering of $ES_B$ is conserved in $TS_B$ (as discussed above via the assumption that binding interactions and geometry are conserved and strengthened at $TS_B$), the net energy of TS is also lowered. This lowering of $TS_B$ is the first major contribution to catalysis.

The second major contribution to catalysis derives from the reaction via the enzyme's catalytic centers, which lower $TS_R$ and therefore contribute to a lowered TS. As a corollary of this, it is important to recall that the increase of $ES_R$ does *not* translate into an increase of $TS_R$, since the energetics of the reaction centers in the ground state are not conserved in the transition state. The net result of these reactions is that the catalyzed transition state (TS) is lowered in comparison to the uncatalyzed transition state ($TS_U$), which results in a lower activation energy that equates to an overall increase in -$\Delta$Gcat (increased kcat/Km). As has been assumed in previous discussions, the kcat/Km variable is predictive of an enhancement of catalytic rate.

Consequently, an increase in $\Delta$Gb (the free energy of enzyme binding to the transition state) is postulated by Murphy (1995) to be the defining parameter for identification of a transition state stabilization effect (Fig. 1b). The most important conclusion from this first scenario of transition state stabilization is that it reconciles the paradox and first major flaw in the "fundamentalist" theory as to how ground state interactions are not wasteful, and demonstrates how they actually participate in accelerating catalysis. *We therefore emphasize again that binding interactions at $ES_B$ and $TS_B$ are very important to catalysis and that an efficacious inhibitor should maximally exploits these forces.*

A second scenario for the transition state effect especially highlights how strong ground state effects help catalysis rather than hindering it. Here, despite the lowering of total ES (low Km) due to tight binding between ES ($ES_B$ is thus lowered more than $ES_R$ is raised), the catalytic rate still increases. Again, this situation explains how ground state interactions can actually be beneficial to catalysis, contrary to fundamentalist theory. Again, the reason for the rate acceleration is that $ES_B$ is conserved at $TS_B$. Excluding any reduction in $TS_R$, the net result is that TS is reduced more than ES is reduced, resulting in an increase in $\Delta$Gb and a consequent increase in the enzymatic rate.

Hence, some enzymes are also postulated to also be driven by a ground state destabilization effect that operates independently, or in conjunction (Albery and Knowles, 1977); Jencks, 1987) with transition state stabilization. One example of this effect where ground state destabilization acts independently (Fig. 1c) is the case where $\Delta G_{ES}$ is increased (and therefore Km is increased, and the apparent ES binding energy is decreased) due to an increase in $ES_R$ while $ES_B$ is constant. Consequently, $TS_B$ is constant due to conservation at $ES_B$. $TS_R$ is also constant, which results in no net change in the energy of TS (which remains constant). Despite the fact that TS has not been lowered, there has still been a decrease in the activation energy required to reach the transition state which equates to an overall increase in -$\Delta$Gcat and increased rate (as predicted by kcat/Km). In this instance, demonstration of a constant $\Delta$Gb with increased catalytic rate, is the definition (Murphy, 1995) of a ground state driven catalytic event. The point here is that, contrary to the fundamentalist notion that transition state stabilization serves as the sole catalytic mechanism for rate enhancement, the ground state destabilization effect provides an alternative and viable strategy for catalysis.

A note will now be made about the preference for Km in either of the above scenarios. Essentially, a high Km will translate into a high ES which will lower the activation energy in the mode of ground state destabilization whether transition state stabilization is inoperative, operative independently of ground state destabilization, or operative in conjunction with ground state destabilization. Again, this paradigm is consistent with the one presented above for optimally evolved enzymes. To review, for these enzymes, $ES_B$ (and $TS_B$) should be low, whereas $ES_R$ should be raised such that ES and Km are high. Kcat drives catalysis. We conclude that an inhibitor which lowers Km through the venue of taking advantage of binding contacts at $ES_B$ and $TS_B$, but with the additional capability of lowering $ES_R$, provides the potential to counter rate enhancement *in both cases where either a transition state stabilization effect drives catalysis or where a ground state destabilization effect drives catalysis*

Finally, the neutral effect of "uniform binding" (Fig. 1d) merits brief mention. In this case, the reduction in TS parallels the same change in $\Delta$Gc, a scenario which results in no net catalysis. In this instance, $ES_B$ is decreased at constant $ES_R$, which results in an equal decrease of $TS_B$ causing both ES and $TS_C$ to be decreased by the same value. The same scenario applies where $ES_B$ is increased at constant $ES_R$. Consequently, none of the intrinsic binding energy is converted into catalysis and there is no change in the $\Delta$Gcat and hence no change in the rate. Therefore, a net change of zero in $\Delta$Gb is the definition of uniform binding (Murphy, 1995).

To summarize, enzymes can follow a pathway where there is no net change in catalysis through means such as uniform binding, or can be catalyzed through either transition state stabilization or ground state destabilization effects. These mechanisms underscore the enormously complicated nature of enzyme kinetics in that substrate turnover can proceed through different mechanistic routes perhaps as a variation or as a combination of the above described mechanisms. Which route is actually followed depends upon the specific enzyme. An awareness of these possibilities is relevant to the custom-design of a protease inhibitor that must work to counteract mechanistically some or all of the possible pathways that are favorable to catalysis. Consequently, the assumptions provided for ground state destabilization and transition state stabilization will be employed in order to provide a basis for the prediction of kinetic phenomena resulting from the protease inhibitor identification method of the invention.


*The Reaction Coordinate*


Based upon the previously outlined assumptions, the reaction coordinate for catalysis will now be described. This analysis applies in situations wherein transition state stabilization is the major mechanism that drives catalysis. Ground state destabilization will be treated in a later discussion.

The catalytic mechanism begins with the binding event between enzyme and substrate to form the Michaelis complex (ES). We assume that enzyme/substrate binding results in a lowered $ES_B$. Two possibilities are that ES can either stay the same such that Km is constant ($ES_B$ is decreased as much as $ES_R$ is increased, which equates to a constant Km, requiring that kcat be high) or can be lowered such that Km is lowered ($ES_B$ is decreased more than $ES_R$ is increased). In both instances, we argue *that it is essential that $ES_B$ is lowered to insure a tight binding between enzyme and substrate so that this complex can proceed to a preliminary transition state and eventually to a rate determining step transition state.* During this process, whereby the initial reacting groups make their preliminary associations, there is a significant entropy loss because of a reduction in the degrees of freedom.

The second major event in catalysis is the postulated formation of the transition state. $TS_B$ is low (due to conservation of the binding energies at $ES_B$). This either leads to or is concomitant with $TS_R$ being lowered because of the favorable catalytic event resulting from subsequent interaction of catalytic residues. Consequently, TS is lowered. If TS is stabilized more than ES is stabilized at the ground state, $\Delta$Gb is increased which means that the enzyme is employing transition state stabilization to move catalytically forward with the high kcat/Km target substrate (Fig. 1). A conclusion that can be drawn is that the enzyme utilizes specific binding energy from the formation of ES in order to overcome the strain and loss of entropy, which occurs because of the raising of $ES_R$ (Jencks, 1987).

To emphasize that tight binding between enzyme and substrate is necessary for successful catalysis, we reiterate that $ES_B$ is always lowered whether or not this results in the lowering of Km. There are two key reasons for this: 1) lowered $ES_B$ is the first event which will cause ES to proceed toward the transition state, which is required if transition state stabilization is to occur at all and 2) the tight bonding at $ES_B$ needs to be continued, if not improved at $TS_B$, such that the catalytic residues can subsequently react and cause the lowering of $TS_R$ and eventually TS by an amount greater than the total lowering of ES. *We now postulate that the maintenance of the quality of the lowered $ES_B$ variable (and thus the favorable binding contacts at $TS_B$) is one of the key shortcomings of the traditional protease*

*inhibitor discovery model.* We will elaborate upon this argument in a later section, but begin with an overview of the traditional protease inhibitor screening model.

*Traditional Design of a Transition State Analog*

A "transition state analog" is a molecule that "resembles" the transition state of the substrate, such that the enzyme will bind to it with an affinity that is significantly greater than its affinity for the free substrate. The Kurz equation describes binding efficacy of a transition state analog: $k_e/k_u$ = $K_S/K_T$ whereas $k_e$ is the rate constant for the catalyzed reaction; $k_u$ is the rate constant for the uncatalyzed reaction; $K_S$ is the substrate dissociation constant; $K_T$ is the transition state dissociation constant. Therefore, if a $10^{12}$ rate is observed for enzyme catalysis, it follows that the enzyme binds to the transition state analog with an affinity that is $10^{12}$ times greater than the affinity with which the enzyme binds to the ground state substrate. (It is important to note that Kurz equation predictions deviate for enzymes that also employ ground-state destabilization as a strategy for rate enhancement).

A traditional and common method of engineering transition state analogs is to attach a reactant molecule to a natural substrate analog that mimics the transition state of the enzyme. Consequently, binding of the transition state analog will render the active site "locked," and consequently incapable of proceeding further down the "energy hill" to the creation of products. In this method, an analog with a high specificity (high kcat/Km) for the target enzyme is first identified. Several excellent high throughput methods (e.g., gene chip) exist which identify such substrate peptide sequences that are conducive to a high turnover by the target enzyme. Next, a reactive moiety is attached to the high kcat/Km peptide core in order to produce a final candidate inhibitor, which will purportedly increase $TS_R$ (based upon the assumptions we have outlined above). As a result, the favorable binding events which lower $TS_B$ are not great enough to counteract the increase of $TS_R$ and hence, $TS_1$ (transition state with inhibitor) remains unchanged or increased. The net result is that $\Delta Gb$ is not increased and there is no rate enhancement.

Several kinetic scenarios are possible that may account for a lack of rate enhancement. 1) Although $TS_B$'s decrease is greater than the increase in $TS_R$, it is not sufficient to cause the net $TS_1$ (transition state with inhibitor) to be lowered enough relative to the lowering of ES such that an increase in $\Delta Gb$ results. 2) $TS_R$ is increased by the same amount that $TS_B$ is lowered such that $TS_1$ remains at the same level as $TS_U$ (transition state, uncatalyzed) and again, $\Delta Gb$ is not increased. 3) $TS_R$ is increased more than $TS_B$ is lowered such that $TS_1$ is greater than $TS_U$ and $\Delta Gb$ becomes positive. We postulate that the lack of rate enhancement observed for conventionally designed transition state analogs occurs when any of the following assumptions for inhibition are met:

> 1. $TS_1$ is never lowered enough that $\Delta Gb$ can be increased, so that the common transition state stabilization effect is in fact, eliminated.
> 2. $TS_R$ is always increased in order to counter the lowering of $TS_B$.
> 3. $ES_B$ is always decreased so that ES can bond and approach a transition state (where inhibition will occur)

We propose that any strategy for protease inhibitor development should meet these criteria.

*Introduction to the Theoretical Development of the Method of the Invention*

As noted above, the "split-site" model represents an advance over the "fundamentalist position." It successfully predicts transition state stabilization-driven (and ground state destabilization-driven) catalysis. In a marked departure from conventional methods of inhibitor design, the present invention employs the "split site" model as the starting point for generating candidate inhibitors.

As a theoretical foundation for inhibitor design, the "split-site" model has several advantages. The model's discrete and separate binding and reaction centers allow for simplified kinetics and calculations. Interestingly, the model's simplicity correlates to better kinetic predictions and allows for the discovery of a successful inhibitor in an efficacious and expeditious manner.

It is our view that the traditional inhibitor development model fails because it does not emulate the "split-site" model. Unfortunately, however, the "split site" model cannot be utilized without modification due to a major problem: it is an oversimplification of enzyme catalysis. In reality, the binding and reactivity centers are not as disconnected as the model presupposes (we offer evidence below). We have devised a way to overcome this obstacle and show that it is possible experimentally to design a peptide scission enzyme inhibitor identification method wherein the split-site model's assumptions are nevertheless as closely upheld as possible. Significantly, the method of the invention *experimentally* drives the process of inhibitor design or identification toward inhibitory compounds that demonstrate tight binding to the target enzyme as well as apparent specificity for a particular configuration of the enzyme that is associated with catalysis, in keeping with the basic *theoretical principles* of the split-site model. Our novel method of inhibitor design and identification thus "rectifies" the major flaw in the traditional method for inhibitor design by redefining experimental kinetic methodologies. Because the method of the invention utilizes the assumptions of the split-site model, it more efficaciously leads to the desired outcome of highly specific and potent peptide scission enzyme inactivators.

The theoretical underpinnings of the present invention are further described by investigating the following questions: (1) where is the flaw in the split-site model?; (2) how is the conventional method for transition state inhibitor development prone to this flaw, and therefore hindered?; and (3) how does our new method rectify the flaw in the traditional design method, and by doing so, have an increased probability for a successful inhibitor "hit"?

*Flan in the split-site model*

In our opinion, the binding and reaction centers of ES and TS are not as disconnected as the theory states. To illustrate, we will consider catalysis by the cysteine protease papain. The cysteine protease active site "oxyanion hole" is involved in pocketing the carbonyl group of the substrate P1 group (Kamphuis et al., 1984). It is composed of hydrogen bonds contributed from the main chain of the active site cysteine residue (Cys25) and the side chain of Gln19. Together, these linkages allow for the important binding interactions that lower $ES_B$ and are also conserved in the $TS_B$ (just as the split-site model would predict). However, binding at $TS_B$ and $ES_B$ is not the only function conferred by these binding center residues, as the split-site model would predict.

On the contrary, both residues also contribute to the $TS_R$ reaction center. The same cysteine residue that is involved in hydrogen bonding the oxygen of the P1 oxygen for the purposes of pocketing it both in the Michaelis complex and in the transition state (e.g., to lower $ES_B$ and $TS_B$), is also involved in nucleophilic attack on the same P1 carbonyl group via its charged thiolate residue (acylation). This latter event allows for the production of a tetrahedral adduct, which is followed by loss of the substrate's amine leaving group, consequently causing a lowering of $TS_R$. In many cysteine proteases, this sequence of events *defines* the rate determining step.

The contribution of these same "binding" residues to the "reactivity" center is supported, for example, by the observation that the same hydrogen bonds from Cys25 and Gln19 that form the basis of $ES_B$ stabilization at the ground state are also involved in acid- -catalysis-like stabilization of the negative charge on the oxyanion that is formed as a result of the nucleophilic attack (an event that also directly lowers the $TS_R$). *Consequently, stabilization of $ES_B$ goes hand in hand witch stabilization of $TS_R$ through the medium of a single residue, in this particular aspect of a cysteine protease mechanism.* Another case where the binding center and reaction center in a cysteine protease are not as spatially separated as the split-site model would predict is illustrated by the actions of the residue Asp158 in papain. Asp158 not only functions to hydrogen bond with the nitrogen of the S1' position of the substrate thereby lowering $ES_B$, but it is also described to function in the role of orienting the catalytic triad of cysteine proteases for biochemical attack in the reaction center, an event which would be involved in lowering $TS_R$ (Wang et al., 1994). These examples serve to demonstrate how a part of the ensemble defining the binding center and the reaction center of the enzyme's active site can be located within the same residue. Hence, the major conclusion here is that *the binding center and reaction centers are in fact, intimately connected and their separation by the split-site theory is entirely an oversimplification of the enzyme active site.*

*Critique of the traditional method*

The traditional method for inhibitor design fails to emulate the "split-site" model, thereby presenting several problems.

*Problem one: interference of the reactive moiety with the binding interactions of the peptide core*

First, the traditional method employs a high kcat/Km peptide core that will not only be involved in binding (lowering $ES_B$) but also in the catalytically favorable stabilization of the transition state (lowering of $TS_R$). When a reactive inhibitory moiety is subsequently attached in such close proximity to this high kcat/Km peptide core, it introduces the unfavorable complication of potentially interfering negatively with the favorable binding that is already present in the peptide core of the system at $ES_B$ and $TS_B$ as demonstrated above. To be specific using our prior assumptions: whereas the reactive moiety is attached with the expectation of only increasing $TS_R$ (second inhibitor assumption, above) independently without influencing binding of the molecule, its close proximity to the binding core may also *concomitantly* and *inadvertently* interfere with the event of $ES_B$ reduction (third inhibitor assumption). The mechanism by which this occurs is via direct interference with residues that participate in both binding (of $ES_B/TS_B$) and in reactivity (of $TS_R$). As argued above, the binding portion of the peptide molecule is not "fixed" independently of the reaction center based upon our argument of the flaw with "split-site" models, and therefore the potential for interference of binding is quite high. For example, we will employ the example of a fluoromethylketone reactant moiety which is attached to the Arg of a reactive high kcat/Km substrate analog like Z-Arg for a serine protease like trypsin (Z-Arg is the natural analog for trypsin). In this instance, the Arg is bound very tightly at P1, and therefore plays a key role in binding (i.e., at $ES_B$ and $TS_B$). In addition to this function, Arg also activates the protease for catalysis, and is therefore also part of the reaction center for trypsin ($TS_R$). By virtue of the proximity of the fluoromethylketone to Arg, it is likely that it can interfere with binding function conferred by Arg. The net conclusion is that it is important that the reactive moiety should not interfere with the favorable binding contacts at $ES_B$ and $TS_B$, which are critical for the purposes of binding the inhibitor.

Note that interference of the reactive moiety with favorable binding contacts of the high kcat/Km peptide core developed from the traditional protease inhibitor design model, in turn, has a "snowball effect" on all the interconnected binding interactions at $ES_B$ and $TS_B$. As we have discussed above, binding and reactivity are connected by an ensemble of complex interactions. To illustrate, we can consider the ample evidence that individual binding interactions at TS are cooperative, additive, and synergistic whereas a small disturbance of these interactions can be catastrophic to the binding of the entire molecule.

We postulate that the potential energetic "links" between the reactive functionalities of the reactive moiety and binding functionalities of the peptide core will exponentially increase the degrees of freedom of the binding interactions occurring in the active site (and will also complicate the kinetics of the reaction by an enormous variable). This level of interference will directly correlate to the degrees of freedom that have been increased by the interaction. This number can be enormous, especially in a case where the reactive moiety may be interacting with $ES_B$ or $TS_B$ in an unlimited fashion. For example, the transition state, which draws many of its properties from $TS_B$, is highly "diffuse" and more interconnected with other ES features than the "split-site" model would predict. The transition state is not a single well-defined (Baldwin and Rose , 1999) "saddle point" on the potential energy surface. Whereas this oversimplified description of a saddle point may explain molecular reactions in the gas phase, it does not suffice for a description of the transition state in an aqueous medium.

Ma et al (2000) argue that the transition state may actually be a surface composed of multiple activated conformations (homogenous or heterogeneous ensembles with the same vibrational-averaged structure), which is what the enzyme will actually bind to. Eigenmode search methods have shown that the number of first-order saddle points will increase exponentially as the cluster size increases (Tsai and Jordan, 1993). Thus, this information implies that the system has the potential to become much more complicated if a reactive moiety is also interacting with the $TS_B$ binding center.

Consequently, the goal of an inhibitor design algorithm should be to simplify the inhibitor/enzyme model so that it may have the least degrees of freedom possible, and consequently, the greatest kinetic predictability. This can be potentially accomplished by a twofold general endeavor: 1) minimization of the number of saddle points on the surface by employing the least number of possible degrees of freedom, and 2) experimental simplification, as efficiently as possible, of the diffuse nature of the transition state so that it may approach (as closely as possible) the assumptions of the split-site model of an *independent* binding and reaction center.

*Problem two: The peptide core is catalytic, and counteractive to inhibition by the reactive moiety*

A second significant problem with the traditional method is that a high kcat/Km peptide core, which is attached to a reactive inhibitor moiety, is also characterized by properties that are by themselves conducive to catalytic turnover and therefore counteractive to enzyme inhibition; that is, a peptide core that essentially codes for cleavage by a particular protease may physically predispose the enzyme toward cleavage to a degree that cannot fully be mitigated by a reactive inhibitor moiety. Since the peptide core is part of a high kcat/Km molecule (and therefore lowers $TS_R$), it is also an efficaciously turned-over analog in the first place. We will illustrate using the example of a tripeptide that is linked to the reactive moiety fluoromethylketone. By virtue of the P1 amino acid being involved in lowering the transition state at $TS_R$ (as would be expected for a substrate with high kcat/Km), it becomes more difficult for the reactive fluoromethylketone electrophile (or nucleophile) to counteract this effect (increasing $TS_R$) as is required by the second inhibitor assumption. Consequently, this is the basis of a major problem.

As discussed above, kcat is more important to catalysis than Km in optimally evolved enzymes. Hence, the high kcat characteristics of the peptide core component of the overall inhibitor, makes it more difficult to counteract the lowering of kcat (via lowering of $TS_R$) by the reactive moiety. In summary, two of the key assumptions described above for successful transition state inhibition are potentially counteracted by the traditional method of inhibitor design. Indeed this may contribute to the great number of failures that occur in transition state analog design (Wolfenden, 1999).

*Goals of a new inhibitor design system*

If the binding and reaction centers were truly separated as elegantly as the split-site model would predict, in principle the traditional method of inhibitor design should work, since the reactive moiety would not interfere with the peptide binding core (thereby avoiding the described "snowball" effect), and similarly, since the peptide binding core would not be involved in lowering $TS_R$ at the enzyme's reaction center.

A new method for inhibitor design should therefore produce inhibitor compounds that include the following features. First, favorable binding contacts should be conserved in $ES_B$ and, subsequently, $TS_B$. This peptide core of the inhibitor would then satisfy the criterion of being characterized by strong binding interactions with the enzyme which, as we have emphasized, is very important to catalysis. Second, the inhibitor should contain a peptide core that does not lower $TS_R$ and therefore does not defeat the purpose of the inhibitor, which is to cause the opposite inhibition event (increase of $TS_R$). Third, the inhibitor should contain a reactive moiety that independently raises $TS_R$ without affecting any of the favorable binding contacts at $ES_B$ and $TS_B$.

These are the goals of this invention. The enzyme/inhibitor system is first simplified by reducing the degrees of freedom within it. To that end, the method employs experimental criteria that separate the binding and reaction centers of the enzyme, as close to the assumptions of the split-site model as possible, and thereby prevents the destructive link between the reactive moiety and the bound peptide core.

*The New Method for Inhibitor Design: Rectification of prior problems with the traditional protease inhibitor design method.*

The experimental method described herein is designed to emulate the split-site model as closely as possible. The kinetics are more empirical such that the calculations are more simplified; the model is subject to evaluation and improvement as the method is ongoing. The method endeavors to separate binding as much as possible from reactivity. The essence of the approach is that the reactive moiety (i.e., the moiety that gives the inhibitor its ability to "lock up" the protease) is attached to a *nonreactive* (i.e., noncleavable) peptide core, which participates exclusively in binding but not to a significant degree in catalysis. Because it is attached to a peptide core that binds but is not cleaved by the scission enzyme, it follows that the reactive moiety is involved exclusively in transition state reactions, without interfering with or influencing the binding core. The goal of the method, which represents a significant departure from current methods, is to find such a nonreactive binding peptide core and use it, not a cleavable substrate, as the basis for design of the inhibitor.

Accordingly, the first part of the invention approach (Step I through Step III, below; particularly Step III) provides methodology to identify such a peptide core. Such a peptide core is characterized by a very low kcat but is nonetheless marked by very tight binding to the target enzyme active site. This noncleavable, tightly binding peptide core is termed herein a "vehicle" in the method. By virtue of the vehicle's tight binding to the active site, $ES_B$ is always decreased more than $ES_R$ is increased, such that Km may be low or at least constant. The point here is that $ES_B$ is lowered due to the favorable *direct* binding contacts at the active site as well as the favorable *weak* contacts which are away from the active site that work cooperatively to lower $ES_B$. Based upon the observation of conservation of binding between $ES_B$ and $TS_B$, these favorable binding contacts between the vehicle and enzyme at $ES_B$ are further strengthened at $TS_B$. Thus, efficient binding between the vehicle and enzyme to form ES insures that the assumptions mentioned previously in the reaction coordinate for successful catalysis are met: (1) binding interactions at $ES_B$ and are taken advantage of; and 2) (ES may have the option to enter a transition state such that the vehicle can also bind to the transition state at $TS_B$.

It may be argued by some critics that the vehicle may only be binding exclusively at the ground state level (to lower Km), and therefore cannot have any interactions with the transition state ensemble. Specifically, if true, this would suggest that no binding interactions were occurring between such a vehicle and $TS_B$. However, we emphasize that in the invention method, the vehicle is marked by the signature quality that it "outcompetes" those substrates that would normally be characterized by the highest possible kcat values for the target enzyme, and is therefore marked by a tight binding to $TS_B$ (Step III). To elaborate based upon the previously stated assumptions, the high kcat substrates are achieving excellent transition-state-level binding interactions with the enzyme, since they are marked by a high turn-over (high kcat). It follows then that the competitive vehicle must also be marked by even greater favorable binding interactions at the transition state (at $TS_B$) than even the natural high Kcat substrate analogs.

*We conclude then, based upon the greater competitive ability of the vehicle for the active site over the high kcat substrate analog, that the vehicle would have a greater affinity for $TS_B$ than the high kcat substrate analogs had for $TS_B$.* Conversely, if the vehicle had an exclusive affinity only for $ES_B$ (a low Km vehicle), it is unlikely that it would bind as favorably at the enzyme's active site as the high kcat substrates would, since exclusive binding to $ES_B$ (without binding to $TS_B$) should not, in principle, abrogate binding of the enzyme to the transition states of the high kcat substrates for which it is naturally specific (based upon the high kcat value).

This method is thus expected to produce vehicles capable of binding with greater affinity to the ensemble of binding contacts at $ES_B$ *and or $TS_B$,* than high kcat substrates, which are typically turned over. A novel competitive inhibition assay for these vehicles is presented in Step III. The key feature of this competitor assay is that it is not designed to identify vehicles with an exclusive and tight binding to ES (low Km), but is rather designed to identify vehicles which have tight binding to $ES_B$ *and or $TS_B$.* To conclude, we postulate that the vehicle is marked by a high affinity to $ES_B$ as well as $TS_B$.

Additionally and importantly, the vehicle is noncatalytic (i.e., it has a low kcat), and therefore it cannot lower TS in a manner that causes catalysis. Specifically, the noncatalytic nature of the vehicle implies that its does not favorably interact with $TS_R$. Hence, $\Delta Gb$ is not increased (the definition of transition state driven catalysis) and the enzyme's catalytic rate is not increased. There are three possible kinetic scenarios to account for this. In the first case, $TS_V$

(transition state with vehicle) may be decreased by the same amount in which ES is reduced, resulting in uniform binding (as described above). Consequently, there is no change in the activation energy and no catalytic rate enhancement. In the second case, $TS_R$ may increase by the same amount by which $TS_B$ decreased which causes $TS_V$ to remain unchanged. In this event, ES is lower and there is a net increase in the activation energy and hence no catalysis. In the third case, $TS_R$ may be increased more than $TS_B$ is decreased, which causes $TS_V$ to increase, inducing the greatest possible increase in activation energy, which is again a noncatalytic scenario. Although this third case offers the most inhibitory scenario for the vehicle, it is unlikely to occur since the exclusive binding interactions of the vehicle are unlikely to interact with the reaction center of $TS_R$.

Regardless of the kinetic manner by which catalysis is prevented, the relevant point here is that in all of these possible scenarios, rate enhancement as a result of vehicle/enzyme binding, is impossible. This leads to the conclusion that the vehicle does not favorably interact with $TS_R$ since the vehicle participates only in binding (as it has a low kcat), and not in turnover. Consequently, the "reaction center" of $TS_R$ is separated from the binding centers of $ES_B$ and $TS_B$, a situation which allows this new method to more closely approach the split-site model assumptions than the traditional model.

Another important part of the method (Step VII, below) screens for a reactive moiety that interacts exclusively with $TS_R$ (as opposed to $ES_R$), with the ultimate goal of raising $TS_R$. This screening is conducted using the methodologies well known in the art for screening transition state interactions at $TS_R$. We now enumerate the major advantages to the method of the invention over the traditional method. Like the traditional method, the method of the invention effectively *identifies high affinity peptide binding cores for the active site.* In the method of the invention, a vehicle is identified that binds with an affinity that is greater than all potential high kcat substrates for that active site. This advantage is also conferred by the traditional method, in which a high kcat/Km substrate analog is identified. However, the traditional method's high kcat/Km substrate analog reduces $TS_R$. Thus, by the traditional method, the same peptide core which has tight binding to $TS_B$ and $ES_B$, also interacts with $TS_R$. The binding centers and reaction centers in the ES reaction are not distinguished by the traditional method's substrate analog. In contrast, the noncatalytic vehicle identified according to the present invention binds with $TS_B$ and $ES_B$, but has no interactions with $TS_R$. Hence $TS_B$ and $ES_B$ are effectively "separated" from $TS_R$ in the invention vehicle, which suggests that *the binding center and reaction center between ES can be treated as if they are distinct, as is consistent with a "split-site" model.*

A major advantage of the new method over the traditional method derives from this split: *the experimental addition in the present method of a $TS_R$-raising reactive moieties to the vehicle can be made without interfering with the positive binding interactions that the vehicle shares with $TS_B$ and $ES_B$, since the binding and reaction are "separated" in the vehicle.* In contrast, the addition of a $TS_R$-raising reactive moiety to a high kcat/Km substrate analog, as in the conventional method, has a greater chance to interfere with the tight binding interactions of the tight binding peptide core, since the binding and reaction centers are highly interconnected and are not "split".

In addition, the traditional method employs a substrate analog that lowers $TS_R$ and therefore counteracts inhibition. In contrast, the vehicle engineered from the method of the invention does not have catalytic properties. This yields second major advantage: because of its noncatalytic properties, *the vehicle identified according to the invention cannot lower $TS_R$ and therefore, does not work against inhibition.*

Moreover, as a result of the complexities between the interactions that occur between the binding and reactive portions of the inhibitor molecule produced from the traditional method, the entire traditional system is not very empirical, the kinetics are more complicated and hard to study, predictions are more difficult, and it is therefore more challenging to identify an effective bound and potent peptide scission enzyme inhibitor. In contrast, the split state assumption that underlies the method of the present invention allows for a simple system in which the kinetics are not very complicated, the system is not as difficult to study, the predictions are more efficient, and the identification of a bound and potent scission enzyme inhibitor is more likely. The method of the invention therefore identifies an efficacious noncatalytic (low kcat) vehicle which increases $TS_R$ in a manner which does not interfere with the favorable binding interactions that occur with $TS_B$ and $ES_B$. Although a kcat of zero may be the most theoretically preferred situation, a non-zero low kcat may also be acceptable, particularly when the inhibitory properties of the peptide core can be supplemented by further functionalization with one or more substituents.

*Inhibition of Catalysis Driven by Ground-State Destabilization*

The above arguments assume that transition state catalysis is the mode of enzymatic turnover. However, the mode of catalysis for the enzyme could, instead, be ground state destabilization (Figure 1c). In that event the method of the invention can still be applied. In ground state destabilization, the ground state of the enzyme is destabilized enough to cause a reduction of the activation energy $E_a$ such that the rate of the enzyme is catalytic, although the transition state is not lowered. In this instance, the difference between ES and TS is lowered. To accomplish this, the $ES_B$ must remain constant since $TS_B$ should also not be lowered. Instead, $ES_R$ is raised, which decreases the apparent binding energy (increases Km) and subsequently raises ES and thereby accelerates the rate. The rate acceleration is therefore independent of $ES_B$. Consequently, intrinsic binding energy is translated from binding to catalysis, such that it ($\Delta Gb$) is constant. This latter feature is argued by Murphy (1995) to be the defining characteristic of a ground state destabilization effect.

There are several excellent arguments in the literature that support ground state destabilization as a significant form of catalysis. A paper by Jencks (1987) argues that destabilization of the ES complex is essential for all forms of catalysis. It is argued that small changes in destabilization are not critical for small changes in catalysis but are essential for large increases in the rate. In fact, it is suggested that transition state stabilization is necessary but it is not sufficient for rate enhancements.

To our knowledge, no methods exist that produce inhibitors which exploit the ground state destabilization method. This may be because ground state destabilization is not considered in the popular literature as a serious mechanism for enzymatic catalysis. However, the method of the invention is in principle versatile enough to produce inhibitors that inhibit enzymes that operate by transition state stabilization, ground state stabilization, or a combination of both. How counteraction of catalytic ground-state destabilization is achieved will now be discussed.

In order to accomplish inhibition, the steps in the method of the invention identify a tight binding noncatalytic peptide core (Steps I thorough III). Since the peptide core is tightly bound, it causes an increase in the apparent binding energy of the enzyme through favorable contacts at the binding centers in the ground state and the transition state. Hence, $ES_B$ is lowered more than $ES_R$ is raised. This may cause ES to be reduced, which translates into either an unchanged or lower Km. Consequently, this effect alone will in principle, cancel out the case where catalysis will occur and where ES is raised in the normal scenario of catalysis. It may be argued that $TS_B$ will also be lowered since $ES_B$ was lowered (in accordance with the principle of conservation between $ES_B$ and $TS_B$). However, the peptide core is noncatalytic, which means that $TS_B$ was not lowered more than $TS_R$ was raised, such that the lowering of TS is not beneficial to catalysis. This is an intrinsic property of the peptide core. Moreover, the addition of a reactive moiety to the noncatalytic peptide can lead to additional effects at the ground state that interfere with the progression to a transition state. Again, since the binding portion of the inhibitor has been constructed separately from the reactive portion, there is a diminished likelihood that the inhibitory binding events of the peptide core will be negatively influenced by the reactive moiety.

From the discussion to this point, it should be evident that the invention is not only novel, but follows an approach that is contraindicated by the previously generally accepted paradigms in the literature and practice of this field. The benefits and empirical success of the invention method will be illustrated in the following sections.

*PART II. METHOD OF THE INVENTION*

Proteases are proteins that catalyze cleavage of substrates that contain a peptide constituent. As they are found universally and are in many ways representative of scission enzymes as a class, proteases will be used as the archetypal example of scission enzymes for purposes of illustrating the invention method.

The term "protease" as used herein means an enzyme that cleaves a polypeptide of any length, short or long, at a peptide bond, for example by hydrolyzing the peptide bond. The substrate of a protease is thus a polypeptide. The substrate can be a naturally occurring polypeptide or a synthetic polypeptide. Most proteases exhibit specificity for certain peptide sequences within a polypeptide, and the invention is designed to exploit this specificity in the identification of potent and specific protease inhibitors, which are termed herein "protease inactivators." "Potency" is defined herein as inhibition that occurs with a tight binding (low kI), and "specificity" is defined herein as inhibition which is marked by maximum complementarity to the configuration of the enzyme which causes catalysis (e.g., transition state or ground state). A third attribute, "selectivity," is defined herein as a characteristic of inhibitors that can discriminate between the target protease and other proteases. Selectivity is often the natural outcome of both "potency" and "specificity." Sometimes the term "peptidase" is used in the art to identify a subset of proteases that cleave short polypeptides (i.e., peptides). However, the term "protease" as used herein is intended to include "peptidases."

Substrates of proteases are conveniently characterized and categorized according to the method of Berger and Schechter (1970) (Fig. 2). These researchers were the first to assess the specificity of papain, and showed seven subsites in the papain active site with four on the acyl group side (S4-S1), and three on the leaving group side (S1'-S3'). Their classic nomenclature is used herein. The "S" positions refer to the subsites of the enzyme, whereas the respective and correlative "P" positions refer to the substrate residues which bind to that particular subsite. The scissile bond cleavage event occurs at the P1/S1 binding groove between substrate and enzyme, respectively, where a the N-terminus of a substrate is nearest to the higher-numbered S sites, and the C-terminus of a substrate is nearer to the higher numbered S' sites.

Protease inactivators identified according to the method of the invention typically have the general formula $R_1$-$(Xaa)_n$-$R_2$ wherein $R_1$ is an optional blocking group, Xaa is an amino acid residue, n is between 1 and 9, preferably between 1 and 6, more preferably between 1 and 4, more preferably 1, 2 or 3, and $R_2$ is optional protease inactivating reactant. $R_1$-$(Xaa)_n$ is referred to as the core or vehicle. Inactivating reactants have the following characteristics. First, they are inert toward the core under ambient conditions. when enzyme is not present. Second, they become activated in the presence of the enzyme. Third, when bound to the target enzyme, they react or bind more quickly than they dissociate (Fersht, 1985). Blocking groups are well-known and routinely used in the art of organic chemistry, and the invention is not limited to the use of any particular blocking groups on the vehicle or protease inactivator; indeed, as noted above, the presence of a blocking group is optional. Examples of blocking groups or moieties include: Z = carbobenzoxy; B = Boc = t-butoxy; Ac = acetyl; Suc = succinyl (COOH and/or $CH_2CH_2CO$ can be attached to the amino end); Mu = morphyline; MeOS = methoxysuccinyl; glutaryl = glutaric acid; D = D-amino acid rather than the naturally occurring L amino acid; nonnatural amino acids such as Nva-norvaline, Abu-aminobutyric acid can also be used, as can phenyl groups and the like.

Examples of inactivating reactants (which include "suicide reactants" as commonly described in the literature) include fluoromethylketone (FMK), chloromethylketone (CMK), diazomethylketone, vinylsulfone (VS), vinylsulfonephenyl (VSP or VSPh), epoxide (Ep), ketoamide (CONH or KA), chloromethane, $CONH-CH_2$-pyridyl or CONH-pyridyl (KPy, KPyr or K), CONH-4-morphophenyl (KM), CONH-ethyl (KE, KC or KE2), an alphaketo acid (AK), a Michael acceptor, peptide aldehyde, acetaldehyde, nitrile, hydroxymate, cyclopropenone and epoxysuccinamide.

The amino acid residues can be naturally occurring amino acids or nonnatural amino acids, such as norleucine and ornithine. They can be D-amino acids or L-amino acids, and can include derivatizations or modifications of any sort. The amino acids can be joined by peptide bonds or by any other type of bond, such as ester bonds. They can be alpha-amino acids, beta-amino acids, peptoids (containing one or more substituents on backbone amide N-sites instead of or in addition to substituents on the alpha amino positions of polyamide backbones) or peptide analogs in which one or more atoms of a peptide have been replaced by different atomic elements or by functional groups, in order to obtain improved properties for pharmacophoric use. The peptides may be of any length.

The portion of the protease inactivator other than the inactivating reactant is referred to herein as the peptide core. Structurally, the peptide core is represented by represented by $R_1$-$(Xaa)_n$ or, if there is no blocking group, simply $(Xaa)_n$ (see above). The peptide core includes one or more amino acids (P through P') that bind to one or more amino acids (S through S') at the substrate binding site on the target protease (Fig. 2).

The method of the invention can be employed to identify inhibitors of proteases produced by organisms, such as bacteria, fungi and protozoans, that are pathogenic to animals or plants, or for any protease whose inhibition would serve a useful purpose. Preferably, the protease inactivator is selective for a protease specific to a pathogenic organism or associated with a particular disease state, and does not inhibit other nontargeted proteases of the host. The target protease can be a known or an unknown protease.

One of skill in the art will appreciate that all the described steps of the method can be performed, or, alternatively, one or more steps can be omitted at the discretion of the investigator, depending for example on the known characteristics of the protease, the substrates, and/or the results of preceding determinations. For example, the general screen for peptide cleavage (Step I) may be omitted in the case of a well-characterized protease. Likewise, testing of candidate substrates (Step IV) or protease inactivators (Step VIII) for their ability to inhibit cleavage of natural substrates of the protease can be omitted if time and money are in short supply, as this step yields information that is useful but not necessary to the ultimate identification of an active site protease inhibitor.

Further, as will be also recognized by one of skill in the art, the order in which some of the determinations are made can be varied. For example, the selectivity study (Step IX) can be performed immediately after Step VII, Part B, which identifies the inhibitor potential of covalently linked inactivating reactants. In this instance, the investigator may wish to assess selectivity of the potential inhibitors before actually going through the time and expense of conducting transition state or ground state assays in Step VII- part C. The following steps must be conducted, without exception, but may be conducted in any order and are not limited to kinetic assays: Step II (Substrate cleavage screen), and Step III (Competitor assay). Step VII (Transition state or ground state assay) may be conducted where desirable.

As noted above, the method of the invention can be performed using a starting material that is a purified target protease or a crude mix that contains the protease activity. Once the crude mix has been characterized, it is utilized in the same manner as the purified protease, except that it is added in substitution for the purified protease. Of course, when available, it is preferable to employ the purified protease as a starting material. It should be understood that the procedures set forth below are equally applicable to purified target proteases and to crude mixes; however, if there are modifications, additions, or deletions to the experimental protocol that are suggested for crude mixes, these are also described. The amount used is the amount that is proteolytically detectable on the detection device.

*Preliminary Step (Step P): Preliminary determination of defined, optimal assay conditions for substrate cleavage*

The optimal environment for proteolytic cleavage by the target protease is established prior to performing the experiments that lead to the identification of a transition state inhibitor of the protease. In this regard, information or determinations regarding the pH and other assay conditions (for example, temperature, cofactor requirements, and buffer conditions) that allow for optimal active site catalysis is useful. In addition, if preliminary characterization data can be obtained or is available, such as a general idea of the mechanistic class of the protease, this information is helpful in optimizing the conditions for catalysis. Examples of mechanistic classes include cysteine proteases, serine proteases, aspartic proteases and metalloproteases.

This preliminary assay for determining optimal assay conditions for substrate cleavage preferably utilizes representative substrates of the target protease. For example, a representative endopeptidase substrate such as Z-Phe-Arg-AFC would be used if the target protease were a cathepsin L-like cysteine protease, or Z-Arg-AFC would be employed if the protease were expected to be a serine protease. AFC represents the chromophore 7-amino-4-trifluoromethylcoumarin, which fluoresces upon cleavage from the peptide. Alternatively, a chromophore that ceases to fluoresce upon cleavage may be employed instead. Examples of other suitable chromophores include para-nitro-analine (PNA), 7-amino-4-methylcoumarin (AMC), and 7-amino-4-carbamoylmethylcoumarin (ACC), where the choice of chromophore may be dictated by: cost and difficulty of synthesis; wavelength for fluorescence detection; compatibility with the protease; and other practical considerations.

Where the target protease is a purified protease, the representative substrate concentrations in the preliminary assay are preferably at least 50 to 100 times greater than the enzyme concentration to insure saturation conditions, and it is also preferable that they be less than the micromolar range in order to prevent introduction of variables that may potentially introduce nonlinear kinetic phenomena. In this assay, the purified protease is added to the saturating amount of substrate in assay buffer.

The target protease can also be part of a "crude mix." In that case, the preliminary assays are altered as described in more detail below.

*A. Determination of optimal pH and pH range*

Of all the assay optimizations, determination of the optimal pH range for the target protease is the most important. A starting pH point can typically be inferred from the biological source of the target protease or it may already be known beforehand. For example, the *Taenia* cysteine protease is present in the lysosomes of the *Taenia* parasite (an acidic environment), thus an acidic buffer with 0.4 M Citrate, pH 4.9 (WO 00/63350) was employed for protease activity studies. The pH profile of the target protease can be investigated by determining a pH range for a battery of substrates representative for the active site class (e.g., endopeptidase substrates if the protease is an endopeptidase, aminopeptidase substrates if the protease is an aminopeptidase, and the like). A pH vs. Activity curve is plotted and the pH, preferably to the hundredth degree, is determined for maximal cleavage of the most representative substrates. In instances where variations in optimal pH are observed for different substrates, the pH closest to the pH appropriate to the biological location of the target protease is typically the optimal pH for subsequent studies. Preferably the assays are conducted at the optimal pH $\pm$ 1 pH unit, more preferably $\pm$ 0.5 pH units, most preferably $\pm$ 0.1 pH units.

*B. Determination of optimal exogenous conditions*

A strategy similar to the one above can be followed to determine whether exogenous components need to be added to promote protease activity. For example, cysteine proteases often require a reducing environment for activity, calpains are activated by the addition of calcium, and metalloproteases commonly require a metal cofactor for activation. Hence, conditions for optimal cleavage by the target protease will vary from protease to protease and should be determined accordingly. For example, it was determined that 10mM L-cysteine should be added to the assay buffer for *Taenia* cysteine protease in order to optimize cleavage conditions for the representative endopeptidase substrate, Z-Phe-Arg-AFC (WO 00/63350).

*C. Determination of optimal incubation temperature*

Assay incubation temperatures may also be varied to determine the optimal temperature for protease cleavage. If the target protease is from a human, this temperature will typically be 37°C, which is the physiological temperature for most enzymatic activity. For the human parasite *Taenia* protease, the optimal temperature for cleavage was determined to be 37°C (WO 00/63350).

*D. Determination of optimal incubation time*

Once the above conditions in the assay have been defined, it may be useful to incubate the most highly cleaved substrates (known as "high kcat substrates") individually with the target protease, then to construct a plot of time vs. cleavage. The time at which less than 10% of the reaction has gone to completion should be noted for future readings. A reaction that has proceeded to less than 10% completion is typically one in which substrate concentrations remain saturating, thereby preventing concentration-dependent pleiotropic caveats that can occur if the substrate concentrations are reduced, as discussed below. This can complicate the kinetic assessments that are conducted later. Hence, if the reaction has proceeded too far, and substrate concentrations are altered, the kinetic variables may not be trusted.

*E. Target protease in a "crude mix"*

As noted above, the target protease can be a purified and isolated protease, or it can be part of a crude mix. A "crude mix" is a collection or medley of biological components within which exists the target protease. Typically, the crude mix is an unpurified or partially purified biological sample. In assays that employ a crude mix, it is especially important to define optimal catalytic conditions as described above. The more "fine tuned" the assay environment, the greater the possibility of narrowing down the number of proteases which are active under those defined conditions. Even when the optimal environmental conditions for cleavage of a battery of substrates have been determined, the possibility still exists that isoforms of the protease or even more than one protease will function optimally at the given conditions. However, even in the event that more than one protease functions optimally in a given assay environment, it is still possible and even likely that the biochemical mechanisms in the catalytic event follow some conserved pattern. This is based upon the fact that cleavage of more than one protease is optimal under the defined assay conditions due to similar biochemical mechanisms within the active site. Indeed, significant biochemical conservation is often present at the active site level for different classes of proteases. For example, both cysteine and serine proteases proceed with a similar mechanism employing a tetrahedral adduct with the substrate at one of the important transition states (Kamphuis et al., 1984; Polgar et al. 1988).

Without an in-depth analysis, it is impossible to assess whether or not the mechanism of two or more proteases in a specific environment is completely conserved. Nevertheless, and without intending to be bound by any particular theory of biological activity or mechanism, these procedures proceed with the assumption that there does exist a conservation of active site mechanism of more than one protease operating at a given environmental condition, and that an inhibitor that is specific for that active site should abrogate the catalytic event for those proteases. Even if this assumption is not completely correct, the procedures outlined below are expected to produce an inhibitor that has some affinity for the active site of one or the other or all. As demonstrated in the Examples, the same inhibitors (Z-LLL-FMK and Z-LLY-FMK) were produced for the purified protease as for the crude mix that included the protease. While this discussion assumes that more than one protease will be active at a very specific environmental condition, in fact there may exist crude mixes which contain only one protease that is active under the defined conditions.

The procedure to determine the optimal catalytic environment for a protease present in a crude mix is similar to that outlined above, with the following optional refinements. Until the optimal incubation time is determined, long incubation times (e.g., 18 - 24 hours) are typically employed. The procedures may vary depending on whether anything is known about the mechanistic class of the protease. In the preliminary activity optimization assay described below, the term "activity" is used interchangeably with "protease" since, by definition, the protease in a crude mix is not purified.

The crude mix is prepared from a biological sample using procedures that are designed to isolate protein and proteinaceous materials from other cellular components, such as lipids, nucleic acids, and carbohydrates. The particular techniques used depend on the known or suspected biological source of the target protease. Many procedures for the liberation of proteins are known in the art, and the invention is not limited to any particular procedure for preparing the crude mix. For example, in the case of the protease from the *Taenia* protease, it was suspected that the protease was a peripheral membrane protein. Consequently, rigorous vortexing of the parasites in acidic buffer was conducted followed by centrifugation, which caused cysteine protease-like activity (based upon substrate cleavage and inhibitor profiles) to be liberated into the supernatant (WO 00/63350). On the other hand, if the protein is suspected to be membrane bound, a detergent solubilization method may be employed.

If desired, the crude mix can be further purified. For example, it can be subjected to size exclusion chromatography, such as gel filtration, which separates components in the crude mix by size. Gel filtration media is available from several vendors including BioSepra, Pharmacia, and others. For example, crude mix containing the proteolytic activity can be loaded onto a gel filtration column and fractionated. The fractions surveyed using the optimal conditions for cleavage determined above can be used to detect subfractions containing the desired activity. Optionally, a gel filtration column with a wide separation range can be used first followed by a narrowing of this column (by a change of the gel filtration resin) to the range that most suitably will partially purify out the protease. If the activity cannot dissolve in solution, then a separate partial purification strategy may be necessary. Optionally, additional purification of the target protease can also be performed, such as by ion-exchange or reverse phase chromatography.

Preferably, a wide pH range is studied. Knowledge of the pH of the biological environment from which the enzyme of biological interest is derived is helpful, although not necessary. If the mechanistic class of the protease is known or is suspected, a pH vs. activity profile is constructed using one or more representative substrates. If the mechanistic class is not known, a pH vs. activity curve for substrates representing the different mechanistic classes is constructed. Possible substrates for different mechanistic classes are included in the following table. For example, possible substrates include Z-Phe-Arg-X for cysteine proteases and Z-Arg-AFC for serine proteases, where X represents the detection group (e.g., X is 7-amino-4-trifluoromethylcoumarin).

Examples of Representative Substrates for Various Enzymes

| Enzyme | Representative Substrate |
|---|---|
| Cathepsin B | B-LRR_AFC |
| Cathepsin C | GR-AFC |
| Cathepsin D | Z-RGFFP-AFC |
| Cathepsin G | Suc-GGF-AFC |
| Cathepsin H | L-R-AFC |
| Cathepsin L | Z-FR-AFC |
| Cathepsin K | B-AGPR-AFC |
| Chymotrypsin | Suc-LLVY-AFC |
| Elastase | MeoSuc-AAA-AFC |
| Trypsin | Z-R-AFC |
| Urokinase | Glutaryl-GR-AFC |
| Plasmin | Z-AKK-AFC |
| Interleukin Converting Enzyme (ICE) | Ac-YVAD-AFC |
| Aminopeptidase B | L-K-AFC |
| Aminopeptidase M | L-L-AFC |

Following construction of the pH vs. activity profile, the results are reviewed to determine the pH optimum and the specific substrate that will be employed for detection. If the mechanistic class of the protease is not known, then an assessment and decision is made based upon an analysis of the protease activity with respect to the various substrates tested. A number of different enzymatic activities may be observed within the crude mix; activities are evidenced by the observed cleavage of various representative substrates. The choice of which activity to investigate is left to the discretion of the investigator. For example, conditions that generate the highest cleavage on a specific substrate can be chosen. On the other hand, a particular pH might be chosen, in instances wherein the activity at that pH appears to be a biological activity of interest. In the case of the *Taenia* protease (WO 00/63350), the activity chosen for further investigation was the highest activity observed at acidic pH. This was because the protease of interest was suspected to be located in the lysosomes, an acidic organelle. The activity selected for investigation was derived from a supernatant resulting from an acid extraction, and maximal activity was noted at pH 4.9, which was equivalent to the pH of the lysosomes.

After an activity has been chosen based upon the observed cleavage of specific substrate, the pH optimum can be refined. It should be cautioned that double peaks might be observed on pH vs. activity curves, which often represent overlapping enzymes or dual isoforms of the same enzyme. The demonstration of such peaks, deviating from bell-shaped behavior, are indicative of more than one major enzyme operating at the pH indicated.

Once the pH optimum and the general detection substrate are identified, a crude mix concentration dose curve is constructed with respect to substrate cleavage activity. The crude mix is added in increasing concentrations to the specific substrate at the determined pH optimum. Following this, the activity of the protease at each concentration is followed over time. The concentration and a time at which activity is registered are determined.

Exogenous variables, optimal incubation temperature and optimal incubation time are determined as described above for the purified target protease.

*Step 1. General screen of protein substrates to identify preferred cleavage patterns*

This is an optional screen that can be performed initially or later during the method in order to obtain general information the cleavage preferences of the target protease. More specifically, this screen is used to identify patterns at the P3, P2 and P1 sites of substrates that are associated with substrate cleavage. This screen is typically performed using naturally occurring proteins as substrates. It is preferred that the protein substrates contain a full complement of amino acids in order to conduct the most general screen. Examples of preferred protein substrates include albumin and insulin. If the natural substrate of the protease is known, then this protein is used as the source for cleavage of the substrate. In example 1, we employed human Immunoglobulin G as the protein source for testing cleavage by the *T. solium* cysteine protease, since it had been previously determined that this protein may serve as a natural target for cleavage. Protein substrates can also be chosen based upon the expected biological location of the protease. For example, acidic cysteine proteases are known to cleave general imported proteins like albumin, so albumin could be used in the screen of a cysteine protease. Preferably, mass spectrometry is used to follow protein substrate cleavage.

The protease is incubated in an assay buffer under the optimal conditions for cleavage as determined in the preliminary optimization assay. The cleavage patterns are analyzed to determine the cleavage preferences of the protease, i.e., amino acids or classes of amino acids that appear frequently at positions P3, P2 and/or P1 of the screened substrates. If no cleavages are observed, the protease concentration, reaction conditions and/or substrates are varied until cleavage is observed.

*Step II. Determination of high and low kcat substrates*

This is another screening procedure. It permits *in vitro* detection of the most efficacious synthetic peptide substrate(s) of the target protease (high kcat substrates). Substrates that are not well cleaved (low kcat substrates) are also identified.

*A. Screen of synthetic substrates*

This screening procedure identifies the synthetic substrate(s) that are most effectively cleaved by the target protease. Preferably, the screened substrates are tripeptides, dipeptides, and/or compounds comprising single amino acids, but longer peptides can be used if desired. The N-termini of the screened substrates are optionally derivatized (for example, with a protecting group as a result of chemical synthesis), and a detection group is preferably attached to the C-terminus to facilitate detection of the cleavage event. It should be understood that the method is not limited to the use of substrates having any particular protecting group and/or detection group, and that a detection group may be attached to the N-terminus instead of or in addition to the identical or a different detection group on the C-terminus.

A detection group that is well-suited to the present method is a fluorometric group such as 7-amino-4-trifluoromethyl coumarin (AFC), which can be detected by a high quality fluorometer apparatus. In the context of the present invention, a fluorometric group is one that is nonfluorescent when attached to the peptide core (which can be a single amino acid, a dipeptide, a tripeptide, and so on without limitation) but fluoresces when it is cleaved. However, it should be noted that fluorometric detection is not the only mechanism by which protease activity can be measured. Indeed, several methods are already known in the art including those involving kinetic isotope effects (Cleland, 1995). Any can be chosen at the discretion of the investigator; the invention is not intended to be limited by the choice of any particular detection group. In Example 2, AFC-linked monopeptides, dipeptides, and tripeptides with a variety of permutations at P3-P1 were obtained from Enzyme Systems Products (Livermore, CA) and cleaved to release the AFC group, which was then measured as a free molecule by the fluorometer (AFC absorbance: 400 nm; AFC emission 505 nm.) AFC cleavage is converted to molar quantities, based upon a previously established standard curve for the available fluorometer.

The researcher can choose whether to screen a broad class of peptide substrates or a more limited class of peptide substrates. Given 20 naturally occurring L-amino acids, the total number of tripeptides, dipeptides, and/or compounds comprising single amino acids theoretically available for screening is 8,420. When dealing with such large numbers, the advantage of accessibility to high throuput instrumentation, such as a microarrayer system or robotics instrumentation, becomes readily apparent. However, if this instrumentation is not available, the bulk of the entire procedure can be conducted quite inexpensively with 10 by 50 mm Flint-Glass tubes (available from Fisher Scientific), an incubator, and a general fluorometer or other detection device.

Cost and time often dictate that a smaller panel of substrates be employed in the screen. In that event, substrates that are representative of those proteins cleaved by the protease in view of its known or suspected mechanistic class are preferably employed. There are several means to select representative substrates for the target protease. For example, the mass spectrometry screen may provide information as to which amino acid combinations at P3-P1 are preferred for cleavage by the target protease. In addition, if a natural substrate of the protease is known, representative substrates can be predicted by analyzing amino acids at positions P1, P2 and/or P3 of the known substrate and selecting substrates having amino acids with similar characteristics (e.g., hydrophobic, polar, nonpolar) at corresponding positions. In addition, it may be known or observed that certain amino acids at certain sites are preferred in view of the active site class of the target protease. For example, many serine proteases prefer an arginine at P1.

Regardless of how many substrates are used, the target protease is incubated, individually, with each of the available substrates using the optimal proteolytic conditions determined in the preliminary assay. It is helpful if the substrate can pre-incubate in the assay buffer for a short period of time in order to reach an equilibrated status (e.g., about 20 minutes), before the addition of the protease. The protease should not be added too much later. In Example 2, we determined that the endopeptidase substrate, Z-Phe-Arg-AFC ($Z= C_6H_5$-$CH_2$-O-CO-, blocking group for the N-terminus) is cleaved more efficiently than several other substrates in a small library for the *Taenia* cysteine protease. This assessment was based upon rapid cleavage of the AFC fluorometric group.

*B. Assessment of kinetic parameters for substrate cleavage*

When the target protease is purified and has been quantified, Vmax is calculated for the cleavage of each substrate, provided that the concentration of the protease is known. Since kcat is a direct function of Vmax, these values can be determined rather quickly. For example, if AFC is used as the detection group, kcat and Vmax are calculated from the kinetics of the liberation of AFC groups. The result is a kinetic representation for substrate cleavage by the target protease. If the concentration of protease cannot be determined (as in a crude mix), only Vmax is calculated. It should be noted that Km need not be and typically is not calculated in this assessment.

*C. Identification of high and low kcat substrates*

Based upon the above results, those substrates with the highest and lowest values for kcat (or Vmax, where protease concentration is unknown) are identified. The high kcat substrates will be employed as detection compounds for the target protease, as well as a source for competition of potential peptide binding cores (Step III) and inhibitors (Step VII)

The low kcat (or low Vmax) substrates can be divided into two classes: substrates that are not bound and therefore not turned over, and substrates that are bound but not turned over (noncatalytic binding), and potentially locked in the active site. Substrates obeying the latter condition are of interest as potential inhibitors of protease activity, since they are bound but possess no biochemical characteristics conducive to turnover. They will be referred to as "vehicles" from this point forth, since they are "delivered" into the active site. As discussed above, it is this possibility that will confer significant advantages to the bound vehicle, since the design goal of the peptide core is to identify a substrate that lowers the binding energy of the enzyme/substrate complex ($ES_B$) and the binding energy of the transition state ($TS_B$) but does *not* lower the reactivity energy of the transition state ($TS_R$) as a catalytically bound peptide core would. *As we have emphasized in Part I, we postulate that the exploitation of $ES_B$ and $TS_B$ by an inhibitor is a key benefit of the method of the invention.*

*Step III. Identification of inhibitory noncatalytic bound substrates*

This binding assay is performed on the set of low kcat substrates and distinguishes nonbound substrates from bound, but noncatalytic, substrates. More specifically, the assay allows for identification of enzyme/substrate pairings that are marked by very tight binding to $ES_B$ and or $TS_B$, but without a lowering effect of $TS_R$, such that the "binding" centers and "reaction" centers of the enzyme are effectively separated. (As will be recalled, $ES_R$ is entirely independent of $ES_B$, $TS_B$, and $TS_R$ and is therefore not considered to a great extent in this discussion).

It is a unique feature of this invention that it identifies cores that are hydrolyzed only minimally or not at all by target enzymes, yet that have substantial binding characteristics at the active site. Non-kinetic methods may be used for this assay, such as nuclear magnetic resonance or other spectroscopy to assess binding within enzyme-substrate complexes. However the most convenient and preferred mode of the invention prefers competitive binding assays.

In addition to identifying those low kcat substrates that exhibit tight binding to the configuration of the enzyme which drives catalysis, this assay further selects for substrates that are so tightly bound that they inhibit the cleavage of high kcat substrates. The end result is the identification of a tripeptide, dipeptide, or single amino acid motif that is capable of binding the active site in a kinetically more favorable fashion than the most commonly bound tripeptide, dipeptide, or single amino acid combination (those which would normally have a high kcat/Km ratio). A specific example of this assay is provided in Example 3. Tightly bound, noncatalytic substrates identified according to this step are termed "noncleavable" or "nonhydrolyzable" herein, and are referred to as inhibitors or inactivators herein, and represent substrates that are cleaved to only a small extent at most, and that noncatalytically bind the target protease.

As mentioned in Part I, it may be argued that the tightly bound substrates in this step are binding only $ES_B$ and not $TS_B$, in the case where transition state stabilization drives catalysis for a specific enzyme. Hence, such binding would be indicative of only a ground state inhibition. However, a key feature of this step is that the bound substrates are outcompeting high kcat substrates which participate in catalysis by also binding to $TS_B$. Hence, if a bound competitive substrate "vehicle" in this instance binds more favorably than such a high kcat substrate, we posit that it must also be binding to $TS_B$, the configuration which would be marked by the tightest binding characteristics. This would thus be the mode of inhibition, since the high kcat substrate is bound most tightly to the transition state.

Determination of Km for a large number of substrates can be a very time consuming task if advanced instrumentation and extensive resources are not available. To minimize that problem, this assay is designed to select quickly and efficaciously for substrates that have a very low Km (or Ki in the steps below) value, without the use of the exhaustive resources or kinetic modeling (note that the actual Km is not determined). A large panel of substrates with extremely tight binding affinities for the target protease can be rapidly analyzed.

The potential competitive inhibition of the target protease is determined in a two part process. First, an initial pool of low kcat substrates is identified that will bind the protease more favorably than a cocktail comprising a small number of the top kcat substrates. The goal of this part of the assay is to obtain a very well differentiated profile representing a ranked inhibition of protease cleavage of the high kcat substrates, by the low kcat competitor substrates. The qualitative profile of inhibition is more important here than the acquisition of quantitative values. Then, the dose of the cocktail is steadily increased by a steadily diversified and more concentrated medley of high kcat substrates in a manner that imposes greater stringency on the competitor assay (multiple dosing screens). The differentiation among the low kcat substrates based on competition ability is thus increased, allowing for selection of only the most tightly bound peptides as the pool size is steadily decreased.

The increase in stringency that results from an increase in the "dosage" of the cocktail is thus expected to select for those substrates that have the highest inhibitory potential. This method serves as an efficacious "filtering" mechanism to identify a pool of inhibitory substrates from the original panel. This final pool can then be assessed kinetically for its Ki values (which are also equivalent to the substrate's Km value).

*A. Choice of high and low kcat substrates*

Of the full panel of substrates assessed, a group (typically the top 1 %) of substrates with the highest kcat values are chosen to compose a "TOP cocktail", and a second group (e.g., the bottom 20%) of the substrates with the lowest kcat values are chosen for testing as the "BOTTOM substrates." The low kcat choices will ideally include substrates that are not cleaved at all. The choice of the starting percentage is subjectively based upon what the investigator determines to be "high" and "low", and can be varied based upon the outcome of the experiments. However, a reasonable starting point is to choose substrates which exhibit kcat values in the top 1% of all substrates screened for high kcat substrates and those which exhibit kcat values in the lowest 1 % for low kcat substrates. As a reminder, the term "vehicle" is used to refer to that subgroup of low kcat substrates that can inhibit cleavage of the TOP cocktail (these substrates are therefore delivered into the active site).

To illustrate, let the TOP cocktail contain, in increasing order of kcat, substrates "v", "w", "x", "y", and "z" ("z" having the highest kcat value). The BOTTOM cocktail (the initial pool of low kcat substrates) can contain, in increasing order of kcat, substrates "a", "b", "c", "d", and "e", ("a" having the lowest kcat value).

*B. Analysis of the initial pool*

The experiment begins with a competitor test of the lowest kcat substrate first (i.e., "a") against the TOP cocktail, followed by the second lowest kcat substrate (i.e., "b"), and so forth. The assay conditions are those identified in the preliminary optimization assay. The TOP cocktail is added to the lowest determined kcat substrate (i.e., "a") in the assay buffer and allowed to equilibrate for a short period of time (about 20-30 minutes). Each of the substrates in the TOP cocktail should be present in equal concentration. Next, the target protease is added. All of the BOTTOM kcat substrates in addition to "a" (i.e., "b", "c", "d", "e") are tested individually and separately for their potential to inhibit the TOP cocktail. The concentration of the tested low kcat substrate should be equivalent to the individual concentration of each substrate in the TOP cocktail, and it is recommended that each of these concentrations be at least 50 to 100 times the concentration of the available enzyme to insure saturation conditions. For example, if the low kcat substrate "a" is present at 1 uM, each of the high kcat substrates chosen for the cocktail (i.e., "v", "w", "x", "y", "z") should be about 1 uM each in the final assay buffer. Since the concentration of the enzyme is at least 100 times less (i.e., enzyme concentration may be 1 picomolar), saturation conditions are still met.

Since the low kcat substrate is being tested as a competitive inhibitor, its binding constant will now be referred to as Ki, whereas the enzyme's affinity for each individual high kcat substrate in the TOP cocktail will be referred to as $Km_a$ (with $Km_a$ = the average of Km values for the high kcat substrates. Note that Ki and $Km_a$ are purely theoretical and are not calculated at this point. The actual Ki of these vehicles need not be determined until later, and $Km_a$ need never be calculated at all. Indeed, the absence of required calculation of Ki at this stage in the method is an important feature that eliminates significant time and cost. Since both the TOP cocktail and the tested low kcat substrate are in saturating quantities, it is presumed that this scenario reflects Profile II described in the background where [S]> $Km_a$, and hence the apparent binding energy of [ES] will be below that of E + S. The goal of this first part of the process is to obtain a *relative* profile of inhibitory ability by the low kcat substrates. There is no emphasis here on quantitative inhibition. Several resulting scenarios are possible.

*Scenario I. Profile II with low concentration of low kcat substrate:* In this scenario, inhibition of the target protease is observed for several of the low kcat substrates and an inhibition profile for the low kcat substrates is obtained without further analysis.

*Scenario II. No inhibition:* Although unlikely, it is possible that no inhibition is observed for any of the low kcat substrates in this initial assay. Without intending to be limited by theory, this might be attributable to the unusual cases where the enzyme does not employ its P3-P1 subsites in its catalytic event. If unsatisfactory inhibition is obtained in the first screening, any one or more of the following three experiments (Scenarios IIA, IIB and IIC) can be conducted.

*Scenario IIA. Profile II with low concentration of low kcat substrate but reduced number of high kcat substrate in cocktail:* In this experiment, the same assay conditions are maintained with respect to the concentration of low kcat substrate and cocktail, except that the *number* of kcat substrates in the original cocktail are reduced. In other words, a lower percent BOTTOM cocktail is employed such as a 0.1% or a 0.2% cocktail instead of a 1.0% BOTTOM cocktail. If this strategy is successful, reduction of the *number* of high kcat substrates in this cocktail will yield an inhibition profile. As in Scenario I, it is important to "fix" the concentrations before proceeding to the second part of this process (the secondary dose screening).

*Scenario IIB. Profile II with high concentration of low kcat substrate.* In this experiment, the substrate concentration remains saturating, however the concentration of the tested low kcat substrate is increased. An example of one means to do this would be to employ the same concentration of the low kcat substrate as the total of all concentrations in the cocktail. Following the example noted above, if 1 uM was present in each of the high kcat substrates in the TOP cocktail for 5 substrates, the new concentration of the low kcat substrate may be 5 uM. (Again, the enzyme concentration remains low at, for example, 1 pM). Without intending to be limited by theory, it is thought that Brownian dynamics may allow for greater collisions between the low kcat substrate and the enzyme even through concentrations of each individual substrate in the cocktail are still saturating to the protease ($[S]>Km_a$) and in accordance with Profile II. Again, if this strategy is successful, increasing the concentrations of the low kcat substrates will yield an inhibition profile.

*Scenario IIC. Profile I with low concentration of high kcat substrates.* In some instances, it may be preferable to shift the assay into a Profile I-like scenario where the substrate concentration of each component in the TOP cocktail is not saturating ($[S]<Km_a$). In this instance, the concentration of the tested low kcat substrate remains saturating, but the concentration of each high kcat substrate in the cocktail is reduced below the natural Km for that individual substrate. In essence, the assay is shifted from the Profile II conditions before, to a Profile I like scenario. In this instance, the apparent binding energy of [ES] will be higher than that of E + S, which increases the possibilities for inhibition by the competitive low kcat substrate, since the latter is still in a saturating concentration and is favored to bind the active site of the protease due to increased Brownian dynamics. Again, the concentrations are balanced until a clear differentiation pattern (inhibition profile) is noted with inhibition apparent by several of the low kcat substrates.

When an inhibition profile is finally achieved, the differentiated low kcat substrates are promoted to the secondary screening (the second part of the process) for further differentiation, and low kcat substrates that induce no inhibition of the target protease are eliminated from the selection pool. As noted earlier, those low kcat substrates that are also inhibitory are referred to herein as "vehicles" since they are indeed delivered into the active site of the target protease. Since $[S]>Km_a$, it is important to "fix" the concentration of the low kcat substrates (the vehicle) and the concentration of the high kcat substrates for future steps.

*C. Secondary dose screening of vehicles*

In this screening assay, conditions are made to be more stringent to allow for greater differentiation of the inhibitory potential of each of the newly discovered vehicles. The concentration of the high kcat substrates in the TOP cocktail is increased. For example, if a 1% TOP cocktail was used in the initial analysis, a 2% cocktail of the high kcat substrates is now made which includes all substrates from II. which are in the top 2% of kcat values. Our experiments have demonstrated that increasing the cocktail percent increased the differentiation of inhibitor potential within low kcat substrates (Example 3). The vehicles promoted from the initial screenings above are again tested for competitive inhibition ability with the new cocktails. Their concentrations should be set initially as identical to those that were "fixed" in the previous protocols. The concentration of each high kcat substrate in the cocktail is also maintained. The addition of each of the new substrates in the new cocktail should also be equivalent to the concentration of each component in the 1% cocktail. In other words, the cocktail should be prepared as before except that an equal concentration of the additional substrates is added to the mix. For example, if the concentration of each component in the original cocktail was 1 uM, then each subsequent new addition to the mix should also be 1 uM. If the inhibition profile was obtained as in one of the experiments described in Scenario II the starting concentrations are the same as those that were "fixed", except that the number of substrate components in the cocktail is increased. This stipulation applies to all four cases mentioned above.

In principle, greater competition is expected to occur between the vehicle and the 2% cocktail than between the vehicle and the 1% cocktail, since a greater combination of favorable high kcat substrates are now available for the target protease for collision.

At this point, a vehicle inhibitory quotient (V) is optionally determined for the promoted vehicles. This is equal to the potency of inhibition P multiplied by the maintenance of inhibition M, such that V= P ∗ M. P is equal to percentage inhibition in the presence of the top cocktail, and M is equal to percentage inhibition in the presence of the last cocktail employed. In this case, this would be the 2% cocktail. The higher the V value, the greater the inhibition. We have noted in our experiments (Example 3) that the V value provides a convenient method to determine the inhibition as a result of the cocktail. Assigning vehicles to different tiers becomes rather straightforward. For instance, in Example 3 we found that the V values of Z-LLL-AFC, Z-LLY-AFC, and Mu-LY-AFC for inhibition of the purified protease were equal to .585, .552, and 0.452, respectively (using the first cocktail for P and the last cocktail for M). This group defined the first "tier" of vehicles. In the second "tier", we found that B-VPR-AFC, Z-SY-AFC, Z-RR-AFC, and B-LGR-AFC were characterized by V values equal to 0.116, 0.09, 0.09, and 0.07, respectively. A break between the two tiers was clearly identifiable. The utility of this formula is that rankings become fairly straightforward , simple, and quick. An additional measure of "robustness" is also referred to in Example 3, although it is employed purely as a qualitative measure of vehicle inhibitor efficacy.

Moreover, we noticed in our experiments on vehicle inhibition of the *T. solium* cysteine protease (Example 2) that vehicles with less inhibitory potential were marked by lower V values, and were therefore more inclined to lose inhibitory potential in comparison to vehicles with high inhibitory potentials. From a qualitative standpoint, we also noticed that the robustness of inhibition was greater in vehicles with higher inhibitory potential (i.e., M-LY-FMK).

This screening provides an additional level of differentiation between low kcat vehicles. Typically, the same relative inhibitory profile of the vehicles that produced top inhibition in the initial screen is maintained, while vehicles with less inhibitory potential have their previous inhibition values eliminated. If this step is performed, the top tier inhibitory vehicles with the highest V values are promoted to the tertiary dose screening.

*D. Tertiary dose screening of vehicles*

In this step, the concentration of the TOP cocktail is again increased. Following the example currently in use, the vehicles that are promoted from the secondary dose screening compete with a 3% cocktail for the protease's active site. The same comments made above about choosing appropriate concentrations apply for this screening procedure. A note should be made about the selection criteria of vehicles. Inhibition of the target protease's cleavage of components in the high kcat cocktail is the primary criteria for selection. Hence, negligible importance is placed at this point on the kcat variable for the vehicle and more is placed on the inhibitory ability (measured as % inhibition here although it will equate to low Ki). For example, if "a" and "b" are the lowest kcat substrates with "a" having a lower kcat value than "b", it is entirely possible that "b" may be chosen for its competitive inhibitory ability over "a", even though the kcat value of "b" was higher than the kcat value of "a". Again, rankings are based upon percentage inhibition of the target protease's cleavage of the cocktail, typically as determined by the V value, and not upon the kcat value. The low kcat substrates that are used for competition should remain in a bottom percentage of the Step II screen, based upon an arbitrary number chosen by the investigator (e.g., the bottom 20%).

Ideally, these kcat values will be so low in the Step I screen that most of the low kcat vehicles have similar kcat values. The point of this screening process is to identify the vehicles that would be expected to have the lowest Ki regardless of the differences in their kcat values.

*E. Final dose screenings of vehicles*

Additional dose screenings of vehicles are conducted, as necessary or desired. The percentage cocktail of the high kcat substrates is progressively increased and added to the vehicles "promoted" from prior steps, which allows for a means to eliminate the vehicles with less inhibitory potential. The goal is to obtain a data profile where the same vehicles provide similar patterns of inhibition even as the competition from highly cleaved substrates is increased. In other words, those vehicles that are consistently inhibiting the enzyme despite increased cocktail components are vehicles that should be promoted as prime inhibitory candidates. As screenings continue with higher stringency, vehicles that were inhibitory in prior steps should fall out of the inhibition pattern, and the most robust vehicles should remain. Hence, cocktail addition continues until a percentage of the cocktail is reached which will differentiate and rank vehicle inhibition capability without actually having to have determined Ki.

The investigator stops the screenings when confidence is achieved that a list of low kcat vehicles can be consistently ranked as inhibitory of the target protease's cleavage of the cocktail. This selection can be conducted quickly and efficaciously from an original starting point of 8,420 total combinations of potential vehicles, if desired. The final list is a ranking of these vehicles based upon consistent inhibition profiles in the 1%, 2%, 3% cocktails and so forth. The top 10% of these ranked vehicles (or another arbitrary percentage) are chosen for Ki calculation in the next step.

*F. Determination of Ki values for most inhibitory vehicles*

Once a manageable set of candidate vehicles is identified, determination of Ki can proceed through conventional kinetic modeling methods which are already well described in the art. Typically, the inhibitor concentration is experimentally varied from 0.5 to 5.0 times Ki in order to obtain a good value of Ki. Hence, determination of the Ki proceeds by treating the vehicle as one would an inhibitor and the cocktail and individual components of the cocktail as one would a substrate. The aggregate concentration of substrates should be maintained at a saturating level (50 to 100 times Km). Regardless of whether a cocktail or individual high kcat substrates in the cocktail is used for the protease's substrate, the Ki values for the vehicle as a competitive inhibitor should be similar against all substrates, since Ki for an inhibitor on a target protease is a function of the inhibitor and active site and is independent of the substrate.

It is recommended that derivation of Ki proceed through nonlinear fitting, although derivations by suitable methods such as Eadie Hofstee, Hanes, and Cornish-Bowden can also be employed if access to nonlinear computer modeling is not available. Due to increased possibilities for error of magnified nonlinearity for the first method and erroneous interpretations of data at low concentrations and low velocity for the last method, these plots are not preferred over nonlinear methods. Secondary replots of the Lineweaver-Burke method can also be used but are not preferred. However, the classical Lineweaver Burke plots are not typically employed for these or other kinetic determinations in this procedure. A convenient aspect to this calculation is that the Ki values determined for these inhibitors are also equivalent to the vehicle's Km value when it is used as a substrate.

*G. Final ranking*

This step concludes with a ranking of candidate inhibitor vehicles. The lower the Ki value, the higher the ranking attributed to the vehicle. Ki determination is the basis of the final ranking. At this point, the initial panel of substrates has been filtered down to a few low kcat/Km candidates that have inhibitory ability. The advantage of this approach is that Ki determination can be deferred until a massive filtering scheme has been carried out, rendering this manner of ranking the vehicles very expeditious.

*H. Theoretical Advantage of Step III*

The following is a brief summary of the advantages of this step. A more elaborate discussion was set forth in Part I. First, the identified vehicle is marked by tight binding with $ES_B$ and $TS_B$. The fact that high kcat substrates are being outcompeted by the vehicle speaks to the efficiency in which the vehicle binds not only to $ES_B$, but also to $TS_B$. Second whereas this vehicle binds with $TS_B$ and $ES_B$, it is marked by a low kcat, and therefore has no interactions with $TS_R$. Hence $TS_B$ and $ES_B$ are effectively "separated" from $TS_R$ in the vehicle, which suggests that the binding center and reaction center between ES are distinct, as is consistent with a "split-site" model. Thus, the second major advantage is that the experimental addition of a $TS_R$ raising reactive moiety in later steps (Step VII forward) to the vehicle is *unlikely to interfere with the positive binding interactions that the vehicle shares with* $TS_B$ *and* $ES_B$, since the binding and reaction are "separated" in the vehicle. Third, because of its noncatalytic properties, it cannot lower $TS_R$ and therefore, *does not produce a situation which is counteractive to inhibition.* The fourth major advantage of the method is that its simplification to "split-site" like assumptions, allows for a simple system in which the kinetics are not very complicated, the system is not as difficult to study, the predictions are more efficient, and the identification of a bound and potent protease inhibitor is more likely.

*I. Additional observations*

*1. Slow binding vehicles.* Slow binding phenomena relate to those tight binding vehicle inhibitors that do not typically follow the kinetics of reversible inhibitors. In these instances, equilibrium is reached very slowly between the enzyme-inhibitor and the enzyme, and hence it requires at least seconds or minutes to observe the steady state. Despite their noncovalent interaction, slow binding inhibitor affinities for the enzyme are so high that even minor quantities will inhibit the target enzyme. Hence, a very tightly bound inhibitor has a slow rate constant for formation of [EI] which means that a slow rate of inhibition becomes obvious in the kinetic model. However, less tightly bound inhibitors may also exhibit slow binding especially if they bind at a higher rate. Slow binding vehicles can be analyzed by several kinetic models known in the art (Szedlacsek and Duggleby, 1995) which, although limited in number, can often distinguish this phenomenon.

*2. Substrate binding that does not involve P3-P1.* Although it is known that residues that bind the active site of a protease can range from P5 through P4'(Berger and Schechter, 1970), subsite positions P3 through P1 are the most likely candidates for binding and are therefore initially chosen for analysis in the method of the invention. However, the target protease may not utilize the P3-P1 sites for binding. If no inhibition is observed for any of the vehicles, the library can be retooled to screen at sites other than P3-P1. For example, the tripeptide core may be used to screen other triplets such as P1'-P3' sites or P2-P1' sites and so on. If needed, several different parts of the substrate may be screened for binding with the substrate library.

*3. Assay workability.*

a. If any of the substrate or vehicle levels must be raised to millimolar levels in order to obtain saturation, then ionic strength effects, nonspecific binding, and dead-end inhibition may cause misleading kinetic results.

b. A substrate concentration of 10 times Km may not be sufficient for saturation of the enzyme. Hence, it is suggested that when saturation conditions are required as in the case of obtaining a Profile II scenario, substrate concentrations should be employed that are greater than 10 times Km, preferably greater than 50 times Km, more preferably greater than 100 times Km.

c. Contamination of reagents, substrate inhibition, or steady-state influences may also cause nonlinear effects and should be guarded against.

d. A high kcat substrate may serve to activate the enzyme (like an exogenous activating component would) and complicate the interpretation of the kinetic results.

Step III for a crude mix proceeds as it would for a purified protease, except that Vm is used instead of kcat.

*Step IV: Natural inhibition tests*

Here, the leading candidate vehicles are tested for their capacity to inhibit cleavage of natural substrates. In Example 4, a lead vehicle for the *T. solium* cysteine protease, suc-Leu-Tyr-AFC was marked by its ability to inhibit the protease's natural cleavage of human IgG, as noted by Western blot analysis.

A natural substrate that might be expected to be a target for cleavage by the protease is chosen based upon the biology of the target protease. For example, if the protease is located in the lysosomes (e.g., a cysteine protease), target substrate molecules may include those that are imported into the lysosome (e.g., albumin). If the protease is suspected of degrading physiological membranes (e.g., collagenase activity), collagen may be chosen as a natural substrate. Preferably, a mass spectrometric analysis is used for analysis of the cleavage reactions due to its speed. The loss of common cleavage points on the natural substrate when the protease is in the presence of the vehicle is a hallmark of the vehicle's potency.

This information is meant only to add an extra qualitative dimension to the potency of ranked inhibitors for potential customers, but is not meant to serve as a selection criterion for promotion or ranking of vehicles. Hence, this step is optional and should be conducted only when time and resources are available.

*Step V: Assessment of noncatalytically bound vehicles to identify transition state properties*

In this step, which is optional, the top newly identified peptide core is screened for its potential to participate in transition state binding: this property is gauged by observing the effect of varying the chemical structure of the linked chromophore, and of the peptide core. If it is decided later to employ the second best peptide core or other peptide cores, then they too, can be screened for transition state character. The core itself is not likely to participate in transition state binding since the peptide core has a tight binding affinity and most likely participates in lowering ES only; nonetheless, some compounds might be identified that do participate in $TS_B$. That is, the core may form bonds that are strengthened at TSB. The process to conduct the transition state assessment is substantially identical to the process described below in Step VII (C), and hence the more in-depth details will be left for that section. In essence, the vehicle is treated as though it is an inhibitor for which Ki is known yet kcat is too low to accommodate an accurate kcat/Km. This invention step effectively borrows a kcat/Km values for each inhibitor and its same-chromophore analogs in a homologous series of peptides (called the inhibitor series), from the respective members of the same series of peptides (called the parallel series) linked to a different chromophore, where each member of the latter series is known to be at least slightly cleavable. This enables an interseries plot of inhibitor Ki values plotted against the respective analog Km/kcat of each respective member of the parallel series.

First, a new series of analogous substrates that carry a different detection group is obtained or synthesized.

For example, if Z-Leu-Leu-Leu-AFC is the tested vehicle, an analogous substrate could be Z-Leu-Leu-Leu-X where X represents another labile detection group (not an inactivating reactant, i.e., unlike in Step VII) such as 7-amino-4-methylcoumarin (AMC). The new X group is preferably more labile than original fluorogenic group, and cleavage of the X group is a detectable event. This part of the procedure is challenging since the peptide core is by its nature not conducive to catalysis. Having access to a wide variety of X groups when designing the analogous substrate is helpful as is access to a very sensitive detection instrument. In the above example, the X chosen is one that is more cleavable from Z-Leu-Leu-Leu than AFC.

Secondly, individual residues in the peptide core of the vehicle and the newly synthesized substrate are varied for a single position in the peptide (e.g., for a Leu-Tyr-AMC core, one might assess Pro-Tyr-AMC, Phe-Tyr-AMC, Ile-Tyr-AMC, Val-Tyr-AMC, etc., in parallel with their AFC analogs): the theory is that these analog series probe the transition state. As described below in Step VII (C), log (Ki) of the vehicle (the AFC-linked peptides here) is plotted against log(Km/kcat) values for the respective corresponding analogous substrates (the AMC-linked peptides here) that have been measured in this step. Preferably the interseries plot contains a dozen or more data points. The data points, which may be diffusely distributed in the plot, are then subjected to a line-fitting exercise to identify at least one, and more usually as many as three or more nonidentical, coexisting straight lines; each point may be assigned to more than one of the identified lines. Any of various statistical line-fitting algorithms may be used; such as are commonly known in the art. For each line the regression value, also known as the correlation value R, is multiplied by the slope to obtain a transition state score, using the following equation, as further described and discussed in Step VII.

$$TSS_X = R/(ABS(1-M) * 1/P)$$
$$= (R * P)/(ABS(1-M))$$

where:

R= regression value
M= slope
P = number of points on the line
X = a selected inactivating reactant

The use of P is optional, since it is not critical to assessment of the inhibition mechanism. In cases where m = 1 (the highly unlikely case where rate determining step inhibition has been identified), the value of "0.99" should be employed in replacement of "1", in order to prevent division by 0.

It is expected that the line with the highest transition state score will be the one having a slope closest to 1.0, since this represents the ideal relationship between transition state analogs for the scission step. The higher the transition state score, the greater the possibility that a binding event is occurring in the transition state of the vehicle. Moreover, if there is not a correlation at the transition state, a correlation at the ground state will be examined. In Step V, the crude mix is treated in a manner identical to that utilized for the purified protease, except that Vm is employed instead of kcat. Consequently, Km/Vm versus Ki is plotted instead of kcat/Km versus Ki.

*Step VI. Selectivity study of vehicles*

In this step, which is optional, the top chosen vehicles are examined for their ability to inhibit only the target protease in comparison to a panel of other proteases within the same and differing active site mechanistic classes. Preferably, different human proteases representing the full complement of protease classes should be chosen, including cysteine proteases, serine proteases, metalloproteases, aminopeptidases, aspartic proteases, and so on, although smaller panels can also be used. The greater the number of these proteases, the more versatile the method will be. The substrates used in these tests (against which the top vehicles are tested) should each be representative of the respective protease class. For example, Z-Phe-Arg-AFC is a representative substrate for cysteine proteases and Z-Arg-AFC is a representative substrate for serine proteases. To perform this assay, the tested vehicle is set up at a concentration several fold greater than its deduced Ki value, such that it inhibits the target protease as close to maximal inhibition (ideally 100%) as possible. This is an instance of Profile II where the apparent binding energy of the vehicle/enzyme complex is expected to be greater than the energy of the enzyme and vehicle by themselves (thus, [I]>Ki). The substrate for this test is based upon the class of the identified target protease. For example, if the target enzyme is a cysteine protease, Z-Phe-Arg-AFC would be a suitable substrate to employ for this initial test.

Consistent conditions for protease, vehicle, and representative synthetic substrate additions are preferably maintained from assay to assay. The protease is the last component added to the assay mix. Those vehicles that selectively inhibit only the target protease are noted, and a selectivity ranking is established. Ideally, the selectivity ranking parallels the vehicle ranking obtained from Step III.

In any event, the establishment of this selectivity ranking is not meant to serve as a criterion to eliminate any of the vehicles, since each vehicle is only a part of the final inhibitor molecule, and since the addition of an inactivating reactant to this peptide core also carries the potential to impart selectivity to the vehicle. Instead, this step serves as a "checkpoint" to provide selectivity information for the vehicle at this point in the process.

*Step VII. Synthesis and collection of vehicle/protease inactivator combinations that form a potentially "potent" transition state vehicle*

Step VII is optional, and the general protocol may be employed for example after Step II, not at all, or using non-peptide analogs of an effectively inhibiting peptide core.

At this point, an elimination criterion has been employed in order to determine one or more low kcat vehicles, expeditiously and efficaciously, which concomitantly have the lowest possible Ki values for substrate binding (in our example, utilizing sites P3-P1) in the enzyme active site. Hence, these vehicles exhibit the lowest possible apparent binding energy states for the target protease binding positions (S3-S1) when they are complexed with it. Some selectivity information has also been gathered by the vehicles' preferences for the target protease in comparison to a panel of other substrates. Moreover, depending upon the results of the transition state score analysis in Step V above, it is possible (although not necessary) that these vehicles may even have noncovalent and partial transition state character (which would be especially noticeable in cases where the enzyme mechanism includes a great number of intermediate transition states). In either event, these substrates are now proven "vehicles" with a low apparent binding energy state [EV], a feature that can be exploited in order to design and deliver a reactive intermediate (the "inactivating reactant") that is potentially able to lock up the active site in a manner consistent with transition state inhibition. The inactivating reactant can bind the target protease reversibly or irreversibly, although in many applications it is preferable that the inactivating reactant binds to the target protease irreversibly. The binding may be covalent or noncovalent. For example, common transition state analogs for cysteine and serine proteases involve a permanent tetrahedral adduct. The focus in this step is thus to identify inactivating reactants that can impart transition state character to the vehicles such that the overall inhibitor will be marked by an extremely low Ki value. The combination of vehicle and inactivating reactant in a single compound is referred to herein as a "protease inactivator". Those protease inactivators with transition state properties will be termed "transition state protease inactivators" or TSPIs.

The choice of which inactivating reactant to link to the C-terminus of the P1 amino acid on the vehicle is an arbitrary one, and is based upon whether or not the mechanism of the identified protease is expected to be conserved. For example, in cases where tetrahedral adducts are important as rate limiting steps (e.g., cysteine and serine proteases), molecules which will have the potential via a nucleophilic or electrophilic attack (based upon the molecule) to mimic the tetrahedral rate limiting step are preferred. In the case of Example 1, we employed a fluoromethylketone and vinylsulfone that are expected to make tetrahedral adducts with the active site. Nevertheless, a wide variety of excellent electrophilic and nucleophilic compounds is available commercially for linking to the top candidate vehicle(s), such as fluoromethylketone, chloromethylketone, diazomethylketone, vinyl sulfone, epoxide, ketoamide, chloromethane, Michael acceptor, peptide aldehyde, epoxide, acetaldehyde, nitrile, hydroxymate, cyclopropenone and epoxysuccinamide. They can be obtained from several vendors including Enzyme Systems Products.

The selection for a tight binding vehicle in Step IV should diminish the need for a highly reactive inactivating reactant. This is an advantage especially for drug design since highly active inactivating reactants can impart toxic effects in the physiological system. For example, fluoromethyl ketone (FMK) is an inactivating reactant which is important for inhibition of the target cysteine protease in Example 5. However, FMK is not preferred as part of a final drug compound since it is known to carry potential toxicity due to its liberation of fluorocitrate metabolites. Thus, a variety of inactivating reactants, some of which are highly active and others that are not, should be included as a part of the medley of inactivating reactants selected for linking to the vehicle. It is preferred to choose inactivating reactants that are not highly reactive, given that the tight binding peptide core will already provide a mechanism for inhibition. It is even more important to choose inhibitors which are inactive in the absence of enzyme. It is also preferable to include inactivating reactants that reversibly inhibit the protease, in order to increase their safety in the event of overdosing or improper diagnosis. The physiological concern is that a toxic side effect, due to an irreversible binding event, cannot be reversed with a competitive inhibitor. This is especially the case for the use of inhibitors in long term treatment for diseases and disorders such as osteoporosis, Alzheimer's' disease, cancer or arthritis.

However, it has been noted by several authors in the field (McKerrow et al., 1999), that toxicity arising from irreversible inhibition is less of a concern when short courses of treatment are required, such as in the case for microbial infections. The advantage of an irreversible inhibitor, of course, is that the covalent linkage modification of the target active site is permanent and potent, and cannot therefore be outcompeted. Additionally, the disadvantage of purported toxicity of an irreversible inhibitor may also not be an issue when, as here, the design model here is set up to produce highly specific and bound inhibitors (i.e., the peptide core is extremely selective and tightly bound). Moreover, these inhibitors should be useful in low, nontoxic dosages due to their potency. Thus there are cases where irreversible drugs are preferred and hence, this decision should be based upon the physiology of the disease as well as the expected metabolism of the inhibitor in the body. These toxicity concerns are more pressing when optimizing the compound into a drug later in the drug discovery pipeline. Although there is no single answer to the type of inhibitor (i.e., reversible or irreversible) preferred, it should be noted that the general preference in the scientific community is for reversible inhibitors. However it should in any event be understood that invention is not limited to the choice or relative reversibility of any particular inactivating reactant.

The first step of this procedure is to link a variety of inactivating reactants with the top vehicle (s). The second part of the procedure determines, for each newly synthesized protease inactivator, the concentration of inhibitor required to achieve 100% inhibition of the target protease ($EC_{100}$), yielding a ranked list of potential transition state protease inactivator (TSPI) candidates. The third part of the procedure identifies which of these candidates are indeed transition state protease inactivators (TSPIs), and yields a final ranked list of TSPIs. In the event that the mode of action of the protease inhibitor is inhibition of a ground state catalytic effect, the best ground state protease inhibitor is to be selected.

*A. Covalent linkage of inactivating reactant to vehicle*

The linking of the inactivating reactant to the vehicle follows the commonly implemented procedures of amino acid linking as reported in art of organic synthesis. The inactivating reactant is typically linked to the C-terminus of the peptide core, replacing the detection group that had been linked to the vehicle in earlier steps. There is no limit as to the variety of inactivating reactants which can be linked to a top vehicle to yield potential TSPI's; indeed, the greater the variety of inactivating reactants tested, the greater the possibility for successful development of a TSPI. Testing more than one top vehicle increases the probability for a successful "hit" as well. However, when resources are limited, it is preferable to develop a large library of compounds composed of the *top* vehicle linked to any of numerous different inactivating reactants, rather than multiple libraries of different vehicles using any of a smaller number of inactivating reactants.

At this point, the top vehicle for inhibition which has favorable binding with $ES_B$ and $TS_B$ has been identified. The focus now shifts to the search for inactivating reactants that will be able to bind exclusively to $TS_R$ (or $GS_R$, if ground state destabilization is the mechanism for catalysis). Thus, the binding portion of the vehicle (i.e., the peptide core) should remain constant, while inactivating reactants are screened for binding to $TS_R$ (or $GS_R$).

The two following examples (Situation 1 and Situation 2) are illustrative. The top candidate vehicles are ranked in accordance with the criteria from step III. In Situation 1, which is depicted in Fig. 3, the top ranking vehicles are relatively close in amino acid composition. This is the more usual case, since significant homology would be expected in the active sites of the most active vehicles, especially if all 8,420 amino acids were screened. In Situation 2, there is a large variation in the peptide amino acid core among the top ranked vehicles.

Top ranked vehicles from Step III

| Situation 1 | Situation 2 |
|---|---|
| 1. Z-LLL-AFC | 1. Z-LLL-AFC |
| 2. Z-LLY-AFC | 2. Z-VPR-AFC |
| 3. Z-LLW-AFC | 3. Z-LLW-AFC |
| 4. Z-LLR-AFC | 4. Z-VPP-AFC |
| 5. Z-VLL-AFC | 5. Z-PVV-AFC |
| 6. Z-LLP-AFC | 6. Z-PVL-AFC |

Z-LLL-AFC is chosen as the primary peptide core. Z-LLL would then be linked to each of a very high number of inactivating reactants in turn. The result is a series of compounds having a homologous peptide core (i.e., the same amino acids in the same order) but different inactivating reagents. If resources are available, it is preferable to have several dozen different inactivating reactants, each linked to a copy of the peptide core. Optionally, this same procedure can be applied to the second highest ranking vehicle in order to increase the chances of finding a potent protease inactivator. The result for situation 1 using five different inactivating reactants U,V,W,X,Y, and Z is as follows:

| Most preferred linked list for Situation 1 (unranked): | Next preferred linked list for Situation 1 (unranked): |
|---|---|
| Z-LLL-U | Z-LLY-U |
| Z-LLL-V | Z-LLY-V |
| Z-LLL-W | Z-LLY-W |
| Z-LLL-X | Z-LLL-X |
| Z-LLL-Y | Z-LLY-Y |
| Z-LLL-Z | Z-LLY-Z |

For situation 2, the second best vehicle peptide core (Z-VPR-) could be derivatized in addition to the best peptide core, Z-LLL-, if desired. The total number of permutations for each top-ranked vehicle thus reflects the number of inactivating reactants being tested. Examples of these combinations to be tested in Situation 1 include Z-Leu-Leu-Leu-fluoromethylketone, Z-Leu-Leu-Leu-vinylsulfone and Z-Leu-Leu-Leu-ketoamide.

*B. Inhibition assays to determined candidate transition state protease inactivators*

Once the protease inactivators are synthesized, they are tested for the concentrations at which they induce 100% inhibition of the target protease ($EC_{100}$) and ranked accordingly. The TOP cocktail that was employed in the final vehicle selection step of Step III is employed as the source of competition for the inhibitors. The result is a ranking of candidate transition state protease inactivators with the highest ranking protease inactivators having the lowest $EC_{100}$ values, i.e., the inactivator that remains effective at the lowest concentrations is the best in the rankings. The process includes the following steps.

*Step one: Initial screening.* Each protease inactivator is first incubated with the TOP cocktail of high kcat substrates that provided the final differentiation of vehicles in Step III. For example, if a 7% high kcat cocktail was employed in the final screenings of Step III, it is convenient to use it as the starting source of competition here. The protease inactivator is incubated at a concentration equal to the concentration of each individual high kcat substrate component in the cocktail. Positive and negative controls are established as described in Example 5, and % inhibition calculated accordingly.

*Step two: Inhibitor dilution studies and determination of $EC_{100}$.* It is possible (although unlikely) that a ranking of inhibitors can be established at this arbitrary starting point as long as at least one of the protease inactivators inhibits the target protease by 100%. In that event, that inhibitor is the top ranked inhibitor followed by inhibitors that have the lower inhibitory potentials (or lowest $EC_{100}$ values). However, it is more likely that numerous inhibitors will inhibit the target protease by 100% at the selected concentrations. In that event, the concentration of each of those protease inactivators is then lowered until an $EC_{100}$ can be determined. For example, 10-fold dilution increments are a good point to start, but the degree of dilution is at the discretion of the investigator. The result of the assay is a ranked candidate list of protease inactivators. The highest ranked inhibitors in this step are those that have the lowest $EC_{100}$. That is, they exhibit complete inhibition of the protease at the lowest inhibitor concentration.

| Most preferred $EC_{100}$ list for Situation 1: | Next preferred $EC_{100}$ list for Situation 1: |
|---|---|
| 1. Z-LLL-X | 1. Z-LLY-V |
| 2. Z-LLL-Y | 2. Z-LLY-W |
| 3. Z-LLL-Z | 3. Z-LLY-X |
| 4. Z-LLL-U | 4. Z-LLY-U |
| 5. Z-LLL-V | 5. Z-LLY-Z |
| 6. Z-LLL-W | 6. Z-LLY-Y |

*Step three: Determination of inhibitor Ki and mode of inhibition, and re-ranking according to Ki.* Ki for the top ranked protease inactivators (i.e., those with the lowest $EC_{100}$ values) is now determined. This is a time-consuming step, and it is left to the discretion of the investigator to decide how many candidate compounds should be promoted to this step. Indeed, one of the most significant advantages of this method is that it defers the Ki calculation to a point "late" in the method, when substantial screening has already occurred, thereby reducing the number of candidate inhibitors with respect to which this calculation needs to be performed.

Inhibitor concentrations are preferably at least .5 to 5 times the Ki in order to determine the Ki value. Any of the high kcat cocktail substrates or the full cocktail can be employed in this step, as Ki of the inhibitor for the enzyme is the same regardless of the substrate. Nevertheless, it is recommended that Ki be determined independently using at least 3 separate substrates in order to have confidence in the calculation. Nonlinear fitting or the Dixon plot can be employed to determine Ki of the top ranked protease inactivators. As discussed in Step III, derivations of the Eadie Hofstee, Hanes, Cornish-Bowden and secondary replots of the Lineweaver Burke methods are not recommended for the determination of Ki values over nonlinear fitting. Direct Lineweaver Burke plots are not to be used for Ki determination. If time is not limiting, pseudo-first order rate constants (Kapp) can be assessed from slopes of time versus the plot of the natural log of percent remaining activity. Finally, a plot is constructed using either Eadie Hofstee or Hanes to determine the mode of inhibition.

These methods are employed to assess the type of inhibition, i.e., competitive, noncompetitive, uncompetitive, or irreversible inhibition. Consistent with the procedures performed thus far, the inhibition mode is expected to be either competitive or irreversible, but could alternatively operate by uncompetitive or noncompetitive mechanisms. The decision about the mode preferred for inhibition needs to be made by the investigator, based upon the physiological goal of the target drug as discussed above (e.g., long term treatments may call for the use of less toxic reversible inhibitors whereas short term treatments can utilize the more potent irreversible inhibitors. Thus this step can be useful to determine which type of candidate inhibition is preferred. The method to determine irreversible inhibition is well known in the art. The protease inactivators are at this point re-ranked, such that those with the lowest Ki become the top ranked protease inactivators. In most instances, a low $EC_{100}$ value will correlate with a low Ki value. Thus, the resulting ranking may be the same as that obtained in the $EC_{100}$ calculations, or it may be slightly different, and thus determination of the Ki value is optional, since it can be inferred from the calculated $EC_{100}$ value. In rare instances, the Ki ranking is significantly different from the $EC_{100}$ ranking, and because of that possibility the Ki determination and ranking are preferred steps.

It is again important to emphasize that these assays need only be conducted for candidate inhibitors that represent the single TOP vehicle linked to different inactivating reactants (e.g., the "most preferred" compounds for Situation 1, which all have the TOP peptide core, LLL, as their peptide core). Investigation of the second top ranked vehicle ("next preferred" compounds for Situation 1; LLY is peptide core) is optional.

Returning to our illustration, assume that Ki values were determined for the top 3 compounds on the $EC_{100}$ lists. The typical result of the experiment is that we obtain the identical list. Although it is unlikely that the $EC_{100}$ ranking should deviate from the Ki ranking, it is preferred to perform the assessment anyway due to complication by diverse kinetic variables. The highest ranked inhibitors have the lowest Ki.

Most preferred Ki list for Situation 1:
1. Z-LLL-X
2. Z-LLL-Y
3. Z-LLL-Z

Next preferred Ki list for Situation 1:
1. Z-LLY-V
2. Z-LLY-W
3. Z-LLY-X

*C. Determination of transition state protease inactivators (TSPI's)*

Mechanistically, the top candidate protease inactivators are either inhibiting the enzyme conformation that corresponds to the substrate transition state or are inducing inhibition in the enzyme geometry that complements the substrate ground state. The mechanism of inhibition depends entirely upon the specific enzyme, as already discussed in the theory section. Candidate inhibitors are first assessed for their potential as transition state analogs, thereby providing an additional selection criterion (i.e., transition state character) from which to select the top candidate protease inactivators. A "transition state score" is employed to assess transition state inhibition. If transition state mimicry turns out to be their mode of inhibition, the candidate protease inactivators are refined for even further potency in transition state inhibition. The process thus offers the opportunity for fine tuning at a molecular level to identify inhibitors that most potently mimic a transition state and inhibit enzyme activity as close to the rate determining step as possible. Preferably, protease inactivators are identified that inhibit the enzyme at the rate determining step.

To evaluate transition state character, the correlation between the potency of inhibition by the protease inactivators and the cleavage efficacy of a congeneric series of analogously substituted vehicles is assessed. This correlation has been well documented in the art. If a correlation is noted, it suggests that enzyme ligand interaction binding energies can be employed to drive inhibition in a manner similar to that employed to drive catalysis. In other words, the mechanistic features responsible for substrate hydrolysis, on the one hand, and enzyme inactivation by the inhibitor, on the other, may be similar.

In returning to Situation 1 in the illustrative example, Z-LLL-X was ranked highest as the inhibitory compound of choice based upon its Ki value. This highest ranked compound is selected for further analysis and becomes the "parent" compound. The object is to analyze the transition state character of the inactivating reactant attached to Z-LLL, which in this example is "X". Without intending to be limited by any particular theory or mechanism, it is desired that the inactivating reactant specifically raise $TS_R$ at the transition state, and not affect $ES_R$ at the ground state. Consistent with the belief that the interactions of Z-LLL of the peptide core are now conserved at $ES_B$ and $TS_B$, excellent binding ability has already been observed (as evidenced by the low Ki value). Moreover, this core has the additional advantage that it is noncatalytically bound and is therefore not expected to lower $TS_R$ (which would counteract the potency of the inactivating reactant). To confirm this fact, the following steps are performed to determine whether the inactivating reactant (X) induces inhibition at the transition state ($TS_R$) without interaction with the ground state. Testing of the second and third ranked inactivating reactants (i.e., Y and Z in the Situation 1 example) can also be performed, if desired.

*Step one: Ranking of transition state protease inactivators*

*1. Ki for parent inhibitor and kcat/Km for parent vehicle*

The top inhibitor and its analogous vehicles will now be referred to as the "parent inhibitor" and the "parent vehicle". For example, in situation 1 of the illustrative example, the parent vehicle is Z-LLL-AFC and the parent inhibitor is Z-LLL-X. Kcat, Km, and Vm for the vehicle were already determined using conventional kinetic methods as discussed in Step III. Ki was determined for the protease inactivators in Step VII(B). As noted above, nonlinear fitting is preferred over the methods of Eadie Hofstee , Hanes, Cornish-Bowden and secondary replots of Lineweaver-Burke, and direct Lineweaver Burke plots are not recommended for any calculations to determine kcat, Km and Vm. For example, if the top vehicle is Z-Leu-Leu-Leu-FMK, then the kcat, Km and Vm for Z-Leu-Leu-Leu-AFC are determined (given that AFC is the congeneric fluorometric substrate group). Ki of Z-LLL-FMK has already been ascertained by Step VII (B) above.

*2. Construction of a Km/kcat vs. Ki profile and a Km/kcat vs. Km profile for structurally analogous protease inactivators and their vehicles*

Kinetic profiles are developed with adaptations. Although a library of 8420 vehicles containing chromophoric groups is preferred for the assays in Steps II and III, it is more economical to synthesize the corresponding inhibitors (containing inactivating functionalities instead of chromophores) only as needed. Thus in the absence of the full parallel "8420 library" of inhibitors, permutations of the promoted best inhibitors are selected and developed as described below. The principle is to produce a systematically varied group of peptide cores from which to identify TS-related characteristics through line regression exercises on kinetic data plots.

Inhibitors characterized by single amino acid substitutions vis a vis the parent inhibitor are synthesized along with identically substituted vehicles if they do not already exist. Although is preferable in the core to choose mono-amino acid substitutions, more extensive substitution is allowed as long as it is the same in the inhibitor and the vehicle. Inhibitor/vehicle pairs contain substitutions in the identical position of the peptide portion of the inhibitor and the vehicle. In Situation 1 of the illustrative example, if "X" for the top vehicle was FMK, such that the top protease inactivator after the Ki ranking was Z-Leu-Leu-Leu-FMK, then examples of conservatively substituted inhibitor/vehicle pairs include Z-Leu-Leu-Tyr-FMK/Z-Leu-Leu-Tyr-AFC, Z-Phe-Leu-Leu-FMK/Z-Phe-Leu-Leu-AFC, Z-Leu-Pro-Leu-FMK/Z-Leu-Pro-Leu-AFC. Examples of nonconservatively substituted inhibitor/vehicle pairs include Z-Leu-Leu-Arg-FMK/Z-Leu-Leu-Arg-AFC, Z-Leu-Lys-Leu-FMK/Z-Leu-Lys-Leu-AFC, Z-Asp-Leu-Leu-FMK/Z-Asp-Leu-Leu-AFC. The term "nonconservatively" here reflects permutations that might introduce considerable differences in the behavior of the analog peptide core relative to the parent peptide core, for example, replacing a relatively nonpolar amino acid residue in a parent peptide with a much more polar amino acid residue.

After they have been synthesized, Ki is determined for these analogous protease inactivators if it is not known already. Kcat, Km, and, optionally, Vm are determined for the related vehicles. These values are plotted in the next step, and used to determine transition state character of the parent inhibitor. The ultimate goal of this portion of the method is to determine whether the parent inhibitor (in Situation 1, this is Z-LLL-X) is inhibiting specifically at the transition state (presumably, raising $TS_R$). For each inactivating reactant, e.g. X, a sufficient number of inhibitor/substrate pairs should be synthesized so that an acceptable line can be constructed in the linear regression analysis.

In determining what amino acid substitutions to make to generate the optimal inhibitor/vehicle analog pairs, the investigator can be guided by information previously gathered about the parent inhibitor and related compounds.

*Case 1: Choice is made based upon prior vehicle inhibition*

It is possible that upon review of the results of vehicle inhibition data, similar "motifs" of inhibition are found. This is illustrated using our Situation 1 most preferred example, where Z-LLL-X is the protease inactivator (parent inhibitor) and Z-LLL-AFC is the parent vehicle. The data show several vehicles that were ranked high and differed from the parent by only one amino acid. This is an optimal case, but is also a likely one considering the conservation in binding. Analogous pairings are constructed based upon that list. For example, if LLY-AFC, LLW-AFC, LLR-AFC, VLL-AFC, and LLP-AFC were all inhibitory and ranked high on the vehicle list, they will be used for the substitutions. Indeed in this case, it appears that L is preferred at P2.

The following kinetic parameters would be determined in this step:

|  | kcat/km for: | Ki for: |
|---|---|---|
| a. | LLL-AFC | LLL-X |
| b. | LL**Y**-AFC | LL**Y**-X |
| c. | LL**W**-AFC | LL**W**-X |
| d. | LL**R**-AFC | LL**R**-X |
| e. | LL**V**-AFC | LL**V**-X |
| f. | LL**P**-AFC | LL**P**-X |
| g. | LL**V**-AFC | LL**V**-X |
| h. | LL**Q**-AFC | LL**Q**-X |
| i. | LL**T**-AFC | LL**T**-X |
| j. | LL**S**-AFC | LL**S**-X |
| k. | LL**T**-AFC | LL**T**-X |
| l. | LL**M**-AFC | LL**M**-X |
| m. | LL**G**-AFC | LL**G**-X |
| n. | LL**E**-AFC | LL**E**-X |

*Case 2: Hints are used to choose the substitutions*

Review of the vehicle data may not yield a list in which there is only one amino acid difference. In that case, the prior vehicle data should be studied in more depth. For example, if it is noticed that L is prominently at P2 in most vehicles with inhibitory character, then L will be employed at P2. If it is noticed also that V at P1 has an inhibitory effect, this may be a substitution that would be attempted (e.g., Z-VLL-AFC and Z-VLL-X). Similarly, if R at P3 was noted to provide inhibition in the vehicle list, then a similar pairing will be employed (e.g., Z-LLR-AFC and Z-LLR-X).

*Case 3: No hints are available from the vehicle list*

In this case, substitutions are fully within the investigator's discretion. Substitutions can be made at any or all positions of the binding vehicle. These substitutions can either be conservative or nonconservative, but it is preferable to start with conservative changes (e.g., hydrophobic amino acids for hydrophobic amino acids, or polar amino acids for polar amino acids).

*Case 4: Truncation*

It may also be desirable to test dipeptide inhibitor/vehicle pairs, or single amino acid peptide cores. For example, Z-LL-AFC and Z-LL-X can be employed in Situation 1 of the illustrative example.

*3. Determination of whether inactivating reactants are inhibiting at the transition state or the ground state: Plots of. log(Ki) of the inhibitor vs. log(Km/kcat) of the vehicle, and log(Ki) of the inhibitor vs. log(Km) of the vehicle*

For the class of inhibitor compounds (derived from a single parent) containing a particular inactivating reactant, log(Ki) of the inhibitor is plotted against log(Km/kcat) of its related vehicle. Optionally, a plot of log (Ki) of the inhibitor versus log(Km) of the vehicle is also prepared. Preferably, one of each of these plots is constructed for each individual inactivating reactant analyzed.

For example, in Situation 1 of the illustrative example, the Ki ranking showed Z-LLL-X, Z-LLL-Y and Z-LLL-Z as being the first, second and third ranked inhibitors. Thus, each of them could serve as a parent inhibitor. Assuming that Z-LLL-X is the first parent inhibitor to be examined (with Z-LLL-AFC being the parent vehicle), that the analogous inhibitor/vehicle pairs were synthesized as described above (i.e., inhibitor/vehicle pairs Z-LLY-X/Z-LLY-AFC, Z-LLW-X/Z-LLW-AFC, Z-LLR-X/Z-LLR-AFC, Z-VLL-X/VLL-AFC, and Z-LLP-X/Z-LLP-AFC), and that the kinetic constants were calculated, log(Ki) of the inhibitor is plotted against log(Km/kcat) for the vehicles for each of the pairs on the same plot, yielding information on the potential transition state character of inhibitors containing X as the inactivating reactant. Likewise, log(Ki) versus log(Km) can be plotted for the vehicles to assess the potential ground state character of the inhibitors containing X as the inactivating reagent. That is, two plots are made for inactivating reactant X: (1) log (Ki) versus log (Km/Kcat) and (2) log (Ki) versus log (Km)).

Optionally, additional plots could be constructed for inactivating reactants Y and/or Z, using Z-LLL-Y/Z-LLL-AFC and structurally analogous inhibitor/vehicle pairs, and Z-LLL-Z/Z-LLL-AFC and structurally analogous pairs, respectively, to assess the transition state and/or ground state character of inhibitors containing Y and/or Z.

At this point, a general assessment is made as to whether or not the inactivating reactant for the parent inhibitor of interest is specifically acting to inhibit as a transition state inhibitor or as a ground state inhibitor. As discussed previously in the background section, enzymes can employ either ground state destabilization or transition state stabilization (or a combination) to drive catalysis. An effective inhibitor with low Ki may bind to the transition state of the enzyme or to the ground state of the enzyme, depending on which mode of catalysis is used by the enzyme. It should be clear by now that the method of the invention assumes that the mode of catalysis for the protease (i.e., transition state destabilization, ground state destabilization, or both) is not known in advance. Indeed, an important strength of the method is that this information is both obtained and then capitalized upon empirically during the practice of the method.

To determine the mechanism by which the inactivating reagent is working, linear regression is performed on each plot and a simple comparison of the R value (i.e., statistical line correlation metric) is made between the two plots. If the R value is greater for the log (Ki) versus log (Km/kcat) plot, then inhibitors containing the inactivating reactant inhibit protease activity by acting on the transition state. Conversely, if the R value is greater for the log (Ki) versus log (Km) plot, then inhibitors containing the inactivating reactant inhibit protease activity by acting on ground state. Assuming the correlation is greater for the log (Ki) versus log(Km/kcat) plot (i.e., transitions state interaction, which is the typical case), the transition state score is calculated as described immediately below. If, instead, the R value is greater for the log (Ki) versus log (Km) plot, ground state inhibition is at work and investigator should skip to "step three" below, which further analyzes ground state inhibition.

It is likely that the mode of inhibition will be the same whenever the peptide core component of the inhibitors is identical. For example, low kcat parent inhibitors Z-LLL-Y and Z-LLL-X will both typically act as either transition state inhibitors, or as ground state inhibitors. As between the two modes of inhibition, transition state inhibition is more likely as low Ki values have been found to be associated with transition state inhibition more often than ground state inhibition.

Although unlikely, it remains possible that inhibitors that include X as the inactivating reactant, for example Z-LLL-X and its structurally analogous inhibitors that contain X, may have a better correlation for the transition state plot (i.e., log (Ki) versus log (Km/kcat)) than the ground state plot (i.e., log (Ki) versus log (Km), whereas the inhibitors that contain the same vehicles but include Y as the inactivating reactant, i.e., Z-LLL-Y and its structurally analogous inhibitors, might have a better correlation for the ground state plot than the transition state plot. Z-LLL-X is predicted to be acting as a transition state inhibitor and would move to the next process step below. Z-LLL-Y, on the other hand, is predicted to be acting as a ground state inhibitor and would move to "step three" below. The question arises: if only one inactivating reactant is to be further pursued, should the transition state inhibitor Z-LLL-X (or one its analogs) be pursued, or should the ground state inhibitor Z-LLL-Y (or one of its analogs) be pursued. One option is to always pursue the transition state inhibitor. However, the preferred practice is to pursue the inhibitor having the lower Ki value, whether it is a transition state inhibitor or a ground state inhibitor. If Z-LLL-X has a lower Ki value than Z-LLL-Y, it is preferred that Z-LLL-X is promoted to the next step. On the other hand, if Z-LLL-X has a higher Ki value than Z-LLL-Y, Z-LLL-Y should be pursued in preference to Z-LLL-Y because of its lower Ki value. The rationale for choosing to pursue the inactivating reactant that yields the parent compound with the lower Ki value (as opposed to choosing based simply upon the *mechanism* of inhibition) is that lower Ki inhibitors are more potent. As a result, in the context of drug design, the lower Ki inhibitor permits lower dosages to be used effectively. In addition, the tight binding reflected in the lower Ki also implies a certain level of selectivity.

*4. Determination of transition state score for an inactivating reactant*

If transition state inhibition is pursued, a transition state score (TSS) can be determined for the inactivating reactant that characterizes the inhibitor based on the plot of log(Ki) versus log (Km/kcat) for the inhibitor and its structural analogs, as detailed above. Inhibitors with high transition state character are expected to be highly specific and potent for the active site of the target protease, as well as demonstrate some selectivity, at least in principle. The potency of inhibition is an additional feature which is incorporated into the final "Inhibitor Score" for particular inhibitors, as explained in detail later.

The formula for TSS reflects three important properties of transition state inhibition. First, an inactivating reactant that imparts good transition state character to the parent inhibitor (and structural analogs containing the same inactivating reactant) is characterized by a high R value for a linear regression performed on the plot of log(Ki) versus log (Km/kcat) for the parent inhibitor and its structural analogs (all containing the same inactivating reactant). A high R value implies that enzyme ligand interaction binding energies can be employed in a similar mechanistic way to drive inhibition as they are employed to drive catalysis. This means that an efficacious "complementarity" exists between the inhibitor and the transition state to which it is binding. Second, the linear regression performed for the plot of log(Ki) versus log (Km/kcat) yields a line with a slope M that is close to 1.0. This implies that a structural modification produces the same incremental binding energy change in an inhibitor that it does in the transition state. Consequently, transition state inhibitor data points that correspond to a line with a slope closer to 1.0 on a plot of log(Ki) versus log (Km/kcat) have higher transition state character. In the ultimate situation where slope = 1.0, the inhibitors may actually be binding to an enzyme conformation that corresponds to the rate determining step. Third, the line should have as many points on it as possible, in order to increase statistical confidence. All of these attributes are incorporated in the transition state score for a particular inactivating reactant X (TSSx) which is calculated by the formula:

$$TSS_X = R/ (ABS (1-M) * 1/P)$$

$$= (R * P) / (ABS (1-M))$$

where:

R= regression value
M= slope
P = number of points on the line
X = a selected inactivating reactant

The use of P is optional, since it is not critical to assessment of the inhibition mechanism.

In cases where M = 1 (the highly unlikely case where rate determining step inhibition has been identified), the value of "0.99" should be employed in replacement of "1", in order to prevent division by 0.

As an example of this transition state score, if 6 points were plotted, the best fit line using the majority of those points is the one that will be used. If desired, statistical programs can be employed to determine the best fit line. Those points that were used to compose the line represent transition state protease inactivators. Points that deviate substantially from the line to reduce the score are eliminated, and are deemed non-transition state inhibitors. For example, in Fig. 3 point "e" represented by Z-LLV-AFC/Z-LLV-X is off on the upper left of the flow-chart example, and is therefore not considered to be a transition state inhibitor. Hence, it is important to note that the transition state score is attributable to *particular inactivating reactant series.* In other words, the inactivating reactant "X" has been screened for its ability to bind to the transition state of the respective vehicle. It follows that all the inhibitors reflected on the plot of log(Ki) vs. log (Km/kcat) for X (provided they were not thrown out in the linear regression calculation) have the same transition state score TSSx.

For example, since Z-LLV-X, Z-LLL-X, Z-LLY-X are part of the "X" inactivating reactant series, these inhibitors all share the same transition state score. Likewise, if Z-LLV-Y, Z-LLL-Y, Z-LLY-Y are part of the "Y" inactivating reactant series, then all these inhibitors will share the same transition state score $TSS_Y$. Once this differentiation among two or more inactivating reactants has been conducted, parent inhibitors having the same peptide cores but different inactivating reactants can be compared. To continue with the example, if the "X" series has a higher transition state score than the "Y" series, then a comparison between the parent inhibitors Z-LLL-X and Z-LLL-Y will result in the conclusion that Z-LLL-X has higher transition state character than Z-LLL-Y. Note also that individual inhibitors *within each* inactivating reactant series (i.e., inhibitors with different peptide cores but the same inactivating reactant which, by definition, are characterized by the same TSS) can be compared and ranked if desired by determining individual inhibitor scores, as detailed below.

The investigator can adjust the stringency that he/she wishes to have in the transition state score. The higher the stringency of the TSS, the higher the confidence that is placed in the transition state binding ability of the inactivating reactant. Preferably, the TSS is at least about 22.5, more preferably at least about 97. For example, if it is decided that a minimum TSS of 100 is needed, the linear regression will be performed upon only that subset of points that yields a TSS of at least 100. It should be understood that the transition state score of a plot can be increased only by sacrificing points on the plot (e.g., eliminating outliers) to increase the regression value, R, also known as the correlation value. However, reducing P, the number of points, works against the effort by *reducing* the TSS, albeit by a lower amount. If too many points are thrown out, P can become so low that confidence in the linear regression disappears. At that point, it may be necessary to synthesize and test more inhibitors, or to lower the stringency. The number of points used to calculate the TSS is preferably at least 5, more preferably at least 8. The final TSS reflects a balance between a linear regression performed with too many points (the case where there is insufficient confidence in the transition state binding ability of the inhibitors) and a linear regression performed with too few points (the case where statistical confidence in the line is in question). A dozen or more data points are acceptable and may be preferred.

*5. Determination of transition state scores for next best inactivating reactants.*

As noted above, transition state plots are preferably subsequently constructed for the next best inactivating reactant series. To illustrate in our example, Z-LLL-Y was the second best ranked protease inactivator. Consequently, now, Z-LLL-Y serves as the parent, and deviations upon its theme are made similarly as described above. The result is that a transition state score is now assignable to the Z-LLL-Y parent and its modified series. In this manner, the transition state score of Z-LLL-Y can be compared to the transition state score of Z-LLL-X, which was previously ranked higher. If the transition state score of Z-LLL-Y is higher than the score of Z-LLL-X, Z-LLL-Y will be ranked higher than Z-LLL-X even though it was ranked lower in the list ranked by Ki.

Next, a transition state plot can be constructed for the third best protease inactivator. In our example, this would be Z-LLL-Z. It is then compared to the other inactivating reactants that were previously screened, and so on.

Many transition state plots can be constructed. *The goal here is to determine which inactivating reactants (e.g., X, Y, or Z) are inhibiting efficaciously with the noncatalytically bound peptide core (Z-LLL), at $TS_R$.* Eventually a large list of transition state scores is achieved. It is entirely possible that a protease inactivator variant will be found that has a higher transition state score than the parent that was being tested. A list of results is composed. Those inactivating reactants that have the highest scores are ranked highest.

*Construction of plots for Situation 1, next most preferred vehicles.* Depending upon time and resources, the above procedure can be performed on the next most preferred vehicles from Situation 1 (Fig. 3a). In the case of our illustration, the process would be conducted on Z-LLY-V.

*6. Determination of individual transition state inhibitor scores*

After the TSS has been calculated, an inhibitor score "I" can determined for any given inhibitor. The inhibitor score incorporates the characteristic of inhibitor "potency", as reflected in the Ki for any given inhibitor, to supplement the previously determined transition state score. Whereas a high transition state score implies high specificity and selectivity for the active site of the target protease, it does not imply as strongly the potency of the inhibition event, although it is generally the trend in the art that transition state inhibitors tend also to be highly potent. Two inhibitors may both have high transition state character, but they will not necessarily be equally potent. The higher the value of "I" for an inhibitor, the higher the inhibitor will be ranked. Note that the inhibitor score "I" can be calculated for a ground state inhibitor as well, as discussed in "step three" below.

The inhibitor score for a transition inhibitor ($I_{TS}$) can be calculated as follows:

$$I_{TS} = \log(TSS/(Ki))$$

.

The inhibitor score "I" can be used to differentiate between several types of inhibitors. For example, as alluded to above in the discussion of TSS calculation, the inhibitor score can differentiate among individual inhibitors that are part of the same inactivating reactant series and which consequently have the same TSS. Each of these, in turn, can also be considered as a potential TSPI candidate. For example, "I" can be used to differentiate inhibitor ability among Z-LLL-X, Z-LLY-X, and Z-LLV-X, as long as Ki is different for each of the inhibitors, whereas TSS cannot (since TSS is the same for each of these). In addition, "I" can be used to differentiate between inhibitors that have the same Ki value provided they have different transition state scores. In this case, the inhibitor with the higher "I" value will be the one that has the greater transition state character (as determined by TSS). For example, if Z-LLL-X and Z-LLL-Y each have a Ki = $1 \times 10^{-10}$, but Z-LLL-X has a higher TSS, then Z-LLL-X will produce a higher "I" value than Z-LLL-Y.

Importantly, "I" can also be used to differentiate between inhibitors with different Ki values *and* different TSS values. Clearly, when an inhibitor characterized by both high transition state character (a hallmark of a good protease inactivator) and a low Ki value (another hallmark of a good protease inactivator) is compared with another inhibitor (characterized by a lower transition state character and a higher Ki value), the formula for calculating "I" compels the result that the former inhibitor has a higher "I" value and is ranked ahead of the latter. Compare Z-LLL-X and Z-LLL-T in the table below for an example of this situation.

Example: kinetic parameters for various inhibitors

| Value | Z-LLL-X | Z-LLL-Y | Z-LLL-U | Z-LLL-W | Z-LLL-T |
|-------|---------|---------|---------|---------|---------|
| R | .95 | .90 | .99 | .99 | .90 |
| m | 0.98 | 0.89 | 0.99 | 0.99 | 0.89 |
| p | 10 | 5 | 10 | 5 | 10 |
| TSS | 475 | 40.9 | 990 | 990 | 40.9 |
| Ki | $10^{-10}$ | $10^{-10}$ | $10^{-9}$ | $10^{-8}$ | $10^{-6}$ |
| I | 12.6 | 11.6 | 11.9 | 10.9 | 7.6 |

On the other hand, an inhibitor may be characterized by a high TSS but also a high Ki value (evidencing looser binding), whereas another inhibitor is characterized by a lower Ki (tighter binding), but also a lower TSS. Calculation of the "I" value for each of these inhibitors allows them to be compared directly. Depending upon the relative magnitudes of the respective TSS and Ki values, "I" could be higher for one inhibitor or for the other. For example, Z-LLL-U in the table above has a very high transition state score (990), but a slightly higher Ki value ($10^{-9}$) than Z-LLL-Y (TSS=40.9; Ki=$10^{-10}$). In this case, the "I" value of Z-LLL-U (11.9) is higher than the "I" value of Z-LLL-Y (11.6), and therefore Z-LLL-U is ranked higher than Z-LLL-Y. It can be seen that the transition state character of the inhibitor predominates in this system *as long as the difference between the Ki values is not greater than a factor of about 10.*

Thus here when the difference between Ki values is greater than a factor of 10, the Ki value becomes more important as a measure of inhibitor ability than the transition state character. This case is illustrated by a comparison between Z-LLL-W (TSS=990; Ki = $10^{-8}$) and Z-LLL-Y (TSS=40.9; Ki=$10^{-10}$). Although Z-LLL-W has a significantly greater transition state score than the transition state score for Z-LLL-Y, its high Ki value (Ki = $10^{-8}$ for Z-LLL-W vs. Ki=$10^{-10}$ for Z-LLL-W) causes it to have a lower "I" value than Z-LLL-Y (1=10.9 for Z-LLL-W and 1=11.6 for Z-LLL-Y). Hence, Z-LLL-Y is ranked higher than Z-LLL-W. *In general, Ki begins to predominate over the transition state score when the Ki values between two inhibitors differ by a factor greater than 10.* It should be understood that the equation used to calculate "I" is not invariant, but can be adjusted by the investigator, as desired, to give more or less weight to Ki vis a vis TSS. For example, a coefficient could be applied to either the numerator (TSS) or the denominator (Ki) of the ratio TSS/Ki to alter the relative weights of the two variables. The goal of the inhibitor score is to serve as an effective quantitative differentiation instrument for comparing inhibitors that are marked by differences in their binding abilities and their transition state character.

*Final rankings.* The result of the prior procedures is that a final ranking of inhibitors is made. Preferably, inhibitors that have the highest "I" values are promoted to the next step. The justification for this is that the "I" value ranks inhibitors which have the most optimal combination of transition state character and potent binding. Alternatively, the investigator may rank inhibitors based solely upon the transition state scores, which do not incorporate the Ki value. This might be done when transition state character is the most desirable feature, for example in a particular drug design. Of course when inhibitors are ranked using TSS alone, individual inhibitors within an inactivating reactant series all have the same transition state scores and will therefore not be differentiable unless some other feature is assessed as well.

*Step two: Fine tuning of transition state protease inactivators*

This procedure can be undertaken in cases where economic resources and time are available. The top transition state protease inactivator is fine-tuned to obtain inhibition even closer to the rate determining step.

*1. Structural permutations of the inactivating reactant*

In this step, the peptide core of the TSPI is left unchanged, but permutations of the inactivating reactant portion of the are employed and the resultant inactivator compounds are analyzed for transition state score. For example, if Z-Leu-Leu-Leu-FMK is ranked as the highest TSPI, a variant of the inactivating reactant (FMK) group is used. If desired, a combinatorial library can be made based upon the inactivating reactant (in this case, FMK) and many permutations of the original FMK structure can be employed for transition state screening. Structural changes introduced by combinatorial chemistry can be fine or coarse. For example, in the case of the FMK, a potential change could involve substitution of the fluorine atom with a chlorine atom, or even a subtler alteration such as a change in the stereochemistry of the structure. The object is to find the structure that yields the highest transition state score. In principle, this inhibitor should be binding as close to the rate determining step of the target protease as possible. A new library of TSPIs is thereby obtained.

*2. Structural modification of the vehicle*

In this step, the same procedure as above is employed except that the vehicle (i.e., peptide core) portion of the transition state protease inactivator is refined. The inactivating reactant portion of the TSPI remains constant. Hence, a combinatorial approach is employed to refine the vehicles. This can be done in any number of ways. Modifications can include substituting a D-amino acid for an L-amino acid, substitution of other amino acids, and other structural changes. Again, the goal is to enhance the TSPI's transition state score.

*Step three: Identification and ranking of ground state protease inactivators (GSPI's)*

As noted above, some protease inactivators may be found to inhibit the protease in the ground state not the transition state. Inhibitors with a stronger correlation (as determined by R) between log (Ki) versus log(Km) in comparison to their correlation in log (Ki) versus log (Km/kcat) are promoted to this step. This procedure analyzes ground state protease inactivators (GSPI's) in the event that the low Ki protease inactivators identified in Step VII do not yield reasonable transition state inhibitors as determined by a good inhibitor score.

Every protease has a unique mechanism for catalysis. Historically, inhibitors with low Ki values were thought to be acting on the transition state of the protease because this is where Pauling's classic cooperative interactions are expected. However, as noted in the introduction, research has shown that a significant mechanism for catalysis by some proteases can also include ground state destabilization as a means for lowering the catalytic free energy of reaction. Sometimes this mechanism can be even more important than the lowering of energy in the transition state. Inhibitors with low Ki values may be acting by inhibiting a ground state in those proteases where ground state destabilization serves as the mode of action for catalysis.

Accordingly, if the low Ki protease inactivators identified in earlier steps do not appear to be transition state inhibitors, they can be assessed and differentiated, according to the present method, for their ground state inhibitory potential. The ground state score $GSS_X$ for a particular series of inhibitors containing inactivating reactant X is obtained in a fashion identical to the transition state score for a particular inactivating reactant series except that the it is taken from the plot of log (Ki) versus log(Km) instead of log(Ki) versus log(Km/kcat). The ground state score is therefore:

$$GSS_X = R/ (ABS (1-M) * 1/P)$$

where:

R = regression value
M = slope
P = number of points on the line
X = a selected inactivating reactant

Here P is optional for the same reasons it is optional for the $TSS_X$. A higher ground state score equates to a more potent inactivating reactant. The inhibitor score "I" for a ground state inhibitor (i.e., $I_{GS}$) is also calculated in a fashion analogous to the calculation for the transition state inhibition situation. Specifically, the ground state inhibitor score is:

$$I_{GS} = log(GSS/ABS(Ki))$$

The same arguments presented above for the inhibitor score as it related to the transition state score, also apply to the ground state score. Again, it is generally preferable to use the inhibitor score as the method of ranking ground state inhibitors.

*Application of inhibitor scoring for cleavable analogs*

The above formulas (TSS , GSS, $I_{TS}$, and $I_{GS}$) can also be applied to inactivators that are linked to highly cleavable substrates. The method of linking inactivators to highly cleavable substrates is already well known in the art. However, the use of the above scoring systems to identify and optimize inactivators that are interacting with transition state or ground state energies is novel.

*Step four: Fine tuning of ground state protease inactivators*

Structural modifications of the template protease inactivator are made as described above in step two, and the resulting compounds are tested for enhanced ground state score. First, the inactivating reactant is structurally modified while keeping the vehicle portion constant to deduce the modified inactivating reactant that yields the highest ground state score. Second, the vehicle portion of the protease inactivator is structurally varied to yield the highest ground state score. Again, this procedure is optional, and is conducted when time and resources are available.

*Step five: Characterization of final inhibitors*

The final inhibitor(s) are preferably characterized using various methods well-known to those of skill in the art. These include the time dependence of inactivation, the observation of saturation kinetics, substrate protection, tests of irreversibility, and measurement of the number of inhibitor sites attached to the enzyme. Pseudo-first-order rate constants (Kapp) can be assessed from the slope of time versus ln of percent remaining activity. Replots of 1/Kapp vs. 1/I will provide Ki and K2.

*Review of Theoretical Advantages of Step VII*

The advantage of this step is that a reactive moiety is identified, for addition to the vehicle, whereby that reactive moiety appears to react exclusively at $TS_R$. As discussed previously, since the binding and reaction centers are well distinguished by the vehicle, the reactive moiety is not expected to interfere with the favorable binding interactions that are present in the vehicle. Moreover, the efficacy of the $TS_R$ increase caused by the reactive moiety is expected to be substantially enhanced because the vehicle takes no part in lowering $TS_R$, since it is noncatalytic.

*Caveats*

Without intending to be bound by any particular theory, it is observed that several assumptions are made for the above calculations. As is already known in the art, experimental measures are taken to insure that these assumptions are valid. In cases in which they are not, feasible experimental methods that are published in the art are utilized to correct for assumptions that are violated.

*Assumption 1.* Km corresponds to the actual substrate dissociation constant, Ks. Hence, it is important that the association or dissociation steps do not play a rate limiting role for the chosen substrates. Km contains binding information but it is fundamentally a kinetic constant that is measured by definition under steady state, non-equilibrium conditions. Km will approximate the dissociation constant of substrate only when a slow step exists. Moreover, if the substrate is bound with high affinity and turnover is very fast, Km may not reflect the true dissociation constant, and steps other than the transition state intermediate production (e.g., tetrahedral intermediates in cysteine and serine proteases) may be partially or wholly rate limiting. In the case where the vehicle is so tightly bound, its inhibition constant may not be susceptible to calculation under steady state conditions. Also, the hydrolytic instability of the vehicle may also mean that the binding affinity cannot be determined under equilibrium conditions, since at vehicle concentrations that allow residual enzyme activity, the rate at which the inhibitor associates with the enzyme is slower than the rate at which it hydrolyzes.

*Assumption 2.* The enzyme is stable over the length of time necessary to reach equilibrium with the inhibitor.

*Assumption 3.* Introduced amino acid variations in the vehicle are independent of the energetics of the active site substrate reaction centers.

*Assumption 4.* Kcat/Km values reflect the same rate determining step throughout the series of substrates. Hence, the rate constant for the non-enzyme-catalyzed transformation of substrate to product does not change for the series and this same chemical step comprises the transition state for each of the substrates used in the correlation.

*Assumption 5.* Slow binding phenomena are to be taken into consideration when evaluating the kinetic results.

*Assumption 6.* If the vehicle binds too tightly, it may be difficult to determine the Ki.

*Assumption 7.* Laboratory problems. The same possibilities for error apply as those that were discussed in the caveats section of Step III.

Step VII for a crude protease mix proceeds as for a purified protease except that Vm is employed as a substitute for kcat, since the concentration of the protease is unknown. The appropriate transition state plot then is, Ki versus Km/Vm. Since no reference for this procedure is known in the art for a crude mix, the correlation may be more scattered than it would be for a purified protease. In this instance, a transition state score may need to be more liberal for a crude soup than it would be for a purified protease.

*Step VIII. Natural substrate tests*

If desired, the leading candidate TSPI's or GSPI's are analyzed for their ability to inhibit cleavage of natural substrates *in vitro,* in cell culture, and/*or in vivo.* This information helps to characterize the inhibitors, but is not meant to serve as a selection criterion for final ranking.

A substrate that is expected to be a natural cleavage target for a particular protease is selected. For example, if the protease is isolated from the lysosomes (e.g., a cysteine protease), then target substrate molecules may include those that are imported into the lysosome, such as albumin. If the protease is expected to degrade cell membranes, collagen may be chosen as a natural substrate. Cleavage can be analyzed using mass spectrometry or western blot analysis, for example. The potency of the inhibitor is determined by its ability to prevent cleavage of the substrate. A crude mix is treated identically to a purified protease.

*Step IX. Differential cleavage test to identify TSPIs or GSPIs which are selective for the target protease*

This step provides selectivity data for the highly ranked TSPIs and GSPI's identified thus far. As already mentioned, selectivity is an important feature of a protease inhibitor drug, since it is preferably capable of inhibiting the target protease of the pathogen but incapable of inhibiting general proteases of the human or nonhuman host. To evaluate selectivity, appropriate controls and assay setups are similar to those described previously in Step VI. Inhibitors are assessed for their ability to inhibit host proteases in the same mechanistic class as the target protease, as well as their ability to inhibit other host proteases. Ideally, the inhibitors are tested on a wide variety of host proteases. Preferred TSPIs or GSPIs inhibit the target protease but not the other proteases. Assessing inhibitor selectivity at this stage of the process is useful because these candidate TSPIs and GSPIs represent nearly "final" compounds. However, these compounds may be yet further modified, and these later modifications may alter selectivity. Hence, performance of this step, although important, remains optional, and it may or may not serve as a selection criterion for a re-ranking of the TSPI or GSPI list. At a minimum, this step serves to verify the degree of selectivity that the highly ranked TSPI or GSPI exhibits for the target protease. In other cases, this step may be utilized as a tool to re-rank the compounds on the TSPI or GSPI list. The crude mix is treated identically to a purified protease in this step.

*Step X. Further refinement of highly ranked TSPIs and GSPIs*

Lead protease inactivator compound(s) can be further refined, if desired, by testing additional structural variations of the compound. It should be understood that structural modifications of the TSPIs of any sort can be made, and the resulting compounds tested, at any stage of the process, without limitation. However, structural refinement is typically more economically done later in the process than earlier. Examples of further refinements include the following procedures.

*Adding one or more amino acids to the peptide core to interact with additional enzyme subsites.* In this method, one or more amino acids are added to the N-terminal and/or C-terminal positions (P or P' subsites) of the protease inactivator, and the activity of the resulting compound is compared to that of the parent compound to determine whether the new compound is a more potent (and/or more selective) inhibitor of the target protease. For example, extension of a tripeptide protease inactivator such as Leu-Leu-Leu-FMK may include the addition of one or more of the 20 naturally occurring amino acids at P4 of the parent compound, and, additionally, P5 if desired.

*Stereochemical modification.* The stereochemical compositions of P-P' subsite amino acids may be modified (e.g., changing a D- stereoisomer to an L-stereoisomer or vice versa). Chiral permutations of the inactivating reactant may also be considered, if possible.

*Step XI: Combinatorial library building on the lead compound scaffold*

Once a lead TSPI or GSPI has been identified, the final molecule can be used as a scaffold for a combinatorial library. Combinatorial library building is a standard procedure and is well referenced in the art. For example, we found that Z-LLL-FMK was a lead compound as a result of our experiments with the *Taenia solium* cysteine protease (Example 8). This compound can then be employed as the parent molecule for combinatorial permutations to enhance the inhibitory effect. Moreover, this procedure allows identification and optimization of the parts of the inhibitor that are characterized by biological structure/activity relationships such that the amino acids in the parent inhibitor are no longer part of the final molecule. This is an important consideration, since amino acids would be expected to be readily metabolized by the body, and it is therefore unlikely that they will ever become final drug compounds.

*Step XII: Crystallization of the target protease and computer modeling*

In cases where an inhibitor has been identified for the protease or for a crude mix, it may be desirable to crystallize the protease with its inhibitor and conduct further drug discovery refinements using computer modeling algorithms. This process of refinement is known in the art and includes, but is not limited to, utilization of three dimensional quantitative structure activity relationships (QSAR) including comparative molecular field analysis and two dimensional QSARs including molecular hologram QSAR and eigenvalue determinations to determine a pharmacophoric hypothesis of the lead TSPI or GSPI, and docking analysis of lead TSPI's with target structures including spatial, grid-based, soft potential energy functions and docking programs including Monte-Carlo, Ligand-Pile Up, and Probe algorithms.

*Step XIII: Addition of delivery molecules*

Once the lead compound has been finalized, delivery molecules can be added to the compound, including oil-water partition enhancers to candidate lead compounds. A delivery molecule is a substituent or independent associated molecule that enhances the permeability of a drug molecule through membranes, preferably without reducing the pharmacophoric effectiveness of the drug's other properties.

EXAMPLES

The present invention is illustrated by the following examples.

*EXAMPLE 1: Determination of optimal environmental conditions for cleavage by the purified Taenia solium cysteine protease and the Taenia solium crude mix ("Step P", the Preliminary Step)*

*Optimal Condition Determination for the Purified Taenia Cysteine Protease*

A cyst wall cysteine protease from *Taenia solium* was purified from the cyst wall. Details of that purification and characterization of cysteine protease activity are chronicled in the patent WO 00/63350 (published October 26, 2000). In this step, the exact pH and environmental conditions to allow for optimal active site catalysis for this cysteine protease was determined by the invention method to be pH 4.9 with the requirement for 10 mM cysteine. Optimal incubation temperature for activity was 37 °C.

*Optimal Condition Determination for "Crude mix" preparation.*

We employed a crude mix of dry-frozen *Taenia solium* cysts. Although we were already aware of the purified cysteine protease's optimal activity parameters, we wished to determine the optimal activity again from a new "mix", to ascertain what those conditions were, and to obtain confidence that they were similar. Because of the numerous proteases in this mix, the attention provided to obtaining the optimal operational catalytic environment for the targeted cysteine protease was especially critical. The more "fine tuned" the determined environment, the greater the possibility that existed for narrowing down the number of proteases which are active within that environmental region. Nevertheless, when the environmental conditions for optimal cleavage of a battery of substrates would be determined, the possibility still existed that isoforms of the protease or even more than one protease would exist optimally at the given conditions. However, even in the event that more than one protease would exist at an optimal level for a given environmental condition, it was still possible and likely that the biochemical mechanisms in the catalytic event followed some conserved pattern. This is based upon the fact that cleavage by more than one variety of protease would be optimal at the specific environmental condition only because of similar biochemical mechanisms within the active site. Indeed, significant conservation is already noted at the active site level for different classes of proteases.
However, without an in-depth analysis, it was impossible to assess whether or not two or more protease varieties in a specific environment had a completely conserved mechanism in common. Nevertheless, these procedures follow forthwith the assumption that there did exist a conservation of active site mechanism of more than one protease operating at a given environmental condition, and that an inhibitor that was specific for that active site, should abrogate the catalytic event for those proteases. Even if this assumption was not completely met, the procedures outlined below were hypothesized to produce an inhibitor which had some affinity for the active site of one or the other or all. As will be noted in later examples, the same inhibitor was produced for the purified protease as for the crude mix. Again, the previous discussion assumes that more than one protease will be active at a very specific environmental condition. However, the fact that there may have been only one active protease at this condition should not be neglected.
The procedures used to determine the optimal environment of the crude mix were as follows:

*1. Preparing the crude mix.*

The crude mix was prepared as follows. Dry frozen *Taenia solium* cysts were obtained from 6-12 month old cysticercotic pigs from villages in an endemic area of porcine cysticercosis in Mexico. Metacestodes were carefully dissected from the parasitized tissues, washed three times with PBS, and lyophilized. Parasite extracts were prepared by suspending 0.01 grams of dry frozen cysts in 10 ml of extraction buffer (0.4 M citrate, pH 4.9). The cysts were vortexed for three five-minute intervals with intermittent incubations (5 min) at 4 °C. Particulate material was removed by centrifugation (20,000 x g, 120 min) and the resulting soluble fraction filtered (0.2 uM).

*2. Determination of optimal pH.*

A broad pH vs. activity curve was constructed using a citrate-phosphate (CP) buffering system (Maniatis). In this assay, Z-Phe-Arg-AFC (Enzyme Systems Products, supplier) was used to screen for activity since this substrate is known to be a common substrate for cysteine proteases. Incubation of Z-Phe-Arg-AFC with a small amount of crude mix produced a bell-shaped pH versus activity curve with a pH optimum at 4.9, which was identical to the pH optimum of the purified protease.

*3. Optimization of crude mix activity.*

Once the pH optima and the general detection substrate were identified, the crude mix concentration dose curve was determined, with respect to substrate cleavage activity. The crude mix was added in increasing concentrations to Z-Phe-Arg-AFC at the determined pH optima. Following this, the activity of the protease at each concentration was followed over time. The concentration and a time at which activity registered was determined. Our results demonstrated detectable proteolytic activity after 5 hours of incubation with the substrate, with as little as 10 uL of starting material.

*4. Determination of exogenous variables and optimal incubation temperature.*

Proteolytic activity by the crude mix on Z-Phe-Arg-AFC was enhanced by the addition of 10mM L-cysteine to the assay buffer.
These results provided the optimal variables within which to incubate the crude mix in subsequent assays.

*EXAMPLE 2. Determination of High and Low Kcat substrates ("Step II")*

In this step, the most efficacious substrate for detection of the cysteine protease (high Kcat substrate) was determined in vitro along with those substrates which were not well cleaved (low kcat substrates). From this point forward, the following conditions refer to all assays: "assay buffer" refers to 0.4 M Citrate, pH 4.9 (the pH optima of the protease). Incubation time was 18 hours at 37°C. These conditions were determined by our preliminary data to be best for kinetic evaluation of the protease. All substrates were purchased fresh from Enzyme Systems Products (Livermore, CA), and used within 3 months of their purchase date to insure the most stable situation. The experiments were conducted in 6X50 mM Borosilicate mini-culture tubes (Fisher Scientific), and a fluorometer (Turner) was employed for detection of AFC cleavage.

*A. Screen of synthetic substrates*

Since the full 8,420 amino acid possibilities for cleavage were not available, representative substrates were employed. Therefore, this example can also be employed to demonstrate how this method can be adapted to situations where only subsets of substrates are employed for use with this step and for the subsequent steps. In order to choose this subset, representative amino acids (i.e., selected from the categories of acidic, basic, nonpolar, uncharged) were chosen so as to create as many categories of motifs as feasible for the P3-P1 positions with a set of 67 small peptides (see lists at Fig. 4a and Fig. 4b). The target cysteine protease from *Taenia solium* was isolated using the purification methods described previously in the patent WO 00/63350.
Approximately 1 ug (or a proteolytically detectable amount) of the purified target cysteine protease was incubated individually, with 1 ug of each of the available substrates in 500 uL of assay buffer. During this setup, the substrate was pre-incubated in the assay buffer for a short period of time (20 minutes) in order to reach an equilibrated status before the addition of the protease. Cleavage assessments were based upon the property of the protease to cleave synthetic peptide substrates linked at the C-terminus to 7-Amino-4-Trifluoromethyl Coumarin (AFC) as previously described. AFC linked tripeptides, dipeptides, and monopeptides, (with a variety of permutations at P3-P1) were available from Enzyme Systems Products (Livermore, CA), and were used as the source of the subset. Following incubation of enzyme preparations overnight (18 h, 37 °C) with peptide in assay buffer (Citrate, pH 4.9, supplemented with 0.01 M cysteine), free AFC concentrations were measured fluorometrically (excitation 405 nm, emission 505 nm). The proteolytic cleavage released the AFC group, which was measurable as a free molecule by a standard fluorometer and converted into molar quantities, based upon a previously established standard curve. The simplicity of AFC detection by the fluorometer was a key factor which expedited and simplified the procedures.

In the assay, 1 ug of the purified *Taenia* cysteine protease was added to a saturating amount of Z-Phe-Arg-AFC ($4 \times 10^{-5}$ M) in assay buffer. The experiment was conducted in triplicate and repeated at least 5 times until we were certain that variability in AFC cleavage was not statistically significant.

*Crude Mix:*

In order to prepare the *Taenia solium* "crude mix", dry frozen *Taenia solium* cysts were obtained from 6-12 month old cysticercotic pigs from villages in an endemic area of porcine cysticercosis in Mexico. Metacestodes were carefully dissected from the parasitized tissues, washed three times with PBS, and lyophilized. Parasite extracts were prepared by suspending 0.1 grams of dry frozen cysts in 10 ml of extraction buffer (0.4 M citrate, pH 4.9) at room temperature. The cysts were vortexed for three five-minute intervals with intermittent incubations (5 min) at 4 °C. Particulate material was removed by centrifugation (20,000 x g, 120 min) and the resulting soluble fraction filtered (0.2 uM). In assay buffers, the detectable amount of the crude mix, as described above, was employed and incubated with the target substrates.

*B. Assessment of kinetic parameters for optimal cleavage of the primary substrate*

Vmax for the cleavage of substrates by the purified target protease and Vmax for substrates cleaved by the crude mix protease were each calculated using conventional kinetic modeling calculations already described in the art.

*C. Identification of high and low Vmax (or Kcat) substrates*

Based upon the above results, those substrates with the highest and lowest values for Vmax were identified. We determined that the endopeptidase substrate, Z-Phe-Arg-AFC (Z= $C_6H_5$-$CH_2$-O-CO-, blocking group for the n-terminus) was the most optimally cleaved substrate by the purified cysteine protease, and was thus marked by the highest Vmax. This was followed by the cleavage of Z-FAR-AFC, Z-AAK-AFC, Z-AP-AFC, and Z-VLR-AFC, respectively in that order. These substrates would compose the "cocktail" to be employed in the next step.

We postulate that there are two possibilities for low Vmax substrates: 1) they are not turned over and not bound, or 2) they are bound noncatalytically, not turned over, and potentially locked in the active site. Those substrates obeying the latter condition were of most interest to this procedure, since they would be expected to be bound, but would possess no biochemical characteristics conducive to turnover. As discussed in the background, it is this characteristic that would confer significant advantages to the peptide core, as the objective for this peptide core is to have maximal favorable binding interactions (via lowering of $ES_B$ and $TS_B$), but not to lower $TS_R$ as a catalytically bound peptide core would.

Based upon our results, there were numerous noncleavable and potentially bound substrates of the *T. solium* cysteine protease, including Z-Leu-Leu-Leu-AFC (Z-LLL-AFC), Z-Leu-Leu-Tyr-AFC (Z-LLY-AFC), Mu-Leu-Tyr-AFC (M-LY-AFC), Boc-Val-Pro-Arg-AFC (B-VPR-AFC), Z-Leu-Gly-Arg-AFC (Z-LGR-AFC), etc. (Fig. 4a). Typically, with the full subset available, all of the noncleavable substrates would immediately be promoted to the next step. That action would end this part of the procedure.

However, there is also the scenario where all of the full 8,420 peptide combinations are not available, and therefore predictions about which substrates may also be noncleavable and bound are made by the interaction search. Therefore, we employed predictions about which amino acids would be potentially "locked" into each of the enzyme subsite positions, P1 through P3, and therefore predicted which of those amino acid combinations would be best to promote to the next step. A sample of this permutation search is now provided with the following steps and can be used by an investigator who does not have access to the full subset resources.

1. Amino acid motifs in turned-over substrates were first identified. Those substrates which were cleaved provided suggestions of amino acid motifs at specific subsite positions which were bound into the active site. This information is useful, although it is used in a cautionary manner. Since the amino acids are in high Kcat substrates, they may be involved in binding at $TS_B$ and $ES_B$. For example, as seen in Fig. 4a, D-VLR-AFC and Z-FR-AFC were both cleaved. In this instance, we made the prediction that Leu or Phe (which each have nonpolar substituent groups) at P2 can be bound and may thus be potentially inhibitory. We thus "fixed" Leu or Phe at P2 in our hypothesis. Moreover, substrates which were not cleaved were examined. It was notable that both Mu-LY-AFC and MeoSuc-FLF-AFC (MeOS-FLF-AFC) were not cleaved. Consequently, it was interesting that Leu was at P2. Since Leu was at P2 is in both a cleaved (e.g., D-VLR-AFC) and a noncleaved (e.g., Mu-LY-AFC) substrate, we could hypothesize that this amino acid may be bound into the active site, and potentially inhibitory (e.g., in a competitive assay with the most cleaved substrate, Z-FR-AFC). Hence, Leu was confirmed as fixed at P2 for the hypothesis.

2. An "interaction" search was performed to identify which amino acid worked well with L at P2. As can be seen, Y at P1 was a potentially cleavable substrate and may have thus been bound as well EY-AFC and Mu-LY-AFC. Moreover, V at P3 and R at P1 appeared to be important (e.g., D-VLR-AFC, Boc-VPR-AFC).

3. An "independent" noncleavage search was performed. Regardless of L at P2, the issue addressed is which motifs are noncleaved. For example, EY-AFC was not cleaved and thus E at P2 may have been a potentially important motif.

4. The motifs were summarized into a vehicle core hypothesis. Our hypothesis for amino acids at different positions in the vehicle core, for the *T. solium* cysteine protease example, was:

P1: R,Y
P2: L,F,E
P3: V

Again, it is emphasized that this type of an algorithmic search for predictive amino acids at each of the subsite positions would be performed only when the full subset of 8,420 amino acids is not available. This hypothesis will become useful after Step III as well.

*Results from the Crude Mix*

Steps similar to those for the purified protease were employed here. As will be noted, similar conclusions were noted on the identification of the protease inhibitors for the crude mix, and the identical hypothesis was derived (Fig. 4b). It is notable that the most cleaved substrates were Z-FR-AFC, Z-FAR-AFC, Z-AAK-AFC, AP-AFC, Z-VLR-AFC, respectively. This cleavage pattern, noted along with others, was highly similar to the cleavage pattern obtained for the purified protease. Based upon these results, it was concluded that proteolytic activity between the crude-mix and purified protease were highly comparable. Moreover, non-cleaved substrates were also highly similar, although there were subtle variations in relative cleavage for the non-cleaved substrates. However, this would be expected as fluorometric readings will tend to have subtle variation at lower readings due to background effects.

*EXAMPLE 3: Identification of inhibitory noncatalytic bound substrates ("Step III")*

There were two goals for this step. First, it was used to determine which of the low Vmax substrates from Step II also exhibited a low Km value. This experiment would differentiate between those low Vmax substrates, that exhibited low turnover due to lack of binding, and those, that had low Vmax's due to tight noncatalytic binding of the protease. The aim was to identify the latter of these. From a kinetic point of view, those enzyme/substrate pairings were identified which were marked by very tight binding (ES is low since $ES_B$ is lowered more than $ES_R$ is raised; $TS_B$ is lowered, but $TS_R$ is not) yet without reduced $TS_R$. Second, the step was employed to differentiate those substrates which were so tightly bound that they inhibited the cleavage of all high Vmax substrates. The outcome was a sequence of either a tripeptide, dipeptide, or single amino acid motif, where the peptide was capable of binding the active site in a kinetically more favorable fashion than the most commonly bound tripeptide, dipeptide, or single amino acid combination (those which would normally have a high Kcat/Km ratio).

*A. Choice of high and low Vmax substrates.*

The top 1.4% of substrates (out of 67) in the full panel of substrates with the highest Vmax values were chosen to compose a "TOP 1.4% cocktail". (We employed "Vmax" here although Kcat could also be used). Due to the lower number of substrates, a percentage was not chosen for the "BOTTOM %" substrates. Alternately, all substrates which were not cleaved as determined by a value of 10 uM/hr or less, were chosen to be tested in this step for competitive ability. Hereinafter the term "vehicle" will represent low Vmax substrates that were capable of inhibiting cleavage of the percent cocktails. In this instance, we tested for "vehicles" which inhibited cleavage of the TOP 1.4% cocktail (they were delivered into the active site). Based upon our results from Step II, the top 1.4% cocktail was composed of Z-Phe-Arg-AFC, the highest cleaved substrate.

We conducted several assays to set our stringency level for the TOP % substrates. We determined over the course of several experiments that we were able to obtain a differentiation in results of inhibition by supplementing the cocktail with 1 substrate. In other words, the increase of the percent cocktail was conducted through an increment increase by 1 substrate. This increment increase provided demonstration of the phenomenon of "diluting out" of inhibition.

*B. Analysis of the initial pool*

The experiment began with a competitor test between the noncleaved low Vmax substrates and the highest Vmax substrate, Z-Phe-Arg-AFC. Concentrations were varied between the low Vmax substrate and Z-Phe-Arg-AFC, as described above under "refinement of assay" until a relative inhibition profile was obtained. It was found that the optimal concentration to obtain a relative inhibition pattern was as follows: the high Vmax substrate needed to be at a concentration of $1 \times 10^{-6}$M in the final assay buffer (final volume = 500 uL), and the low Kcat substrate needed to be at a concentration of $8 \times 10^{-6}$M in the final assay buffer.

Hence, to set up the test control, Z-Phe-Arg-AFC was added first to assay buffer (0.2 M Citrate, pH 4.9 supplemented with 10 mM L-cysteine) at a concentration of $1 \times 10^{-6}$ M. Next, each of the noncleaved low Vmax substrates were added individually to a respective reaction tube at a final concentration of $8 \times 10^{-6}$M. The mixture was allowed to equilibrate for a period of 30 minutes. Finally, active purified enzyme was added to the tubes at a concentration that was always 50 times less than the concentration of the high and low Vmax substrates, respectively, in order to insure saturation conditions. This concentration varied from purification to purification and the amount added depended upon the yield and specific activity of the purified protease.

Positive controls were set up in a manner similar to that described for test controls, except that solvent was added in substitution for the low Vmax substrate (in a volume equal to the low Vmax volume addition). All of the substrates could be dissolved in dimethylsulfoxide (DMSO), and therefore, this was the solvent of choice. Negative controls were set up by adding the low Vmax and the high Vmax substrate in a tube without the protease. All potential non-proteolytic induced liberation of AFC was therefore controlled for in this tube (a feature which also monitored stability of the AFC substrates, since these substrates will freely liberate AFC when unstable). A tube set was established for this negative control.

All controls were set up in triplicate, and experiments were repeated a minimum of five times. The assays were incubated at 37°C, for 18 hours overnight.

Percent inhibition was calculated as:

$$\% \text{ Inhibition} = \frac{(\text{AFC positive for "Y"} - \text{AFC negative for "Y"}) - (\text{AFC test} - \text{AFC negative for "Y"})}{\text{AFC positive for "Y"} - \text{AFC negative for "Y"}} \times 100$$

Those low Vmax substrates that were inhibitory were now to be referred to as "vehicles" since they were indeed delivered into the active site of the target protease. The above concentrations for the high Vmax substrate cocktail and the vehicle, which produced the most differentiated inhibitor profile were now "fixed" for future use.

*C-E: Additional dose screenings of vehicles*

In this step, conditions were made to be more stringent to allow for better differentiation amongst the inhibitory potential of highly promising candidate vehicles. To do so, vehicles would be allowed to compete with an increasing medley of the most highly cleaved substrates, with each increase representing the addition of an extra substrate.

A 2.9% cocktail of the high Vmax substrates was made. This was composed of Z-FR-AFC and the second most cleaved substrate, Z-FAR-AFC. The vehicles promoted from the initial screenings above were again tested for competitive inhibition ability with the new cocktails. Their concentrations were set identically to those which were "fixed" in the previous protocols. In other words, $8 \times 10^{-6}$ M of the vehicle was added to the culture tube with $1 \times 10^{-6}$ M of Z-FR-AFC and $1 \times 10^{-6}$ M of Z-FAR-AFC. Active protease was added last at a concentration that was 50 times less than the concentration of the high Vmax substrate (to ensure saturating conditions).

In similar experimental fashion, a 4.4%, 5.9%, and 7.4% cocktail were tested with each of the promising vehicles. Each increase in the percentage represented an increase by an additional high Vmax substrate. Each of the individual components in the cocktail were added at $1 \times 10^{-6}$ M each, to $8 \times 10^{-6}$ M of the vehicle. The composition of the cocktails was as follows: 4.4% cocktail: Z-FR-AFC, Z-FAR-AFC, and Z-AAK-AFC (third highest cleaved substrate); 5.9% cocktail: Z-FR-AFC, Z-FAR-AFC, Z-AAK-AFC, and AP-AFC (fourth highest cleaved substrate); 7.4% cocktail: Z-FR-FC, Z-FAR-AFC, Z-AAK-AFC, AP-AFC, and Z-VLR-AFC. We stopped at this number of substrates in the cocktail, since confidence was obtained that inhibitory potential of vehicles could be "diluted out."

*F, G: Determination of Ki and Final Ranking*

Finally, a manageable set of candidate vehicles was determined. Ki determination was then conducted on each of the viable candidates (data not shown). This determination was conducted through a Dixon plot and was reinforced through conventional kinetic modeling methods, which are well known to one of skill in the art. In these studies, the vehicle was treated as an "inhibitor". The vehicle concentration was experimentally varied .5 to 5 times Ki in order to obtain a good value of Ki. All of the different substrates in the high Vmax cocktail were used to determine Ki, whereby those aggregate substrate concentrations were maintained at a saturating level (50 to 100 times Km). A convenient aspect to this calculation was that the Ki values determined for these vehicles were also equivalent to the vehicle's Km value when it is used as a substrate.

The step concluded with a final ranking of candidate inhibitor vehicles. The lower the Ki value, the higher the ranking which was attributed to the vehicle. Ki determination was the sole basis of the final ranking.

*Results and Discussion*

Through the experimental design in this step, we were able to derive two major conclusions. First, we determined which of the low Vmax substrates caused inhibition of the purified cysteine protease. These low Vmax substrates were termed "vehicles." Second, the experiment was able to differentiate inhibitory potential amongst the vehicles, and this culminated into a final ranking based upon Ki values.

The hypothesis that increasing the number of substrates in the competitor pool would increase the stringency of the competition between the vehicles and the high Vmax substrates, and therefore "dilute" out the inhibitor potential of less inhibitory vehicles, was a successful one. To illustrate, Z-FR-AFC was employed as the high Vmax substrate in the TOP 1.4% cocktail, and tested as a competitor against all noncleaved low Vmax substrates. Competition yielded the following sample relative inhibitory profile of the following vehicles possessing greatest inhibition ability to lowest inhibition ability as shown in Fig. 5a, respectively: Z-LLL-AFC, Z-LLY-AFC, Mu-LY-AFC, Boc-VPR-AFC, Z-RR-AFC, Z-SY-AFC, B-LGR-AFC, Z-FLF-AFC, m-PK-AFC, L-W-AFC, etc. These substrates were now deemed "vehicles" due to our observation that they were being "delivered" into the active site. We assessed the success of a vehicle through three parameters: potency of inhibition, maintenance of inhibition, and robustness of inhibition.

The first two of these parameters would be employed to provide a vehicle inhibition quotient (V).

The first parameter of vehicle potency was defined as the percent inhibition in the presence of the TOP % cocktail. In other words, the higher the percentage of inhibition in the presence of the 1.4% cocktail, the more potent we deemed the inhibition to be. To analyze these vehicles, we found three tiers of inhibition. As mentioned, they were defined by their inhibition in the presence of the 1.4% cocktail. These tiers should be broken out just as a "bell curve" may be subdivided into tiers. The first tier was composed of those vehicles that produced inhibition greater than 50% when in the presence of the 1.4% cocktail. The second tier was composed of those vehicles that produced inhibition between 20-50% inhibition when in the presence of the 1.4% cocktail. The third tier was composed of those vehicles that produced inhibition less than 20% when in the presence of the 1.4% cocktail. Typically, only the vehicles in the top tier would be promoted to the next step for analysis by increasing the cocktail %, as discussed in above. However, in order to study the effects of inhibition, all of the vehicles were promoted to the next cocktail step.

To study "maintenance" of inhibition, the vehicles were subjected to competition by an increase in the composition of the cocktail. "Maintenance of inhibition" was determined by the inhibition potential of vehicles in the presence of the final % cocktail (7.4%). Thus, the next cocktail additions were increased to 2.9%, 4.4%, 5.9%, and 7.4% (2.9%: Z-FR-AFC + Z-FAR-AFC; 4.4%: Z-FR-AFC + Z-FAR-AFC+Z-AAK-AFC; 5.9%: Z-FR-AFC+ Z-FAR-AFC+ Z-AAK-AFC + AP-AFC; 7.4%: Z-FR-AFC + Z-FAR-AFC + Z-AAK-AFC + AP-AFC+ Z-VLR-AFC). Hence, the purpose of this test was to determine which vehicles maintained respectable inhibition, and which ones lost inhibitory ability by being "diluted out." In order to maintain respectable inhibition, we defined 50% as the cutoff parameter for top tier inhibition. Thus, those vehicles that were marked by inhibition of 50% or greater in the presence of the 7.9% cocktail were considered to have "maintained" inhibition. As is seen in Fig. 4a, the inhibition potential of each of Z-LLL-AFC, Z-LLY-AFC and mu-LY-AFC was maintained with the final cocktail (7.4%) producing inhibition of 50% or greater. Conversely, the inhibitory potential of the second tier of inhibitory vehicles, Boc-VPR-AFC, Z-SY-AFC, Z-RR-AFC, Z-FLF-AFC and Z-LGR-AFC became diluted with the final cocktail (7.4%) producing inhibition that was less than 20%. The third tier of vehicles included all the remaining vehicles: they produced very minimal inhibition and were thus not maintained.

From this data, we concluded that tightly bound vehicles would most likely "maintain" their inhibition potential. This would be expected for a vehicle which is truly bound in a tight manner. Although we did not observe such a case, a vehicle that produced a potent inhibition yet that did not "maintain" it with the final % cocktail, would be eliminated. For example, if Z-LLL-AFC produced a potent inhibition of 90% with the 1.4% cocktail, but produced 20% inhibition with the final 7.4 % cocktail, it would be eliminated because it could not maintain inhibition. Conversely, if Mu-LY-AFC produced a potency of inhibition of 80% but maintained it at 60% for the 7.4% cocktail, it would be ranked higher than Z-LLL-AFC.

Thus, maintenance of inhibition was considered to be a critical parameter. However, the parameter for elimination would be based upon the vehicle inhibition quotient, which is discussed below.

Additionally, "robustness" of inhibition was noted. We defined robustness of inhibition as those vehicles which lost only a small percent of inhibition between the 1.4% and the 7.9% cocktail. It was notable that the percentage drop in inhibition for the first tier vehicles was typically less than the percentage drop of inhibition in the second and third tier vehicles. For example, Z-LLL-AFC was a significantly more robust vehicle than B-VPR-AFC. As shown in Fig. 5, the percentage drop in inhibition between the 1.4% and the 7.9% cocktail for Z-LLL-AFC was only 25%, whereas the same drop in B-VPR-AFC was 38%. However, "robustness" was noted here as more of a qualitative measure of the assay, but does not provide the best mode of determination of the vehicle inhibitor potential.

Alternatively, the parameters of potency and maintenance provided a straightforward vehicle inhibition quotient, where V= P * M. P was equal to percentage inhibition in the presence of the top cocktail, and M was equal to percentage inhibition in the presence of the last cocktail employed. The higher the V value, the greater the inhibition. Based upon these results, we calculated the following V values (Table 1).

Table 1. Vehicles and V value rankings.

| Vehicle | Tier | V Value |
| --- | --- | --- |
| Z-LLL-AFC | 1 | 0.585 |
| Z-LLY-AFC | | 0.550 |
| Mu-LY-AFC | | 0.450 |
| B-VPR-AC | 2 | 0.120 |
| Z-FLF-AFC | | 0.100 |
| Z-RR-AFC | | 0.090 |
| SY-AFC | | 0.090 |
| B-LGR-AFC | 3 | 0.07 |

We demonstrated that the stringency of the competition between the cocktail and vehicle increased, when the percentage of the cocktail was increased. In other words, an increased "dosage" of the cocktail enabled selection of those substrates that had the highest inhibitory potential while "diluting out" the inhibitory potential of the less impressive substrates. These results were consistent from experiment to experiment.

Finally, Ki was determined for all of the promoted top inhibitory vehicles, as determined by their I values. The vehicles were then ranked based upon the Ki value. The highest ranked vehicles based upon this scheme were Z-LLL-AFC, Z-LLY-AFC, and Mu-LY-AFC. We have identified several advantages to this experiment in determining inhibitory potential of vehicles. First, this method was expeditious and served as an efficacious "filtering" mechanism to identify a key pool of inhibitory *noncatalytic* substrates that had greater activity for the cysteine protease's active site than the most highly cleaved substrates identified from the original list of 67 substrates. Second, increasing the percentage of cocktail allowed for ranking of vehicle inhibition capability without actually incurring the experimental burden of determining Ki. Alternatively, a straightforward V quotient was calculated from the data in the bar graph. Ki values were then determined for those vehicles with the highest V value in the top tier. Ki was not actually calculated until the very end of the procedure.

There were two major conclusions to take from these experiments: one theoretical and the other practical. First, we believe Z-LLL-AFC, Z-LLY-AFC, and Mu-LY-AFC were marked by a tight binding to the protease's active site cleft, such that it lowered $ES_B$ By virtue of lowering $ES_B$, we believe that this bound substrate would provide binding interactions which may be conducive to the lowering of $TS_B$, based upon the hypothesis that binding interactions at $ES_B$ are conserved at $TS_B$ (Menger, 1992). This was advantageous for the sake of identifying an amino acid combination with bound properties. Moreover, since the peptide core was not catalytic, TS was not lowered enough to cause turnover (since $TS_R$ is not lowered), as the peptide core was noncatalytic. Hence, this peptide core would not only be expected to aid the final inhibitor molecule by virtue of its strong binding properties, but was also predicted to be not counteractive to the final inhibitor molecule as it would not theoretically lower $TS_R$. The experiment demonstrated that a noncatalytically bound vehicle was identified for the cysteine protease, in a manner where binding could be determined in an efficacious manner, without identification of individual components that were activating to catalysis.

The second major conclusion from this experiment was that we were able to formulate a top vehicle "hypothesis" which could be used in the subsequent inhibitor testing step. This hypothesis would form a template for a subsequent combinatorial series of inactivating reactant additions (Step VII). Typically, a full set of 8,420 vehicles would be available and the top inhibitor(s) with the lowest Ki values would subsequently be promoted to the next step. However, since we did not have the full subset of inhibitors, we were not completely confident that the top inhibitors from this screening, Z-LLL-AFC, Z-LLY-AFC, and Mu-LY-AFC would be the top vehicles amongst the entire 8,420 possible amino acid combinations for tripeptides, dipeptides, and monopeptides, had they been available.

Because of this we wished to use this information to make useful predictions about other possibilities for other preferable substrate amino acid sites on the motif of Z-LLL-AFC for inhibition. In other words, we wished to explore other hypotheses about amino acids at P1 through P3, and to develop a new vehicle core hypothesis. Typically, this information can be employed to design new vehicles to test, and so on. Again, this situation is for the case when the full subset of 8,420 amino acids is not available. The following excerpt demonstrates how a design prediction may be performed with this example.

To begin prediction, we first decided to re-examine the core "vehicle" hypothesis from step II.

We return and now critique the success of that core hypothesis:

P1: R,Y
P2: L,F,Q
P3: V

Several of the conclusions from that hypothesis were correct. The following discussion demonstrates the concept that potentially much information about vehicle inhibition can be gathered by Step II, which is therefore useful for prediction. For example, L and V at P3 did demonstrate inhibitory potential (e.g., Boc-VPR-AFC , Z-LLL-AFC, and Z-LLY-AFC) as was predicted. Moreover, L at P2 was certainly beneficial as demonstrated by vehicle inhibition via Z-LLY-AFC, Z-LLL-AFC, and Mu-LY-AFC. Finally, both R at P1 demonstrated inhibitory potential (e.g., Z-VPR-AFC, Z-RR-AFC) as did Y at P1 (e.g., Mu-LY-AFC, Z-EY-AFC). However, because several of the most cleaved substrates used in the cocktail also possessed an R at P1 (e.g., Z-FR-AFC, Z-VLR-AFC), we postulated that R at P1 may be activating, and may thereby potentially contribute to the lowering of $TS_R$. As a result, we concluded that R at P1 was an unfavorable situation under the Step II hypothesis. Despite this latter conclusion, these results demonstrated that the core vehicle hypothesis from Step II was capable of providing successful predictions for inhibition, to be tested in Step III.

Based upon the inhibitor results from this step using the competitor assay, we derived a new vehicle hypothesis. The top vehicles take precedence. Those vehicles were: Z-LLL-AFC, Z-LLY-AFC, and Mu-LY-AFC. Therefore, the top candidate vehicle was Z-LLL-AFC and the second top vehicle was Z-LLY-AFC. It is important to note that although inhibition by these three vehicles were always higher than in the rest of the panel of vehicles, it remained very close from experiment to experiment. A sample experiment is shown below for diagnostic purposes. However, based upon these results, the new vehicle hypothesis was:

Primary hypothesis: L at P3, L at P2, and L or Y at P1
Secondary hypothesis: F or V at P3 or F at P1

This hypothesis was reinforced by the following observation. Whenever L or F was at P3 for any of the inhibitors, notable inhibition (greater than 20%) was noted for all 1.4% cocktails. (e.g., Z-FLF-AFC, Z-LLL-AFC, Z-LLY-AFC, Z-LRR-AFC, Z-LGR-AFC). Moreover, L at P2 was reinforced by the observation that it was predicted in the Step II core vehicle hypothesis as well as being part of the top inhibitory vehicle. L at P2 produced notable inhibition (greater than 20%) with the 1.4% cocktail, in substrates with L at P2 including (Z-FLF-AFC, Z-LLL-AFC, Z-LLY-AFC). L at P1 was not reinforced, but is part of the hypothesis due to its inhibition ability in this step via Z-LLL-AFC. Y at P1 was reinforced by this step (e.g., Z-EY-AFC and Mu-LY-AFC) as well as by its prediction in Step I as a potential candidate. Moreover, as a secondary hypothesis we posited that V at P3 (e.g., based upon inhibition by B-VPR-AFC) and F at P3 (e.g., based upon inhibition by Z-FLF-AFC) may be beneficial.

As for vehicles with low inhibition capabilities, it is notable that there was less predictability in the "dilution" effect. In other words, as inhibition capability was lowered, it was more difficult to obtain a consistent dilution effect. It is possible that as inhibitor binding affinity by these noncatalytic peptide cores became diminished, the kinetics were less predictable.

In conclusion, the above example illustrates how a prediction algorithm may be derived when resources are limited. New vehicles may be tested after the first screening of vehicles. The final result is a vehicle hypothesis when the full subset of 8,420 amino acids are not available.

We drew five conclusions from this experiment.

1. Inhibition by top vehicles tended to be potent, maintained, and robust.
2. Inhibition by lesser vehicles was not potent, maintained, and robust.
3. Inhibition by this competitor assay could be used to predict vehicles which would have a low Ki value.
4. Inhibition by lesser vehicles was less predictable and consistent, whereas inhibition by top vehicles was more predictable and more consistent.
5. A prediction algorithm could be employed in order to determine new vehicles which were to be "fed" back into this step. This prediction algorithm would be based upon the vehicle core hypothesis derived from Step II, as well as upon conclusions about binding and inhibition derived from this step.

We did not purchase additional vehicles based upon the prediction algorithm. Instead, based upon our first screening, we immediately promoted the primary and secondary hypotheses outlined above to the next step. The primary hypothesis was based upon inhibition by Z-LLL-AFC, Z-LLY-AFC, and Mu-LY-AFC. The low Ki value of these vehicles (data not shown) also reinforced their inhibitory efficacy.

*Crude-mix.*

One of the most intriguing findings in this experiment was that a highly similar inhibition pattern for the competitor assay resulted when the crude mix was employed as the source of the target protease. Although there were some variations in the effects of inhibitor dilution, the most highly ranked vehicles remained inhibitory consistently in comparison to the profile with the target protease. The relative profile and composition of the top tier of vehicles was identical. Z-LLL-AFC, Z-LLY-AFC, Mu-LY-AFC, Boc-VPR-AFC, Z-SY-AFC, Z-RR-AFC, Z-FLF-AFC, and Z-LGR-AFC consistently emerged as the top eight vehicles (Fig. 5b), respectively, based upon their V values (Table 2). From these results Z-LLL-AFC, Z-LLY-AFC, and Mu-LY-AFC were again determined to provide the most potent, maintained, and robust inhibition. It was notable that the three vehicles had very similar V values. Interestingly, in the sample experiment shown in Fig. 5b, Mu-LY-AFC yielded the highest V value. As was mentioned, this vehicle along with Z-LLL-AFC and Z-LLY-AFC, were consistently the most inhibitory vehicles. The V values of the most inhibitory vehicle series are shown below in Table 2. Again, a clear break between the first and second tier vehicles is discernible for the V value.

Table 2. Vehicles and V value rankings.

| Vehicle | Tier | V Value |
|---|---|---|
| Z-LLL-AFC | 1 | 0.611 |
| Z-LLY-AFC | | 0.55 |
| Mu-LY-AFC | | 0.45 |
| B-VPR-AFC | 2 | 0.12 |
| Z-FLF-AFC | | 0.10 |
| SY-AFC | | 0.09 |
| Z-RR-AFC | | 0.09 |
| B-LGR-AFC | 3 | 0.02 |

As mentioned, less predictability in vehicles with a lower binding ability may be expected. Similarly, a small variation in inhibition profile was noted for the third tier of vehicles. Again, this is not surprising given that we would not expect ideal binding by these compounds, and therefore, the binding interactions are perhaps not as predictable.
Our results demonstrated that a highly similar profile for the top vehicles was obtained from both the purified protease and the crude parasite mix from which it was purified. These results demonstrated that proteolytic behavior of the purified cysteine protease and the crude mix, in an assay buffer of pH 4.9 supplemented with 10 mM exogenous L-cysteine, demonstrated a similar active site behavior. Based upon these results, we were confident that similar mechanisms of catalysis were being employed by both the crude mix and the purified protease under these environmental conditions of cleavage. Moreover, we were more confident in the hypothesis: L or Y at P1, L at P2, and L at P3 confer tight binding interactions with the active site in a manner that was potent, maintained, and robust. It is important to note that if a marked difference had been noted between the top vehicles' inhibition profiles of the purified protease and the crude mix, then the same proteolytic activity may not be operating at the given environmental condition.

*EXAMPLE 4. Natural Inhibition Tests ("Step IV")*

Here, the leading candidate vehicles were tested for their capabilities to inhibit cleavage of human IgG in comparison to the inhibitors of differing mechanistic classes, which we have shown in the WO 00/63350 patent disclosure to be a natural substrate for the protease. The basis for identification of this natural substrate was our postulation that the purified cysteine proteinase may degrade IgG in lysosomal vacuoles where the pH and reducing environment is similar to that used in our IgG degradation assays. Threadgold and colleagues have identified acidic vacuoles in the cyst wall of *T. crassiceps* (Threadgold et al., 1983). In a previous study, electron microscopy was used to localize electron dense cleavage products of Z-Phe-Arg-methoxynaphthylamide in the *T. crassiceps* lysosomal vacuoles of the tegumentary cytons and internuncial processes, which have also been linked to adsorptive endocytosis (Khalil et al., 1998). Since the hydrolysis of Z-Phe-Arg was reversed by E-64, they identified the responsible enzyme as a cysteine proteinase.

Coupled with the observation that altered host IgG has been found in the cyst fluid of *Taenia* cysts (Hayunga et al., 1989), we believe that human IgG may be a natural substrate for the cysteine proteinase in acidic cyst wall vacuoles. We hypothesized that this interaction may be highly specific and catalytically efficient if the kinetics of IgG hydrolysis were in fact similar to that of Z-Phe-Arg cleavage.

To conduct this assessment, a western blot was performed. IgG digestion was detected by incubating 20 ul of *T. solium* purified enzyme or *T. sodium* extract with 1 ug of human IgG (Sigma, St. Louis) in a final volume of 100 ul of assay buffer. Exogenous thiol activation was assessed by incubating the assay in the presence or absence of cysteine. The catalytic class of the IgG protease was determined by pre-incubating the following proteinase inhibitors at their active concentrations, with the enzyme: E 64 ($10^{-6}$ M , cysteine proteinases), phenylmethylsulfonyl fluoride (PMSF) ($10^{-3}$ M, serine proteinases), pepstatin A ($10^{-6}$M, aspartic proteinases), and 1,10 phenanthroline ($10^{-3}$ M, metalloproteinases), and the vehicle, Mu-Leu-Tyr-AFC ($10^{-3}$ M). After addition of IgG, the assay was incubated overnight (37°C, 18 hrs). Subsequently, 100 ul of the mixture was boiled in sodium dodecyl sulfate polyacrylamide gel electrophoresis (SDS-PAGE) sample buffer with 2% 2-mercaptoethanol, separated by 12.5% SDS-PAGE, and blotted onto a polyvinylidene difluoride membrane. Tris buffered saline containing 0.1 % bovine serum albumin and 0.05% Tween 20 (TBS-BSA) was used to block nonspecific binding. The blot was washed three times with TBS containing 0.05% Tween (TBS-Tween), and incubated with biotinylated antihuman heavy and light chain antibody diluted 1:1000 in TBS-BSA (Boehringer-Mannheim, Indianapolis, IN) followed by incubation with a peroxidase conjugated strepavidin-avidin complex. The blot was then subjected to chemiluminescence (ECL kit, Amersham International, Sweden) and autoradiography.

*Results and Discussion*

After human IgG was incubated with the purified cysteine proteinase, we observed degradation of both the heavy and light chains. Inhibition was noted with the general cysteine protease inhibitor, E64. Typically, IgG degradation would also be tested by inhibition via the top vehicle, Z-LLL-AFC. For this example the third most inhibitory vehicle, Mu-Leu-Tyr-AFC with a Suc (succinyl) derivation as opposed to Leu, was tested instead. Degradation of the heavy chain is shown in Fig. 6. Identical results were noted within the crude mix (data not shown), although the concentration of Suc-Leu-Tyr-AFC required for inhibition of IgG cleavage was greater in the crude mix. This may be expected since the inhibition may not be as precise with the presence of other proteases in the mixture.

*EXAMPLE 5: Synthesis and collection of vehicle/inactivating inhibitor combinations which form a potentially "potent" Transition State or Ground State Protease Inactivator Vehicle ("Step VII").*

Up until this point, the top vehicle hypothesis (i.e., preferred peptide residue) from Step III was identified as L or Y. A "hypothesis" rather than a top vehicle was promoted to this step, since we did not have access to the full subset of 8,420 amino acids. Based upon our hypothesis, we believed that this vehicle core represented high binding interactions that were conserved at both $ES_B$ and $TS_B$, without a concomitant and inhibitor-"unfriendly" reduction of $TS_R$. Hence, the goal in this phase was to shift attention from the binding of the peptide core (already characteristic of the vehicle) to a search for an inactivating reactant which would now increase reactive energy at $TS_R$ and not at the ground state. Hence, the binding portion of the vehicle would remain constant, presumably in both the ground state and the transition state structure, while the inactivating reactant was varied. Essentially, the next few steps screened for an inactivating reactant that inhibited the target protease at the transition state.

*A. Covalent linkage of inactivating reactant to vehicle*

Inactivating reactants were organically linked to the C-terminus of the P1 residue of the top vehicle hypothesis in order to construct a protease inactivator. The hypothesis favored both Z-LLL and Z-LLY. The inactivating reactants chosen for this purpose were fluoromethylketone (FMK), vinylsulfone (VS), and epoxide. The fluoromethylketone linking to Z-LLL was conducted at Enzyme Systems Products (Livermore, CA) and the Z-LLL-vinyl sulfones and Z-LLL-epoxides were generously supplied by Dr. James Powers (GA Tech). For the second top vehicle hypothesis, Z-LLY, fluoromethylketone and diazomethylketone links were synthesized by Enzyme Systems Products. The linking of the inactivating reactant with the vehicle follows the commonly implemented procedures of amino acid linking which are well known in the art of organic synthesis. Other variations on the Z-LLL motif as well as the secondary hypotheses (F at P3 and F at P1, V at P3 was unavailable) were generously supplied by Dr. James Powers (GA Tech) and are included in Fig. 7a.

The synthesized products were provided at very high purity. The inactivating reactant functionalities were chosen based upon their potential to form a tetrahedral adduct with the active site of the cysteine protease. For example, in cases where tetrahedral adducts are important in the rate limiting step (e.g., cysteine and serine proteases), inactivating reactants that would have the potential via a nucleophilic or electrophilic attack (depending upon the molecule) to mimic the tetrahedral geometry of the rate limiting step, were preferred.

There was of course no theoretical limit as to the number of inactivating reactants which could be linked to the top vehicle, and it would have been entirely dependent upon the resources available to the investigator. However, the greater the number of inactivating reactant links to the top vehicle for the formation of several protease inactivator, the greater were the possibilities for successful development of a transition state protease inactivator (TSPI). Given that our resources were limited, a significant preference was placed upon the development of a larger library of inactivating reactants for *the* top vehicle rather than a library of several vehicles with less inactivating reactants.

*B. Inhibition assays for determination of candidate transition state protease inactivators*

Once the protease inactivators were synthesized in the organic laboratory, they were to be tested for the lowest efficacious concentrations at which they induced 100% inhibition of the target protease ($EC_{100}$). The top percent cocktail that was employed in the final vehicle selection step of Step III was employed as the source of competition for the inhibitors. In this case, this was the 7.9% cocktail that was comprised of Z-FR-AFC, Z-FAR-AFC, Z-AP-AFC, Z-AAK-AFC, and Z-VLR-AFC. The final result would be a ranking of transition state candidate protease inactivators with a high ranking directly correlating to a low $EC_{100}$ value. The following steps describe the assay:

*Step A: Initial Screening.* To set up the test control, each protease inactivator was first incubated with the 5% cocktail (which provided the final differentiation of vehicles in Step III). To begin with, the protease inactivator was incubated at a concentration of $3 \times 10^{-9}$M in 500 ul of assay buffer (0.2 M Citrate, pH 4.9, supplemented with 10mM L-cysteine). This concentration was determined by a series of inhibitor dilutions to be the best inhibitor concentration at which a differentiation in inhibitor potential could be noted. Next, a concentration equal to $4 \times 10^{-5}$M for each of the individual cocktail components, was added to the mix. The components were allowed to equilibrate for a period of 30 minutes. Finally, active purified enzyme was added to the tubes at a concentration which was always at least 50 times less than the concentration of the lowest concentration in the mix (so as to provide saturating conditions.) This concentration varied from purification to purification, and the amount that was added depended upon the yield and specific activity of the purified protease.
Positive controls were set up similarly as described for test controls, except that solvent was added in substitution for the protease inactivator (in a volume equal to the low Kcat volume addition). All of the protease inactivators could be dissolved in dimethylsulfoxide (DMSO), and therefore, this was the solvent of choice. Negative controls were set up by adding the protease inactivator and the cocktail substrates in a tube, without the protease. All potential non-proteolytic induced liberation of AFC was therefore controlled for in this tube.
All controls were set up in triplicate, and experiments repeated a minimum of five times. The assays were incubated at 37°C, overnight. Percent inhibition was calculated as:

$$\% \text{ Inhibition} = \frac{(\text{AFC positive for "SI"} - \text{AFC negative for "SI"}) - (\text{AFC test} - \text{AFC negative for "SI"})}{\text{AFC positive for "SI"} - \text{AFC negative for "SI"}} \times 100$$

*Step B: Inhibitor Dilution Studies and formation of a ranked list of protease inactivators based upon $EC_{100}$ values.* Although it was possible that a ranking of inhibitors may have been established just through the initial screening, it was found that a significant number of the protease inactivators inhibited the target protease by 100% (Fig. 7a). Because of this, a dilution study was required in order to determine a viable ranking. Thus, the concentration of each of the protease inactivators was lowered with a dose response setup until the most efficacious concentration for 100% inhibition ($EC_{100}$) could be determined. The inhibitors had to be diluted to nanomolar levels in order to establish a differentiable $EC_{100}$ pattern. The final result of the assay was a ranked candidate list of protease inactivators. A lower $EC_{100}$ was attributable to a higher ranking and vice-versa.
*Step C: Determination of Inhibitor Ki and mode of inhibition, and re-ranking of $EC_{100}$ list with inhibitor Ki.* Ki of each of the top ranked protease inactivators, based upon low $EC_{100}$ values, was assessed next for the top 5 $EC_{100}$ inhibitors.

In order to determine Ki, all of the high Vmax cocktail substrates were employed for Ki determination (since Ki of the inhibitor for the enzyme is the same regardless of the substrate). To insure the highest accuracy, Ki was to be determined upon a minimum of at least three of the high Kcat substrates. Nonlinear fitting and the Dixon plot were both employed to determine Ki of the top ranked protease inactivators. A further plot was constructed using Eadie Hofstee and or Hanes to determine the mode of inhibition.
In this experiment, we identified several protease inactivators that inhibited the target cysteine protease. The top inhibitors were determined to be Z-LLL-FMK, Z-LLY-FMK, Z-LLL-Epoxide, and Z-LLL-VS. Next, Ki values for the top five inhibitors were assessed and determined (data not shown).

Based upon these results, Z-LLL-FMK and Z-LLY-FMK had the lowest Ki values, respectively. The remarkably low Ki value of these inhibitors was suggestive of a mode of inhibition occurring at the transition state, since Ki values in this range would be typical of transition state inhibitors, as is demonstrated in the art. Certainly, the question then remained as to whether or not the mode of inhibition was as we had postulated. We believed that the Z-LLL- moiety for our top inhibitor provided significant binding interactions, which lowered ESB and TSB but did not raise $TS_R$. Moreover, we hypothesized that the fluoromethylketone moiety may be interacting specifically to raise $TS_R$. To study this hypothesis, we endeavored next to screen the fluoromethylketone moiety (coupled with Z-LLL) for its ability to inhibit the cysteine protease in the transition state.

*Results from the Crude Mix*

When a crude mix of parasite extract was used instead of the purified cysteine protease, a highly similar inhibition series was noted. This remarkable similarity demonstrated that the proteolytic activity determined for the crude mix, at pH 4.9, was indeed caused by the operative cysteine protease. Moreover, we believe it is also indicative that other isoforms (with different proteolytic properties) did not predominate at pH 4.9.

*EXAMPLE 6: Differential cleavage test to identify TSPIs or GSPIs that are selective for the target protease ("Step VI" and "Step IX")*

This example explores the selectivity of vehicles and of inhibitors towards human proteases. As already discussed, selectivity is an important and preferred feature of a protease inhibitor drug since selectivity renders it capable of inhibiting the target protease of the pathogen, without affecting general proteases of the human or nonhuman host. To evaluate selectivity, appropriate controls and assay setups were set up similarly in both Step VI and Step IX. For this reason, both steps will be elaborated upon in this one example.

In order to perform the assay, the vehicle (for Step VI) or inhibitor (for Step IX) were incubated with the appropriate substrate in the correct assay buffer and allowed to equilibrate. Subsequently, the protease was added, and the assay was allowed to incubate for an appropriate time. The concentrations used in this experiment were as follows: Vehicle was set up at $4 \times 10^{-5}$ M in final assay buffer; Inhibitor was set up at $3 \times 10^{-10}$ M in the final assay buffer. Preferably the dosages employed should be the minimal amount of vehicle or inhibitor necessary to induce inhibition. The dosages indicated here were found to be sufficient for inhibition. Substrates were set up based upon the preferred substrate for cleavage of the particular enzyme.

Positive controls were set up similarly as described for test controls, except that a solvent blank was added in substitution for the inhibitor or vehicle (in a volume equal to the volume of inhibitor or vehicle addition). All of the inhibitors and vehicles could be dissolved in dimethylsulfoxide (DMSO), and therefore, this was the solvent of choice. Negative controls were set up by adding the inhibitor or vehicle and the preferred substrates in a tube without the protease. All potential non-proteolytic liberation of AFC was therefore controlled for in this tube.

All controls were set up in triplicate, and experiments were repeated a minimum of five times.

Percent inhibition was calculated as:

$$\% \text{ Inhibition} = \frac{(\text{AFC positive for "SI"} - \text{AFC negative for "SI"}) - (\text{AFC test} - \text{AFC negative for "SI"}) \times 100}{\text{AFC positive for "SI"} - \text{AFC negative for "SI"}}$$

In this preliminary test, we employed the following human-derived enzymes (with indicated mechanistic class in parentheses): Cathepsin B (cysteine), Cathepsin L (cysteine), Cathepsin H (cysteine), Cathepsin D (aspartic), Trypsin (serine), the purified *Taenia solium* cysteine protease, and the *Taenia solium* crude mix. Cathepsin B was prepared in an assay buffer of 10 mM L-cysteine in 0.4 M Citrate buffer, pH 6.1, using Z-ARR-AFC as the cleavage substrate. Cathepsin L was prepared in an assay buffer of 10 mM L-cysteine in 0.4 M Citrate buffer, pH 5.5, using Z-FR-AFC as the cleavage substrate. Cathepsin H was prepared in an assay buffer of 10 mM L-cysteine in 0.15 M PBS buffer, pH 6.8, using L-R-AFC as the cleavage substrate. Cathepsin D was prepared in an assay buffer of 0.15 M PBS, pH 7.1, using Z-RGFFP-AFC as the cleavage substrate. Trypsin was prepared in an assay buffer of 0.15 M PBS, pH 8.0, supplemented with 100 mM $CaCl_2$ and with Z-R-AFC as the cleavage substrate. Enzyme was incubated so that it was at least 50 times less than the concentration of substrate in order to insure saturating conditions.

Our results (Table 3) demonstrated that the vehicles, Z-LLL-AFC, Z-LLY-AFC, Mu-LY-AFC and the inhibitors, Z-LLL-FMK and Z-LLY-FMK were selective for the inhibition of the *Taenia solium* cysteine protease. Inhibition of other proteases was completely negligible. Notably however, inhibition of cathepsin L by Z-LLL-FMK was apparent, although it was almost half of the inhibition of the *Taenia solium* cysteine protease. This inhibition may indicate a subtle similarity of mechanisms shared between cathepsin L and the *Taenia solium* cysteine protease, with respect to inhibitor binding, although there was certainly a preference of Z-LLL-FMK for the latter protease. Moreover, although Z-LLY-FMK (62%) did not inhibit the *Taenia solium* protease as efficaciously as Z-LLL-FMK(98%), it did not inhibit cathepsin L at all. This finding may indicate that Y at P1 would be a preferable amino acid substitution for a selective result. As discussed in the above, it would be up to the investigator to determine whether or not to potentially employ these results for further selection of the vehicle. Based upon the fact that there was a small, but notable inhibition of cathepsin L by Z-LLL-FMK, and that this inhibition was absent when Z-LLY-FMK was employed as a substrate, we decided to further explore the efficacy of Z-LLY-FMK (in addition to Z-LLL-FMK) as a candidate lead compound. Presently, transition state assessments of both Z-LLL-FMK and Z-LLY-FMK are being conducted, whereas preliminary results suggest that both have transition state character.

Among the vehicles, Mu-LY-AFC was noted to cause subtle inhibition of the cathepsin L protease although this was only a small fraction of inhibition of the *Taenia solium* cysteine protease. It was notable that Z-LLL-AFC produced slightly less inhibition than Z-LLY-AFC and Mu-LY-AFC, when all were used at the same concentration. This was not a surprising finding. As discussed above, Z-LLL-AFC, Z-LLY-AFC, and Mu-LY-AFC tended to produce similar inhibition profiles which remained rather close and sometimes indistinguishable from experiment to experiment.

Based upon these results, we concluded that a differential inhibition of the *Taenia solium* cysteine protease by all vehicles and inhibitors chosen was apparent. It was notable that (with the slight exception of cathepsin L) no inhibitors or vehicles inhibited any of the other tested cysteine protease enzymes to any substantial degree.

Table 3. Inhibitor selectivity profile of top inhibitors and vehicles against a panel of proteases of different mechanistic classes.

| Protease (mechanistic class) | Z-LLL-FMK | Z-LLY-FMK | Z-LLL-AFC | Z-LLY-AFC | Mu-LY-AFC |
|---|---|---|---|---|---|
| *T. solium* (cysteine) | 98% | 62% | 68% | 70% | 75% |
| Cathepsin B (cysteine) | 0% | 0% | 0% | 0% | 0% |
| Cathepsin H (cysteine) | 0% | 0% | 0% | 0% | 0% |
| Cathepsin L (cysteine) | 50% | 0% | 0% | 0% | 25% |
| Cathepsin D (aspartic) | 0% | 0% | 0% | 0% | 0% |
| Trypsin (serine) | 0% | 0% | 0% | 0% | 0% |

*EXAMPLE 7: Assessment of protease inactivator in reducing T. crassiceps cysteine protease cleavage in BALB/c mice ("Step VIII")*

The goal of the following experiments was to assess the proof of concept for Z-LLL-FMK and Z-LLY-FMK beyond their transition state data and into animal models. For this purpose, the animal model for human neurocysticercosis (NCC) was employed. NCC is cited as the most common parasitic disease of the human central nervous system (Del Brutto et al., 1993), as well as the leading cause of epileptic form seizures in many parts of the Third World (Tsang et a11995). We hypothesized that the target cysteine protease helps the causative parasite, *Taenia solium,* survive in the host. Fortunately, a related parasite, *Taenia crassiceps,* can be propagated effectively in an animal model employing BALB/c mice. This was the focus of the proof of concept studies for Z-LLL-FMK and Z-LLY-FMK. We wished to employ K-Pyr (Leu-Phe-Ketoamide-Pyridyl) as a control, as it also represented a third, although less efficacious, inhibitor for the target cysteine protease.

We had produced significant data in the past as described in the WO 00/63350 patent indicating that there was a similar protease in *Taenia crassiceps* and that its active site is similar to the active site of the *Taenia solium* cysteine protease. Part of this data included the finding that the top inhibitors found here similarly inhibited the purified *T. crassiceps* cysteine protease, further demonstrating the biochemical similarity of the two enzymes' active sites (data not shown). Based upon this homology, our prediction was that an anti-*T. solium* cysteine protease inhibitor would have a inhibitory effect against the *T. crassiceps* cysteine protease, and cause a biological problem for the cyst if the cysteine protease was truly important to the parasite. To our knowledge, this cysteine protease conferred the function of immune evasion and immune exploitation to the parasite (WO 00/63350, White et al., 1997). Our prediction was that inhibiting it would cause recognition of the cyst in the animal by the immune response, which would thereby inhibit its budding process and growth. This was a testable hypothesis. BALB/c mice treated with the specific inhibitors Leucine-Leucine-Tyrosine-Fluoromethylketone and Leucine-Leucine-Leucine-Fluoromethylketone were protected 85-97% and 100%, respectively, from cysticercosis infection in a representative experiment.

*Short-Term Studies*

Based upon the premise that the cyst wall cysteine protease may serve a critical function to the life cycle of the *Taenia* cyst, we tested our most efficacious inhibitors, LLL-FMK and LLY-FMK (K-Pyr was used as a control), for their abilities to protect mice from cysticercosis infection. LLY-FMK and LLL-FMK were individually tested as prophylactics in a preliminary trial with BALB/c mice. Mice in treated groups were pre-injected for two days with the inhibitor [-1.4 X 10-$^2$M, dissolved in an injection vol. of 150 ul 0.15M PBS] followed by infection with 10 *T. crassiceps* cysts/mouse. Mice were subsequently dosed daily with the same concentration for four weeks. After one month, the mice were euthanized and the cysts counted. It was found that mice in groups that had been treated with LLL-FMK were protected 100% from cysticercosis infection (Table 4). Mice in groups that had been treated with LLY-FMK were protected 85%-97% from cysticercosis infection, in comparison to untreated controls (Fig. 8) a full representative study is shown). A subsequent study repeated the identical prophylactic experiment with LLY-FMK and demonstrated similar protection data (75%-90%) Interestingly, a high percentage of cysts that did survive the treatment demonstrated abnormal morphology. Many of these cysts exhibited apolar multilocularity (multi-lobed appearances), an abnormal budding pattern. Under histological exam, multilobed cysts showed enlarged walls compared to normal lobed cysts. Mice in groups treated with K-Pyr (Leu-Phe-Ketoamide-Pyridyl) were protected 40 %.

A therapeutic study with LLL-FMK was also conducted and revealed 60% protection when inhibitor treatment began two weeks after mice had been infected with *T. crassiceps* cysts. Mice in all groups survived without noticeable side effects (e.g., no coat condition, tail dragging or paresis, etc).

*Long-Term Studies*

Long-term studies were also conducted to assess the period over which these inhibitors could maintain a protective effect. In a representative experiment shown here, mice were divided into treated and untreated groups (5 mice/group). Each mouse in the treated group was pre-injected intraperitoneally with inhibitor for two days. Subsequently, all groups were challenged with 10 *T. crassiceps* cysts in 200 ul 0.15M PBS. Treatment with inhibitor or placebo (0.15M PBS) was carried out every day for 30 days post-challenge. Cysts were then left alone (untreated) for approximately 5 months, and then euthanized. Cysts were then counted by visual inspection upon autopsy. Percent protection was based reduction of total cyst number in comparison to controls (did not receive treatment). As can be seen, the top two inhibitors, Z-LLY-FMK and LLL-FMK continued to protect the mice 95% and 80%, respectively. The conclusion of this study is that protection of the animals from cysticercosis was persistent, even over a long period of time, which is a further testimony to the efficacy of the inhibitors in the models. It is also notable that no side effects were noted for the treatments with Z-LLL-FMK and Z-LLY-FMK, possibly due to the fact that very low dosages of the inhibitor were used. Conversely, side effects including goiter and neurological problems were noted in mice within the groups B-FA-CH2F and Z-LF-VSPH, where VSPH is vinylsulfonephenyl.

*EXAMPLE 8. Demonstration that the cysteine protease was the target of the specific inhibition by Z-LLY-FMK.*

Finally, it is notable that a dose response of *in vitro* inhibition of the *T. solium* cysteine protease correlates with *in vivo* protection in BALB/c mice. Moreover, cysts removed from mice treated with the less effective inhibitor, K-Pyr, had neither adherent immune cells nor damaged tegument. These observations are suggestive that inhibition of the *Taenia* cysteine protease is the mode of action for these protection results.

Table 4. Correlation between *in vitro* inhibition of *T. solium* cysteine protease and *in vivo* protection. "n/a" refers to unavailable data.

| Inhibitor | % Inhibition of T. solium CP in vitro | % Prophylactic protection of BALB/c mice | % Therapeutic protection of BALB/c mice | Immune cells observed by SEM on cyst surfaces |
|---|---|---|---|---|
| LLL-FMK | 100% | 100% | 60% | N/a |
| LLY-FMK | 97% | 85-97% | n/a | Intense |
| K-Pyr | 80% | 40% | n/a | None observed |

*EXAMPLE 9. Scanning electron microscopy of surfaces on cysts removed from mice treated with LLY-FMK demonstrating immunological debris on the tegumentary surface.*

SEM examination of the few surviving cysts that were removed from mice treated with LLY-FMK in the experiment from Fig. 9, revealed consistencies with our theory about the role of the cysteine protease in the host/parasite interaction. For example, we had hypothesized that the cysteine protease was a key parasitic molecule that enabled the cyst to evade the host immune response by cleaving host antibodies. By inhibiting the *Taenia* cysteine protease with Z-LLY-FMK, we hypothesized that host immunoglobulin could now bind the parasites, and initiate immunological responses (e.g., complement via IgG2a), thus causing damage to the cyst wall. Our SEM results confirmed the presence of adherent immune cells, which were concomitantly present where the cyst wall appeared to be undergoing destruction (Fig. 9). We identified macrophages, neutrophils, fibroblasts, and collagenous deposits on the cyst surfaces from treated mice, in comparison to their absence on cysts from untreated mice. General surface atrophy was also apparent. Microtriches (microvilli) were either sloughed off in entire regions or significantly shortened in general, compared to cysts that were removed from untreated mice. Breaks in the integrity of the cyst surface were also noted for the cysts removed from treated mice. Higher magnification revealed tegumental erosion near immunological cells. Cysts removed from untreated mice were characterized by no immune cells, by intact and longer microtriches, and by homogeneity of the cyst surface. These results are consistent with other studies, which have shown that viable cysticerci in pig muscle and those removed from humans at autopsy show little surrounding host inflammation.

As mentioned earlier, the other mode of action of the inhibitor could be the prevention of immune exploitation, e.g., the inhibitor may have weakened the cysts by blocking the cysteine protease's breakdown of IgG, which could be a significant source of nutrients to the parasite (Damian, 1987). "Weakened" cysts may consequently have become more susceptible to immune attack, although it appears that the host mounted a contained immune response since there were no side effects in the treated mice. We hypothesize that the consequence of these processes is the halting of further cyst growth and proliferation, and thus a protective capability of this inhibitor *in vivo.* With the treatment of human NCC as one of our goals (the other being a prophylactic for porcine cysticercosis), the prospect of an *in situ* immunological response in the CNS following inhibitor treatment can be of some concern. However, use of such a drug in conjunction with anti-inflammatory steroids and possibly with the present treatment of Albendazole, should result in amelioration of such a response if indeed it were to occur.

*EXAMPLE 10. Nontoxicity of Protease Inactivation*

Our preliminary results have shown that LLL-FMK and LLY-FMK are nontoxic to normal BALB/c mouse splenocytes *in vitro.* In these experiments, mouse splenocytes were removed and treated with either inhibitor alone or inhibitor and Con-A (Fig. 10). As can be seen, the inhibitors do not induce a proliferative response (as measured by $^3$H-thymidine incorporation). Furthermore, the inhibitors do not affect the responsiveness of the splenocytes to Con-A.

Furthermore, trypan blue staining of all cells demonstrates that all of the mouse cells are viable, even if the inhibitor is present (Fig 11). These studies further support our observation that no host side effects were observed in mice when they were treated with the specific inhibitors *in vivo* (Fig. 8a and 8b).

These data suggest the feasibility with which the transition state level protease inactivators, Z-LLL-FMK and Z-LLY-FMK, developed *in vitro,* may work *in vivo.* Their specificity for the target cysteine protease is attested to by the success of animal protection studies. This is demonstrated by the observations that *in vitro* inhibition of the cysteine protease is correlated with *in vivo* protection. Moreover, as the most potent inhibitor *in vitro* demonstrated the most rigorous and destructive immune response on cysts *in vivo,* these results suggest that the inhibitors were specific for the target cysteine protease and that the cysteine protease was the target. Additionally, the inhibitors showed selectivity *in vitro* as demonstrated by Step VIII. *In vivo* selectivity towards the target protease was demonstrated by the fact that only negligible side effects were ever noticed in animals treated with Z-LLL-FMK and Z-LLY-FMK during a long or short-term period.

*EXAMPLE 11: Phosphatases*

Protein phosphatases illustrate the utility of the invention method for developing inhibitors and inactivators toward for categories of scission enzymes other than proteases. For each scission enzyme, the peptide core is used in the same manner at each step as it would be for protease inhibitor development, except that the detection means are tailored to the group cleaved by the enzyme. Thus whereas chromophores linked to a peptide terminus were employed to assess the degree of cleavage for phosphatases, for protein phosphatases radiolabelled phosphate moieties may serve as the detection group, these being assayable by a scintillation counter. Similarly, a fluorescent assay system an be used for phosphatase detection as is known in the art. Likewise for glycoproteins, glucose assays may be employed instead of chromophores to measure cleavage, although the peptide core itself remains constant.

EP 1 379 634 B1

Thus in an illustrative example of the use of the method with protein phosphatases, a series of monophosphotripeptides is assayed against the target protein phosphatase in step II. Elsewhere in the method, at each point where the chromophore is employed in the protease system, the phosphoro group is used for the protein phosphatase system. However, if desired the same inactivating reactants may be for protein phosphatase inhibitors as for protease inhibitors.

It should be noted that in many cases the entire method that has been illustrated using proteases is applicable without modification to other types of scission enzymes, and that the invention is not limited by the choice of cleavable group or by the manner of detecting cleavage.

REFERENCES

Albery and Knowles, "Efficiency and evolution of enzyme catalysis," Angew Chem Int Ed Engl. 1977 May;16(5):285-93.

Appelt et al. "Design of enzyme inhibitors using iterative protein crystallographic analysis," J Med Chem. 1991 Jul;34(7):1925-34.

Baldwin and Rose. "Is protein folding hierarchic? II. Folding intermediates and transition states," Trends Biochem Sci. 1999 Feb;24(2):77-83.

Berger and Schechter. "Mapping the active site of papain with the aid of peptide substrates and inhibitors," Philos Trans R Soc Lond B Biol Sci. 1970 Feb 12;257(813):249-64.

Bergmann et al., J. Biol. Chem. 1950; 196:693.

Cannon et al., "A perspective on biological catalysis," Nat Struct Biol. 1996 Oct;3(10):821-33.

Cleland, "Isotope effects: determination of enzyme transition state structure," Methods Enzymol. 1995;249:341-73.

Damian, "The exploitation of host immune responses by parasites," J Parasitol. 1987 Feb;73(1):3-13.

Del Brutto et al., "Therapy for neurocysticercosis: a reappraisal," Clin Infect Dis. 1993 Oct;17(4):730-5.

Erickson et al. "Design, activity, and 2.8 A crystal structure of a C2 symmetric inhibitor complexed to HIV-1 protease," Science. 1990 Aug 3;249(4968):527-33

Fersht. 1985. Enzyme Structure and Mechanism. Ch. 12. W.H. Freeman and Company. New York

Fersht et al., "Reconstruction by site-directed mutagenesis of the transition state for the activation of tyrosine by the tyrosyl-tRNA synthetase: a mobile loop envelopes the transition state in an induced-fit mechanism," Biochemistry. 1988 Mar 8;27(5):1581-7.

Hayunga et al., "Evidence for selective incorporation of host immunoglobulin by strobilocerci of Taenia taeniaeformis," J Parasitol. 1989 Aug;75(4):638-42.

Jencks, Cold Spring Harbor Symposia on Quantitative Biology. LII: 65-73 (1987).

Johnson, et al., "Changes in absorption spectrum and crystal structure of triose phosphate isomerase brought about by 2-phosphoglycollate, a potential transition state analogue," J Mol Biol. 1970 Jan 14;47(1):93-100.

Kamphuis et al., "Structure of papain refined at 1.65 A revolution," J Mol Biol. 1984 Oct 25;179(2):233-56.

Kraut, J., "How do enzymes work?" Science. 1988 Oct 28;242(4878):533-40.

Kreevoy and Truhlar. Techniques of Chemistry. (C.f. Bernasconi, Ed).6:14-97. Wiley, New York, New York.

Kubinyi, "Chance favors the prepared mind--from serendipity to rational drug design, J Recept Signal Transduct Res. 1999 Jan-Jul;19(1-4):15-39

Ladbury, J et al., "Turning up the heat on rational drug design," Biotechnology (N Y). 1994 Nov;12(11):1083-5.

Lahana, "How many leads from HTS?" Drug Discov Today. 1999 Oct;4(10):447-448.

Ma et al., "Transition-state ensemble in enzyme catalysis: possibility, reality, or necessity?" J Theor Biol. 2000 Apr 21;203(4):383-97.

McKerrow et al., "Cysteine protease inhibitors as chemotherapy for parasitic infections," Bioorg Med Chem. 1999 Apr;7(4):639-44.

Menger, "Analysis of ground-state and transition-state effects in enzyme catalysis," Biochemistry. 1992 Jun 16;31(23):5368-73.

Murphy, "Revisiting ground-state and transition-state effects, the split-site model, and the "fundamentalist position" of enzyme catalysis," Biochemistry. 1995 Apr 11;34(14):4507-10.

Parril, A. and Reddy, R. 1999. Rational Drug Design: Novel Methodology and Practical Applications. ACS Symposium Series 719. Oxford University Press.

Pauling, 1946. Chem. Eng. News. 24: 1375.

Pauling, 1948. Nature. 161:707.

Polgar, "The different mechanisms of protease action have a basic feature in common: proton transfer from the attacking nucleophile to the substrate leaving group," Acta Biochim Biophys Hung. 1988;23(3-4):207-13.

Radzicka and Wolfenden. 1996. Contemporary Enzyme Kinetics and Mechanism. 2nd ed. (Purich, Ed). San Diego CA Pp273-298.

Roberts et al., "Rational design of peptide-based HIV proteinase inhibitors," Science. 1990 Apr 20;248(4953):358-61.

Schowen. 1978. Transition States of Biochemical Processes (Gandour, R.D., and Schowen, R.L., Eds.) Chapter 2, Plenum, New York.

Service, "Structural genomics offers high-speed look at proteins," Science. 2000 Mar 17;287(5460):1954-6.

Szedlacsek et al., "Kinetics of slow and tight-binding inhibitors," Methods Enzymol. 1995;249:144-80.

Tsai and Jordan, 1993. J. Phys. Chem. 97(11): 11227-11237.

Threadgold et al., "Taenia crassiceps: regional variations in ultrastructure and evidence of endocytosis in the cysticercus' tegument," J Exp Parasitol. 1983 Feb;55(1):121-31.

Tsang et al., Parasitol. Today, 11, 124-126 (1995).

Vacca et al., "L-735,524: an orally bioavailable human immunodeficiency virus type 1 protease inhibitor," Proc Natl Acad Sci U S A. 1994 Apr 26;91(9):4096-100.

Wang et al., "The double catalytic triad, Cys25-His159-Asp158 and Cys25-His159-Asn175, in papain catalysis: role of Asp158 and Asn175," Protein Eng. 1994 Jan;7(1):75-82.

Wolfenden, "Conformational aspects of inhibitor design: enzyme-substrate interactions in the transition state," Bioorg Med Chem. 1999 May;7(5):647-52.

**Claims**

1. A method for identifying a peptide scission enzyme inhibitor comprising:

   a. performing a low kcat substrate assay by contacting each of a plurality of substrates with a target peptide scission enzyme to identify at least one high kcat peptide substrate and at least one low kcat peptide substrate being poorly cleavable by the target peptide scission enzyme, wherein the low kcat peptide substrate has a cleavability rate of less than 35% of the cleavability rate of the most highly cleaved substrate in the assay; and
   b. performing an enzyme active site competitive binding assay using the target peptide scission enzyme, at least one high kcat substrate identified in step a. and at least one low kcat substrate identified in step a. and identifying thereby at least one low kcat substrate being an active site binding inhibitor, which inhibits the cleavage of at least one high kcat substrate by the target peptide scission enzyme by binding to the active site of the target peptide scission enzyme, thereby identifying a low kcat active site binding enzyme inhibitor comprising an amino acid core or peptide core.

2. The method of claim 1, wherein the low kcat substrate has a cleavability rate of less than 20% of the cleavability rate of the most highly cleaved substrate in the assay.

3. The method of claim 2, wherein the low kcat substrate has a cleavability rate of less than 10% of the cleavability rate of the most highly cleaved substrate in the assay.

4. The method of claim 1, wherein the enzyme active site competitive binding assay comprises assessment of the binding of the substrate to the enzyme by employing a spectroscopic method selected from the group consisting of nuclear magnetic resonance spectroscopy, infrared spectroscopy, ultra-violet-visual spectroscopy, fluorometric spectroscopy, phosphorometric spectroscopy, mass spectrometry, and microwave spectroscopy.

5. The method of claim 1, wherein performing the low kcat substrate assay comprises assessing the kcat of the substrate for the enzyme by employing a spectroscopic method selected from the group consisting of nuclear magnetic resonance spectroscopy, infrared spectroscopy, ultraviolet-visual spectroscopy, fluorometric spectroscopy, phosphorometric spectroscopy, mass spectrometry, and microwave spectroscopy.

6. The method of claim 1, further comprising the additional steps of selecting a functional and structural analog of the low kcat inhibitor and performing an inhibitory assay using the target peptide scission enzyme and at least one structural analog of the low kcat inhibitor to identify at least one low kcat inhibitor.

7. The method of claim 1, further comprising assessing the selectivity of the low kcat inhibitor with respect to enzymes of the same class as the target peptide scission enzyme in order to determine whether the inhibitor is selective for the target peptide scission enzyme.

8. The method of claim 1 further comprising determining optimal substrate cleavage conditions for the target peptide scission enzyme prior to contacting the target peptide scission enzyme with the plurality of substrates.

9. The method of claim 1, wherein the peptide scission enzyme inhibitor comprises an amino acid core or a peptide core containing between 1 and 9 amino acid residues.

10. The method of claim 9 wherein the amino acid core or peptide core contains between 1 and 6 amino acid residues.

11. The method of claim 9 wherein the amino acid core or peptide core consists of 1, 2 or 3 amino acid residues.

12. The method of any of the preceding claims wherein the amino acid core or peptide core comprises one or more residues from the group consisting of L-amino acids, D-amino acids, derivatized amino acids, and modified amino acids.

13. The method of any of the preceding claims wherein the amino acid core or peptide core is composed entirely or in part of peptide analogs selected from the group consisting of: beta-amino acids, peptoids, and amino acids in which one or more atoms have been replaced by different atomic elements or by functional groups.

14. The method of any of the preceding claims wherein the amino acid core or peptide core comprises two or more amino acids linked by a bond other than a peptide bond.

15. The method of claim 1, wherein the target peptide scission enzyme is provided as a purified enzyme or in a crude mix.

16. The method of claim 1, further comprising prior to performing the enzyme active site binding assay, determining an optimal pH range of about 2 pH units for the cleavage reaction.

17. The method of claim 1, comprising performing a competitive binding assay on a selected number of low kcat substrates, wherein performing the competitive binding assay comprises, for each selected low kcat substrate:

performing a first competitive binding assay using the target peptide scission enzyme, a first population of high kcat substrates, and the selected low kcat substrate to identify a plurality of low kcat inhibitors;
for each low kcat inhibitor, performing a second competitive binding assay using the target peptide scission enzyme, a second population of high kcat substrates and the low kcat inhibitor, wherein the second population of high kcat substrates includes a greater number of substrates that the first population of high kcat substrates; and
quantifying the inhibitory effect of the low kcat inhibitors to yield a ranked list of low kcat inhibitors.

18. The method of claim 1 wherein a blocking group is located at a site selected from the following combination: the C-terminus of the peptide core; the N-terminus of the peptide core.

19. The method of claim 1 wherein the enzyme is selected from the group consisting of: proteases, protein phosphatases, esterases, enzymes cleaving saccharide-based side chains of proteins, enzymes that cleave lipoproteins or lipid-functionalized proteins, and enzymes that cleave cofactors from proteins.

20. The method of claim 1 wherein the amino acid core or peptide core comprises one or more residues from the group consisting of L-amino acids, D-amino acids, derivatized amino acids, and modified amino acids.

21. The method of claim 1 wherein the amino acid core or peptide core is composed entirely or in part of peptide analogs selected from the group consisting of: beta-amino acids, peptoids, and amino acids in which one or more atoms have been replaced by different atomic elements or by functional groups.

22. The method of claim 1 wherein the amino acid core or peptide core comprises two or more amino acids linked by a bond other than a peptide bond.

**Patentansprüche**

1. Verfahren zum Identifizieren eines Peptidspaltungsenzyminhibitors umfassend:

a. Durchführen einer Untersuchung auf Niedrig-kcat-Substrat durch Kontaktieren von jeweils einer Vielzahl von Substraten mit einem Zielpeptidspaltungsenzym, um wenigstens ein Hoch-kcat-Peptidsubstrat und wenigstens ein Niedrig-kcat-Peptidsubstrat, welches durch das Zielpeptidspaltungsenzym nur schlecht gespalten wird, zu identifizieren, wobei das Niedrig-kcat-Peptidsubstrat eine Spaltungsrate von weniger als 35 % der Spaltungsrate des am höchsten gespaltenen Substrats in der Untersuchung aufweist, und
b. Durchführen einer Untersuchung auf Enzym-aktives Zentrum-konkurrierende Bindung unter Verwendung des Zielpeptidspaltungsenzyms, wenigstens eines Hoch-kcat-Substrats, welches in dem Schritt a. identifiziert worden ist, und wenigstens eines Niedrig-kcat-Substrats, welches in dem Schritt a. identifiziert worden ist, und Identifizieren dadurch wenigstens eines Niedrig-kcat-Substrats, welches ein an aktives Zentrum bindender Inhibitor ist, welcher das Spalten wenigstens einen Hoch-kcat-Substrats durch das Targetpeptidspaltungsenzym durch Binden an das aktive Zentrum des Zielpeptidspaltungszenzyms inhibiert, um dadurch einen Niedrig-kcat-aktives Zentrum-Bindungsenzyminhibitor umfassend einen Aminosäurekern oder Peptidkern zu identifizieren.

2. Verfahren nach Anspruch 1, wobei das Niedrig-kcat-Substrat eine Spaltungsrate von weniger als 20 % der Spaltungsrate des am höchsten gespaltenen Substrats in der Untersuchung aufweist.

3. Verfahren nach Anspruch 2, wobei das Niedrig-kcat-Substrat eine Spaltungsrate von weniger als 10 % der Spaltungsrate des am höchsten gespaltenen Substrats in der Untersuchung aufweist.

**4.** Verfahren nach Anspruch 1, wobei die Untersuchung auf Enzym-aktives Zentrum-konkurrierende Bindung die Beurteilung des Bindens des Substrats an das Enzym durch Einsatz eines spektroskopischen Verfahrens umfasst, welches aus der Gruppe ausgewählt ist, welche besteht aus nuklearer magnetischer Resonanzspektroskopie, Infrarotspektroskopie, ultraviolett-sichtbares Licht-Spektroskopie, Fluorometrie-Spektroskopie, Phosphorometrie-Spektroskopie, Massenspektroskopie und Mikrowellenspektroskopie.

**5.** Verfahren nach Anspruch 1, wobei die Durchführung der Niedrig-kcat-Substratuntersuchung das Beurteilen von kcat des Substrats für das Enzym durch Einsatz eines spektroskopischen Verfahrens umfasst, welches aus der Gruppe ausgewählt ist, welche besteht aus nuklearer magnetischer Resonanzspektroskopie, Infrarotspektroskopie, ultraviolett-sichtbare Spektroskopie, fluorometrische Spektroskopie, Phosphorometriespektroskopie, Massenspektroskopie und Mikrowellenspektroskomie.

**6.** Verfahren nach Anspruch 1, des Weiteren umfassend die weiteren Schritte des Auswählens eines funktionellen und strukturellen Analogs des Niedrig-kcat-Inhibitors und Durchführen einer inhibitorischen Untersuchung unter Verwendung des Targetpeptidspaltungsenzyms und wenigstens eines strukturellen Analogs des Niedrig-kcat-Inhibitors zur Identifizierung wenigstens eines Niedrig-kcat-Inhibitors.

**7.** Verfahren nach Anspruch 1, des Weiteren umfassend das Bestimmen der Selektivität des Niedrig-kcat-Inhibotors in Bezug auf Enzyme derselben Klasse wie des Zielpeptidspaltungsenzyms, um zu bestimmen, ob der Inhibitor für das Zielpeptidspaltungsenzym selektiv ist.

**8.** Verfahren nach Anspruch 1, des Weiteren umfassend das Bestimmen der optimalen Substratspaltungsbedingungen für das Zielpeptidspaltungsenzym vor dem Kontaktieren des Zielpeptidspaltungsenzyms mit der Vielzahl von Substraten.

**9.** Verfahren nach Anspruch 1, wobei der Peptidspaltungsenzyminhibitor einen Aminosäurekern oder einen Peptidkern umfassend zwischen 1 und 9 Aminosäureresten umfasst.

**10.** Verfahren nach Anspruch 9, wobei der Aminosäurekern oder der Peptidkern zwischen 1 und 6 Aminosäureresten umfasst.

**11.** Verfahren nach Anspruch 9, wobei der Aminosäurekern oder der Peptidkern aus 1, 2 oder 3 Aminosäureresten besteht.

**12.** Verfahren nach einem der vorstehenden Ansprüche, wobei der Aminosäurekern oder der Peptidkern ein oder mehrere Reste ausgewählt aus der Gruppe bestehend aus L-Aminosäuren, D-Aminosäuren, derivatisierten Aminosäuren und modifizierten Aminosäuren umfasst.

**13.** Verfahren nach einem der vorstehenden Ansprüche, wobei der Aminosäurekern oder Peptidkern vollständig oder teilweise aus Peptidanaloga ausgewählt aus der Gruppe bestehend aus: Beta-Aminosäuren, Peptoiden und Aminosäuren, in denen ein oder mehrere Atome durch verschiedene Atomelemente oder funktionelle Gruppen ersetzt worden sind, zusammengesetzt ist.

**14.** Verfahren nach einem der vorstehenden Ansprüche, wobei der Aminosäurekern oder der Peptidkern zwei oder mehr Aminosäuren umfasst, die durch eine Bindung verschieden von einer Peptidbindung miteinander verbunden sind.

**15.** Verfahren nach Anspruch 1, wobei das Zielpeptidspaltungsenzym als ein gereinigtes Enzym oder in einer Rohmischung vorliegt.

**16.** Verfahren nach Anspruch 1, des Weiteren umfassend vor der Durchführung der Enzym-aktives Zentrum-Bindungsuntersuchung das Bestimmen eines optimalen pH-Bereichs von ungefähr 2 pH-Einheiten für die Spaltungsreaktion.

**17.** Verfahren nach Anspruch 1, umfassend das Durchführen einer konkurrierenden Bindungsuntersuchung mit einer ausgewählten Anzahl von Niedrig-kcat-Substraten, wobei das Durchführen der konkurrierenden Bindungsuntersuchung für jedes ausgewählte Niedrig-kcat-Substrat umfasst:

Durchführen einer ersten konkurrierenden Bindungsuntersuchung unter Verwendung des Zielpeptidspaltungsenzyms, einer ersten Population von Hoch-kcat-Substraten und des ausgewählten Niedrig-kcat-Substrats, um eine Vielzahl von Niedrig-kcat-Inhibitoren zu identifizieren,

für jeden Niedrig-kcat-Inhibitor das Durchführen einer zweiten konkurrierenden Bindungsuntersuchung unter Verwendung des Zielpeptidspaltungsenzyms, einer zweiten Population von Hoch-kcat-Substraten und des Niedrig-kcat-Inhibitors, wobei die zweite Population von Hoch-kcat-Substraten eine größere Anzahl von Substraten als die erste Population von Hoch-kcat-Substraten umfasst, und

Quantifizieren des inhibitorischen Effekts der Niedrig-kcat-Inhibitoren, um eine Rangfolgeliste von Niedrig-kcat-Inhibitoren zu erhalten.

**18.** Verfahren nach Anspruch 1, wobei eine Blockierungsgruppe an einer Stelle ausgewählt aus der folgenden Kombination: dem C-Ende des Peptidkerns, dem N-Ende des Peptidkems lokalisiert ist.

**19.** Verfahren nach Anspruch 1, wobei das Enzym aus der Gruppe ausgewählt ist, welche besteht aus: Proteasen, Proteinphosphatasen, Esterasen, Seitenketten von Proteinen auf Basis von Saccharid spaltenden Enzymen, Enzymen, welche Lipoproteine oder lipid-funktionalisierte Proteine spalten, und Enzymen, welche Co-Faktoren von Proteinen spalten.

**20.** Verfahren nach Anspruch 1, wobei der Aminosäurekern oder Peptidkern ein oder mehrere Reste aus der Gruppe bestehend aus L-Aminosäuren, D-Aminosäuren, derivatisierten Aminosäuren und modifizierten Aminosäuren umfasst.

**21.** Verfahren nach Anspruch 1, wobei der Aminosäurekern oder Peptidkern vollständig oder teilweise aus Peptidanaloga zusammengesetzt ist, welche aus der Gruppe ausgewählt werden, welche besteht aus: Beta-Aminosäuren, Peptoiden und Aminosäuren, in denen ein oder mehrere Atome durch verschiedene Atomelemente oder durch funktionelle Gruppen ersetzt worden sind.

**22.** Verfahren nach Anspruch **1,** wobei der Aminosäurekern oder Peptidkern zwei oder mehr Aminosäuren umfasst, welche durch eine von einer Peptidbindung verschiedene Bindung miteinander verbunden sind.

**Revendications**

**1.** Procédé d'identification d'un inhibiteur d'enzyme de scission peptidique comprenant :

a. la mise en oeuvre d'un dosage de substrat à basse valeur kcat par mise en contact de chacun d'une pluralité de substrats avec une enzyme de scission peptidique cible pour identifier au moins un substrat peptidique à valeur kcat élevée et au moins un substrat peptidique à basse valeur kcat difficilement clivable par l'enzyme de scission peptidique cible, dans lequel le substrat peptidique à basse valeur kcat a un taux de clivabilité inférieur à 35 % du taux de clivabilité du substrat le plus fortement clivé dans le dosage ; et

b. la mise en oeuvre d'un dosage de liaison par compétition au site actif de l'enzyme à l'aide de l'enzyme de scission peptidique cible, d'au moins un substrat à valeur kcat élevée identifié à l'étape a. et d'au moins un substrat à basse valeur kcat identifié à l'étape a. et l'identification ce faisant d'au moins un substrat à basse valeur kcat qui est un inhibiteur de liaison au site actif, qui inhibe le clivage dudit au moins un substrat à valeur kcat élevée par l'enzyme de scission peptidique cible par liaison au site actif de l'enzyme de scission peptidique cible, pour identifier ainsi un inhibiteur d'enzyme se liant au site actif comprenant un noyau acides aminés ou un noyau peptidique.

**2.** Procédé selon la revendication 1, dans lequel le substrat à basse valeur kcat a un taux de clivabilité inférieur à 20 % du taux de clivabilité du substrat le plus fortement clivé dans le dosage.

**3.** Procédé selon la revendication 2, dans lequel le substrat à basse valeur kcat a un taux de clivabilité inférieur à 10 % du taux de clivabilité du substrat le plus fortement clivé dans le dosage.

**4.** Procédé selon la revendication 1, dans lequel le dosage de liaison par compétition au site actif de l'enzyme comprend l'évaluation de la liaison du substrat à l'enzyme à l'aide d'un procédé spectroscopique choisi dans le groupe constitué par la spectroscopie par résonance magnétique nucléaire, la spectroscopie infrarouge, la spectroscopie ultraviolet-visible, la spectroscopie de fluorescence, la spectroscopie de phosphorescence, la spectroscopie de masse, et la spectroscopie micro-ondes.

**5.** Procédé selon la revendication 1, dans lequel la mise en oeuvre du dosage de substrat à basse valeur kcat comprend l'évaluation de la valeur kcat du substrat pour l'enzyme par un procédé spectroscopique choisi dans le groupe constitué par la spectroscopie par résonance magnétique nucléaire, la spectroscopie infrarouge, la spectroscopie ultraviolet-visible, la spectroscopie de fluorescence, la spectroscopie de phosphorescence, la spectroscopie de masse, et la spectroscopie micro-ondes.

**6.** Procédé selon la revendication 1, comprenant en outre les étapes supplémentaires de choix d'un analogue fonctionnel et structural de l'inhibiteur à basse valeur kcat et la mise en oeuvre d'un dosage d'inhibition à l'aide de l'enzyme de scission peptidique cible et d'au moins un analogue structural de l'inhibiteur à basse valeur kcat pour identifier au moins un inhibiteur à basse valeur kcat.

**7.** Procédé selon la revendication 1, comprenant en outre l'évaluation de la sélectivité de l'inhibiteur à basse valeur kcat par rapport aux enzymes de la même classe que l'enzyme de scission peptidique cible afin de déterminer si l'inhibiteur est sélectif ou non envers l'enzyme de scission peptidique cible.

**8.** Procédé selon la revendication 1 comprenant en outre la détermination des conditions de clivage optimales du substrat pour l'enzyme de scission peptidique cible avant mise en contact de l'enzyme de scission peptidique cible avec la pluralité de substrats.

**9.** Procédé selon la revendication 1, dans lequel l'inhibiteur d'enzyme de scission peptidique comprend un noyau acides aminés ou un noyau peptidique contenant entre 1 et 9 résidus acides aminés.

**10.** Procédé selon la revendication 9 dans lequel le noyau acides aminés ou le noyau peptidique contient entre 1 et 6 résidus acides aminés.

**11.** Procédé selon la revendication 9, dans lequel le noyau acides aminés ou le noyau peptidique est constitué de 1, 2 ou 3 résidus acides aminés.

**12.** Procédé selon l'une quelconque des revendications précédentes dans lequel le noyau acides aminés ou le noyau peptidique comprend un ou plusieurs résidus choisis dans le groupe constitué par les acides L-aminés, les acides D-aminés, les acides aminés obtenus par formation de dérivés, et les acides aminés modifiés.

**13.** Procédé selon l'une quelconque des revendications précédentes dans lequel le noyau acides aminés ou le noyau peptidique est en tout ou partie composé d'analogues peptidiques choisis dans le groupe constitué par : les acides bêta-aminés, les peptoïdes, et les acides aminés dans lesquels un ou plusieurs atomes ont été remplacés par des éléments atomiques différents ou par des groupes fonctionnels.

**14.** Procédé selon l'une quelconque des revendications précédentes dans lequel le noyau acides aminés ou le noyau peptidique comprend deux acides aminés ou plus joints par une liaison autre qu'une liaison peptidique.

**15.** Procédé selon la revendication 1, dans lequel l'enzyme de scission peptidique cible est fournie sous forme d'une enzyme purifiée ou dans un mélange brut.

**16.** Procédé selon la revendication 1, comprenant en outre avant la mise en oeuvre du dosage de liaison au site actif de l'enzyme, la détermination d'une plage de pH optimale d'environ 2 unités de pH pour la réaction de clivage.

**17.** Procédé selon la revendication 1, comprenant la mise en oeuvre d'un dosage de liaison par compétition sur un nombre choisi de substrats à basse valeur kcat, dans lequel la mise en oeuvre du dosage de liaison par compétition comprend, pour chaque substrat à basse valeur kcat choisi :

la mise en oeuvre d'un premier dosage de liaison par compétition à l'aide de l'enzyme de scission peptidique cible, d'une première population de substrats à valeur kcat élevée, et du substrat à basse valeur kcat choisi pour identifier une pluralité d'inhibiteurs à basse valeur kcat ;
pour chaque inhibiteur à basse valeur kcat, la mise en oeuvre d'un second dosage de liaison par compétition à l'aide de l'enzyme de scission peptidique cible, d'une seconde population de substrats à valeur kcat élevée et de l'inhibiteur à basse valeur kcat, dans lequel la seconde population de substrats à valeur kcat élevée comprend un plus grand nombre de substrats que la première population de substrats à valeur kcat élevée ; et
la quantification de l'effet inhibiteur des inhibiteurs à basse valeur kcat pour obtenir une liste ordonnée d'inhibiteurs à basse valeur kcat.

18. Procédé selon la revendication 1 dans lequel un groupe de blocage se trouve sur un site choisi en fonction de la combinaison suivante : l'extrémité C-terminale du noyau peptidique ; l'extrémité N-terminale du noyau peptidique.

19. Procédé selon la revendication 1 dans lequel l'enzyme est choisie dans le groupe constitué par : les protéases, les phosphatases protéiques, les estérases, les enzymes qui clivent les chaînes latérales saccharidiques des protéines, les enzymes qui clivent les lipoprotéines ou les protéines à fonction lipidique, et les enzymes qui clivent les co-facteurs des protéines.

20. Procédé selon la revendication 1 dans lequel le noyau acides aminés ou le noyau peptidique comprend un ou plusieurs résidus choisis dans le groupe constitué par les acides L-aminés, les acides D-aminés, les acides aminés obtenus par formation de dérivés, et les acides aminés modifiés.

21. Procédé selon la revendication 1 dans lequel le noyau acides aminés ou le noyau peptidique est en tout ou partie composé d'analogues peptidiques choisis dans le groupe constitué par : les acides bêta-aminés, les peptoïdes, et les acides aminés dans lesquels un ou plusieurs atomes ont été remplacés par des éléments atomiques différents ou par des groupes fonctionnels.

22. Procédé selon la revendication 1 dans lequel le noyau acides aminés ou le noyau peptidique comprend deux acides aminés ou plus joints par une liaison autre qu'une liaison peptidique.

Figure 1a

Figure 1b

Figure 1c

Figure 1d

Figure 2

EP 1 379 634 B1

| III. Competitor Assay | ◄— | II. High/Low Kcat Substrate Identification | ◄— | I. General Peptide Cleavage |

*Top Vehicles (Situation 1)*    Increasing % Inhibition using competitor assays with Cocktails

◄──────

1. LLL-AFC  2. LLY-AFC  3. LLW-AFC  4. LLR-AFC  5. VLL-AFC  6. LLP-AFC    ◄

*High V Values*

## Figure 3

Promoted to
Step VII (A)

Organic linking of inactivating reactants (U,V,W,X,Y,Z) to Top 2 Vehicles

(Top Vehicle)          (2nd Best Vehicle)
LLL-AFC                 LLY-AFC

LLL-U  LLL-V  LLL-W  LLL-X  LLL-Y  LLL-Z        LLY-U  LLY-V  LLY-W  LLY-X  LLY-Y  LLY-Z

⇓                                          ⇓

Step VII(B) - B                              Step VII(B) - B

Lower EC$_{100}$ Values                      Lower EC$_{100}$ Values

◄──────                                    ◄──────

1. LLL-X  2. LLL-Y  3. LLL-Z  4. LLL-U  5. LLL-V  6.LLL-W      1. LLY-V  2. LLY-W  3. LLY-X  4.LLY-U  5.LLY-Z  6. LLY-Y

⇓                                          ⇓

Step VII(B) - C, D                            Step VII(B)- C, D

Lower Ki Values                              Lower Ki Values

◄──────                                    ◄──────

1. LLL-X  2. LLL-Y  3. LLL-Z                  1. LLY-V  2. LLY-W  3. LLY-X

Promoted  ⇓

| VII(C): TSPI  or GSPI Determination |

| Parent: LLL-X tested for Transition State inhibition vs. Ground State inhibition | ——► | Construct Log (Km/kcat) vs. Ki and Log (Km) vs. Ki. Compare the R values. |

Case 1: Prior vehicle inhibition is noted with "LLL" theme.
Single amino acids variations are conducted based
upon this theme.

|   | Km/Kcat | Ki | Log Ki (M) |
|---|---------|-----|-----------|
| a. | LLL-AFC | LLL-X | |
| b. | LLY-AFC | LLY-X | |
| c. | LLW-AFC | LLW-X | |
| d. | LLR-AFC | LLR-X | |
| e. | LLV-AFC | LLV-X | |
| f. | LLP-AFC | LLP-X | |
| g. | LLV-AFC | LLV-X | |
| h. | LLQ-AFC | LLQ-X | |
| i. | LLT-AFC | LLT-X | |
| j. | LLS-AFC | LLS-X | |
| k. | LLT-AFC | LLT-X | |
| l. | LLM-AFC | LLM-X | |
| m. | LLG-AFC | LLG-X | |
| n. | LLE-AFC | LLE-X | |

(j)  m=1.5, R=.92    L1    L2   m=.96, R=.97
(e)
(g) (h)  (m)    (f)  L3
(k)        (d)    m=.8, R=.85
(c)          (i)
(l)
(b)    (n)
(a)

Log Km/kcat  (M Sec$^{-1}$)

| Compare R values of two Plots: |
| If R of log (Km/kcat) vs. log (Kj) > R of Log (Km) vs. Log (Ki), proceed with TS Assessment. Otherwise, proceed with GS assessment (next page). In this case, we assume R is greater for the former vs. the latter. Hence, "LLL" is analyzed for transition state properties. |

**Case 2:** Hints are used to make substitutions. For example, if L is noted to be predominant in inhibitory vehicles at P2, L at P2 may be a viable candidate. Similarly, if V at P1 was noticed in 50% of vehicles that inhibited 50% or greater, then V at P1 may be a good substitution. Therefore, these hints are entirely based upon the amino acids at different motifs.

**Case 3:** Random. In the event no hints are available, mutations are made at random sites.

**Case 4:** Truncation or Elongation. It may be desirable to decrease the number of amino acids or to increase the number of amino acids. For example, Z-LL may be attempted or Z-LLLL may be another possibility.

Parent: LLL-Y and LLL-Z Tested for TSS

**LLL-Y: Sample Pair**    **I**
| | |
|---|---|
| LLL-AFC; LLL-Y | 11.21 |
| LLY-AFC;LLY-Y | 8.42 |
| LLT-AFC; LLT-Y | 8.27 |
| LLR-AFC; LLR-Y | 7.31 |
| LLW-AFC; LLW-Y | 6.21 |

**LLL-Z: Sample Pair**    **I**
| | |
|---|---|
| LLL-AFC; LLL-Z | 10.12 |
| LLS-AFC; LLS-Z | 6.11 |
| LLW-AFC;LLW-Z | 4.32 |
| LLY-AFC; LLY-Z | 3.12 |
| LLR-AFC; LLR-Z | 2.33 |

TSS or GSS Assessments. (In this example we assume that we are pursuing a Transition State assessment)

(1) Determination of TSS (or GSS) for (X) Series = $R/(ABS(1-M)*1/P)$
Line 1 utilizes pts a,k,g,h.    (L1) score = $.92/[ABS(1-1.5)*(1/4)]= 7.36$
Line 2 utilizes pts a,b,l,c,d,m    (L2) score = $.96/[ABS(1-.96)*(1/6)]= 14.4$
Line 3 utilizes pts a,b,l,c,d,n,i,f (L3) score = $.88/[ABS(1-.91)*(1/8)]= 7.82$

3 Line examples are provided (more are possible). It is virtually impossible to reach a slope of 1. If a slope of 1 is reached, a value equivalent to .99 may be used to prevent division by 0.

As is noticed, the TSS (or GSS) is a combination of three variables: slope, regression and the number of points. The best line is Line 2, and it will be employed for the TSS (or GSS). Thus, a,k,l,c,d,m are the closest inhibitors showing transition state analog inhibition.

(2) Determination of Inhibitor Score for Compounds:
$I= Log(TSS/((Ki))$
a (LLL-X): $Log (14.4/ (10^{-12})) = 13.16$
b (LLY-X): $Log (14.4 (10^{-10})= 11.15$
l (LLM-X): $Log (14.4/(10^{-8})= 9.15$
c (LLW-X): $Log (14.4/(10^{-6})= 7.15$
d (LLR-X): $Log (14.4/(10^{-4}) = 5.15$
m (LLG-X): $Log (14.4 /(10^{-3}) = 2.16$

Reranking of Parent TSPI using I :

| Rank | TSPI | I | |
|---|---|---|---|
| 1. | LLL-X | 13.16 | |
| 2. | LLL-Y | 11.21 | Parent Rankings |
| 3. | LLY-X | 11.15 | (LLY-X is accidental) |
| 4. | LLL-Z | 10.12 | |
| 5. | LLM-X | 9.15 | |
| 6. | LLY-Y | 8.42 | Further Rankings |
| 7. | LLW-X | 8.31 | |
| 8. | LLT-Y | 8.27 | |

Molecular rearrangement of inactivating reactant. LLL-X. X is rearranged atomically. Atoms are replaced, or such. For ex., if X=Fluoromethylketone, the Fluorine might be replaced with a Chlorine.

OR

Molecular rearrangement of vehicle, keeping inactivating reactant constant.

**X. Further Refinement**

1. Extension of vehicle into additional enzyme subsites.
2. Stereochemical modification on vehicle or inactivating reactant.

Re-enter TSPI or GSPI determination

$I > ??????$

NO

YES

Test a large spectrum of more inactivating reactants, e.g., U and V on LLL for transition state inhibition (Or ground state inhibition if this is the case).

Find inhibitor scores for 2nd best vehicle core. Compare LLY-V, LLY-W, LLY-X.

**Ground State Assessments**

Test for Ground State(GS) Inhibition of LLL-X, LLL-Y, LLL-Z. Repeat identical experiments as testing for TSS of LLL-X, LLL-Y, and LLL-Z, *except plot log Km vs. log Ki.*

Calculate I score= Log (GSS/Ki)

If I is low, next option is to pursue ground state inhibition testing of more inactivating reactants. If I is still low, 2nd best vehicle core (e.g., LLY-V, LLY-W, LLY-X) can be tested.

$GSS > ??$

VIII. Natural Substrate

IX. Differential Cleavage Test

X. Refinement

XI. Combinatorial Library

XII. Crystallization

XIII. Addition of Delivery Molecules

Figure 4a

71

Figure 4b

72

Figure 5a

Figure 5b

| IgG | IgG with<br>CP<br>Minus cysteine | IgG with<br>CP with<br>cysteine | IgG with<br>CP with<br>E64 | IgG with<br>CP with<br>S-LY-AFC |

Figure 6

Figure 7a

Figure 7b

# Figure 8a

| Non-treated controls | Cyst # | Avg | SD | % Prot. | # ML | % ML | Avg |
|---|---|---|---|---|---|---|---|
| **Mouse 1** | 240 | 197.7 | 34.3 | | 30 | 12.5 % | 14.4% |
| Mouse 2 | 156 | | | | 16 | 10.3% | |
| Mouse 3 | 197 | | | | 40 | 20.3% | |
| *Treated controls* | | | | | | | |
| Mouse 4 | 7 | 21.3 | 10.2 | 96.5% | 4 | 57% | 51% |
| Mouse 5 | 30 | | | 84.8% | 13 | 43% | |
| Mouse 6 | 27 | | | 86.4% | 14 | 52% | |

# Figure 8a

% Protection (Versus Untreated)

Figure 8b

Figure 9

Figure 10

Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0063350 A1
- WO 0009653 A1

- WO 0063350 A

### Non-patent literature cited in the description

- **WHITE et al.** *Am. Soc. of parasitologists,* 1996, vol. 82 (2), 7-10
- **WHITE et al.** *Mol. Biochem. Parasit.,* 1997, 243-253
- **BARRETT et al.** *Biochem. J.,* 1982, vol. E64, 189-198
- **MURPHY.** *Biochemistry,* 1995, vol. 34 (14), 4507-4510
- **ALBERY ; KNOWLES.** Efficiency and evolution of enzyme catalysis. *Angew Chem Int Ed Engl.,* May 1977, vol. 16 (5), 285-93
- **APPELT et al.** Design of enzyme inhibitors using iterative protein crystallographic analysis. *J Med Chem,* July 1991, vol. 34 (7), 1925-34
- **BALDWIN ; ROSE.** Is protein folding hierarchic? II. Folding intermediates and transition states. *Trends Biochem Sci.,* February 1999, vol. 24 (2), 77-83
- **BERGER ; SCHECHTER.** Mapping the active site of papain with the aid of peptide substrates and inhibitors. *Philos Trans R Soc Lond B Biol Sci.,* 12 February 1970, vol. 257 (813), 249-64
- **BERGMANN et al.** *J. Biol. Chem,* 1950, vol. 196, 693
- **CANNON et al.** A perspective on biological catalysis. *Nat Struct Biol.,* October 1996, vol. 3 (10), 821-33
- **CLELAND.** Isotope effects: determination of enzyme transition state structure. *Methods Enzymol,* 1995, vol. 249, 341-73
- **DAMIAN.** The exploitation of host immune responses by parasites. *J Parasitol.,* February 1987, vol. 73 (1), 3-13
- **DEL BRUTTO et al.** Therapy for neurocysticercosis: a reappraisal. *Clin Infect Dis.,* October 1993, vol. 17 (4), 730-5
- **ERICKSON et al.** Design, activity, and 2.8 A crystal structure of a C2 symmetric inhibitor complexed to HIV-1 protease. *Science,* 03 August 1990, vol. 249 (4968), 527-33
- **FERSHT.** Enzyme Structure and Mechanism. Ch. W.H. Freeman and Company, 1985, vol. 12

- **FERSHT et al.** Reconstruction by site-directed mutagenesis of the transition state for the activation of tyrosine by the tyrosyl-tRNA synthetase: a mobile loop envelopes the transition state in an induced-fit mechanism. *Biochemistry,* 08 March 1988, vol. 27 (5), 1581-7
- **HAYUNGA et al.** Evidence for selective incorporation of host immunoglobulin by strobilocerci of Taenia taeniaeformis. *J Parasitol.,* August 1989, vol. 75 (4), 638-42
- **JENCKS.** *Cold Spring Harbor Symposia on Quantitative Biology,* 1987, vol. LII, 65-73
- **JOHNSON et al.** Changes in absorption spectrum and crystal structure of triose phosphate isomerase brought about by 2-phosphoglycollate, a potential transition state analogue. *J Mol Biol,* 14 January 1970, vol. 47 (1), 93-100
- **KAMPHUIS et al.** Structure of papain refined at 1.65 A revolution. *J Mol Biol,* 25 October 1984, vol. 179 (2), 233-56
- **KRAUT, J.** How do enzymes work?. *Science,* 28 October 1988, vol. 242 (4878), 533-40
- **KREEVOY ; TRUHLAR.** Techniques of Chemistry. Wiley, vol. 6, 14-97
- **KUBINYI.** Chance favors the prepared mind--from serendipity to rational drug design. *J Recept Signal Transduct Res.,* January 1999, vol. 19 (1-4), 15-39
- **LADBURY et al.** Turning up the heat on rational drug design. *Biotechnology (N Y).,* November 1994, vol. 12 (11), 1083-5
- **LAHANA.** How many leads from HTS?. *Drug Discov Today.,* October 1999, vol. 4 (10), 447-448
- **MA et al.** Transition-state ensemble in enzyme catalysis: possibility, reality, or necessity?. *J Theor Biol.,* 21 April 2000, vol. 203 (4), 383-97
- **MCKERROW et al.** Cysteine protease inhibitors as chemotherapy for parasitic infections. *Bioorg Med Chem.,* April 1999, vol. 7 (4), 639-44
- **MENGER.** Analysis of ground-state and transition-state effects in enzyme catalysis. *Biochemistry,* 16 June 1992, vol. 31 (23), 5368-73

- **MURPHY.** Revisiting ground-state and transition-state effects, the split-site model, and the "fundamentalist position" of enzyme catalysis. *Biochemistry,* 11 April 1995, vol. 34 (14), 4507-10
- Rational Drug Design: Novel Methodology and Practical Applications. **PARRIL, A. ; REDDY, R.** ACS Symposium Series. Oxford University Press, 1999, vol. 719
- **PAULING.** *Chem. Eng. News,* 1946, vol. 24, 1375
- **PAULING.** *Nature,* 1948, vol. 161, 707
- **POLGAR.** The different mechanisms of protease action have a basic feature in common: proton transfer from the attacking nucleophile to the substrate leaving group. *Acta Biochim Biophys Hung.,* 1988, vol. 23 (3-4), 207-13
- **RADZICKA ; WOLFENDEN.** Contemporary Enzyme Kinetics and Mechanism. 1996, 273-298
- **ROBERTS et al.** Rational design of peptide-based HIV proteinase inhibitors. *Science,* 20 April 1990, vol. 248 (4953), 358-61
- **SCHOWEN.** Transition States of Biochemical Processes. Plenum, 1978
- **SERVICE.** Structural genomics offers high-speed look at proteins. *Science,* 17 March 2000, vol. 287 (5460), 1954-6
- **SZEDLACSEK et al.** Kinetics of slow and tight-binding inhibitors. *Methods Enzymol,* 1995, vol. 249, 144-80
- **TSAI ; JORDAN.** *J. Phys. Chem.,* 1993, vol. 97 (11), 11227-11237
- **THREADGOLD et al.** Taenia crassiceps: regional variations in ultrastructure and evidence of endocytosis in the cysticercus' tegument. *J Exp Parasitol.,* February 1983, vol. 55 (1), 121-31
- **TSANG et al.** *Parasitol. Today,* 1995, vol. 11, 124-126
- **VACCA et al.** L-735,524: an orally bioavailable human immunodeficiency virus type 1 protease inhibitor. *Proc Natl Acad Sci U S A,* 26 April 1994, vol. 91 (9), 4096-100
- **WANG et al.** The double catalytic triad, Cys25-His159-Asp158 and Cys25-His159-Asn175, in papain catalysis: role of Asp158 and Asn175. *Protein Eng.,* January 1994, vol. 7 (1), 75-82
- **WOLFENDEN.** Conformational aspects of inhibitor design: enzyme-substrate interactions in the transition state. *Bioorg Med Chem.,* May 1999, vol. 7 (5), 647-52